(19) Europäisches Patentamt
European Patent Office
Office européen des brevets

(11) **EP 3 890 600 B1**

(12) **EUROPÄISCHE PATENTSCHRIFT**

(45) Veröffentlichungstag und Bekanntmachung des Hinweises auf die Patenterteilung:
**10.07.2024 Patentblatt 2024/28**

(21) Anmeldenummer: **19828572.8**

(22) Anmeldetag: **05.12.2019**

(51) Internationale Patentklassifikation (IPC):
*A61B 5/021* [(2006.01)]   *A61B 5/0215* [(2006.01)]

(52) Gemeinsame Patentklassifikation (CPC):
**A61B 5/02108; A61B 5/02156**

(86) Internationale Anmeldenummer:
**PCT/EP2019/083834**

(87) Internationale Veröffentlichungsnummer:
**WO 2020/115216 (11.06.2020 Gazette 2020/24)**

(54) **KALIBRIERUNGSVERFAHREN UND KALIBIERUNGSSYSTEM FÜR KONTINUIERLICHE BLUTDRUCKMESSUNGEN**

CALIBRATION METHOD AND CALIBRATION SYSTEM FOR CONTINUOUS BLOOD PRESSURE MEASUREMENTS

PROCÉDÉ DE CALIBRATION ET DISPOSITIF DE CALIBRATION POUR DE MESURES CONTINUES DE LA PRESSION ARTÉRIELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.12.2018 DE 102018009457**

(43) Veröffentlichungstag der Anmeldung:
**13.10.2021 Patentblatt 2021/41**

(73) Patentinhaber: **Redtel, Holger**
**19348 Perleberg (DE)**

(72) Erfinder: **Redtel, Holger**
**19348 Perleberg (DE)**

(74) Vertreter: **Raffay & Fleck**
**Patentanwälte**
**Grosse Bleichen 8**
**20354 Hamburg (DE)**

(56) Entgegenhaltungen:
**WO-A1-2018/102486     US-A1- 2012 136 261**

• **JENS FIALA ET AL: "Implantable sensor for blood pressure determination via pulse transit time", SENSORS, 2010 IEEE, IEEE, PISCATAWAY, NJ, USA, 1 November 2010 (2010-11-01), pages 1226 - 1229, XP031851564, ISBN: 978-1-4244-8170-5**

**Beschreibung**

**Stand der Technik**

Kontinuierliche Blutdruckmessungen werden heute invasiv in der Klinik eingesetzt, um Patienten zu überwachen und den Krankheitsverlauf zu dokumentieren.

Bei der invasiven Blutdruckmessung wird eine Arterie, vorzugsweise die A. Radialis, kanüliert. Die arterielle Kanüle wird über einen Schlauch mit einem Druckaufnehmer verbunden. Der Schlauch ist mit einer Elektrolytlösung gefüllt. Der Druck in der Arterie setzt sich also über die Kanüle in den Schlauch fort und gelangt zum Druckaufnehmer, welcher dieses Drucksignal in ein elektrisches Signal zur Anzeige auf einem Monitor umwandelt.

Damit die Kanüle und die Schläuche nicht durch Bluteinfluss und Gerinnselbildung verstopfen, ist ein Druckbeutel angebracht, mit dem Elektrolytlösung durch den Schlauch und die Kanüle in den Körper gespült wird, in der Regel mit 3 ml/Stunde.

Damit das Signal möglichst wenig gedämpft wird, darf keine Luft im System sein. Daher muss vor der Anwendung der invasiven Blutdruckmessung das gesamte System sorgfältig mit Elektrolytlösung gespült werden. Des Weiteren kann es aufgrund der Wahl der Schlauchlänge zu Resonanzen des Systems als Kopplung mit dem Herzkreislaufsystem kommen. Dies drückt sich durch eine überlagerte Welle in den Messwerten der Blutdruckwelle aus und verfälscht somit die Messung. Um dies zu beheben, muss eine andere Schlauchlänge gewählt werden.

Eine weitere Fehlerquelle ist die Höhe des Druckabnehmers. Dieser muss auf der Höhe des HIP (hydrostatischer Indifferenzpunkt), also auf Herzhöhe, befestigt sein. Da im Klinikalltag Patienten aufgesetzt und wieder hingelegt werden, wird oftmals die Positionsanpassung des Druckabnehmers vergessen. Ist der Druckabnehmer unterhalb des HIPs wird ein zu hoher Blutdruck gemessen und umgekehrt für oberhalb des HIPs.

Eine invasive Blutdruckmessung ist wie jedes invasive Verfahren nicht ohne Risiken. So treten bei 1-4 % der Untersuchungen Sepsen und Infekte auf. Auch Blutungen können mit einer Wahrscheinlichkeit von 0,5-2,6% auftreten. Weitaus häufiger sind Hämatome mit 14%. Am häufigsten treten jedoch mit fast 20% temporäre Okklusionen der Arterien auf. In seltenen Fällen kommt es zu kritischen Durchblutungsstörungen (0,09%), jedoch sind diese Komplikationen besonders fatal, da diese zu einer Amputation oder zu einer Funktionsstörung von Gliedmaßen führen können.

Aufgrund dieser Risiken und der möglichen Komplikationen ist die tagtägliche und millionenfache Verwendung der invasiven Blutdruckmessung eigentlich nicht zu verantworten. Da jedoch bis heute kein adäquater Ersatz vorliegt, gehört diese Methode zu den notwendigen Übeln der heutigen Medizin.

Mit der Erfindung soll daher eine Alternative zur invasiven Blutdruckmessung aufgezeigt werden.

Systeme und Messgeräte zur nichtinvasiven kontinuierlichen Blutdruckmessung werden nur von wenigen Herstellern angeboten. Diese Messgeräte sind aufgrund von notwendigen und aufwendigen Kalibrierungsschritten nicht für jedermann alltagstauglich zu verwenden.

Eine heutzutage verwendete Methode zur nichtinvasiven kontinuierlichen Blutdruckmessung ist die Ermittlung des Blutdrucks aus der Pulswellenlaufzeit. Eine Kalibrierung im Ruhezustand und im belasteten Zustand (bei oder direkt nach sportlicher Betätigung) der zu untersuchenden Person ist notwendig, um den Wert der Pulswellenlaufzeit auf verwertbare Wertepaare von Systole und Diastole übertragen zu können. Diese Messmethode ist indirekt, da die Pulswellenlaufzeit für die Messung genutzt wird. Die Pulswellenlaufzeit weist zwar mit den Werten für den Blutdruck eine Korrelation auf, die genaue Korrelation gilt jedoch nur für eine Person und verändert sich auch mit der Zeit. Daher ist Korrelation zwischen Pulswellenlaufzeit und Blutdruck von Person zu Person unterschiedlich. Hinzu kommt, dass die Korrelation bei Belastung unterschiedlich reagiert. Medikamente verstärken diese Unsicherheiten. Aus diesem Grund muss derzeit notwendigerweise eine Kalibrierung, wie oben beschrieben, wiederholend durchgeführt werden. Aktuell ist eine solche Kalibrierung auf Grund der Ungenauigkeiten, insbesondere auch der beaufschlagten Blutdruckmessung nach Riva Rocci vergleichsweise häufig nötig. Daher kann der Blutdruck über die Pulswellenlaufzeit, bzw. die Pulswellengeschwindigkeit nur abgeschätzt werden. Wobei die Pulswellengeschwindigkeit aus der Pulswellenlaufzeit berechenbar ist, indem der Abstand zwischen zwei örtlich beabstandeten Messpunkten zur Ermittlung der Pulswellenlaufzeit durch die Pulswellenlaufzeit geteilt wird.

Eine andere Methode zur nichtinvasiven kontinuierlichen Blutdruckmessung, welche heutzutage Verwendung findet, beruht auf den Arbeiten von Jan Peňáz. Hierbei wird durch einen Finger Licht gestrahlt. Die Lichtstärke des transmittierten Lichts hängt vom Blutfluss ab und variiert somit mit dem Herzschlag. Gleichzeitig wird der Finger um die Lichtquelle und den Lichtempfänger abgeschnürt, jedoch so, dass der Blutfluss konstant bleibt. Daher muss die Stärke der Abschnürung mit einer Regelelektronik kontinuierlich angepasst werden. Der Druck der Abschnürung aus der Regelelektronik wird als Ausgangssignal verwendet und mit einer Kalibrierung zu einem Blutdrucksignal übertragen. Daher ist auch diese Methode indirekt.

Derzeit gibt es kein Gerät am Markt, das direkt nichtinvasiv kontinuierlich Blutdruck messen kann.

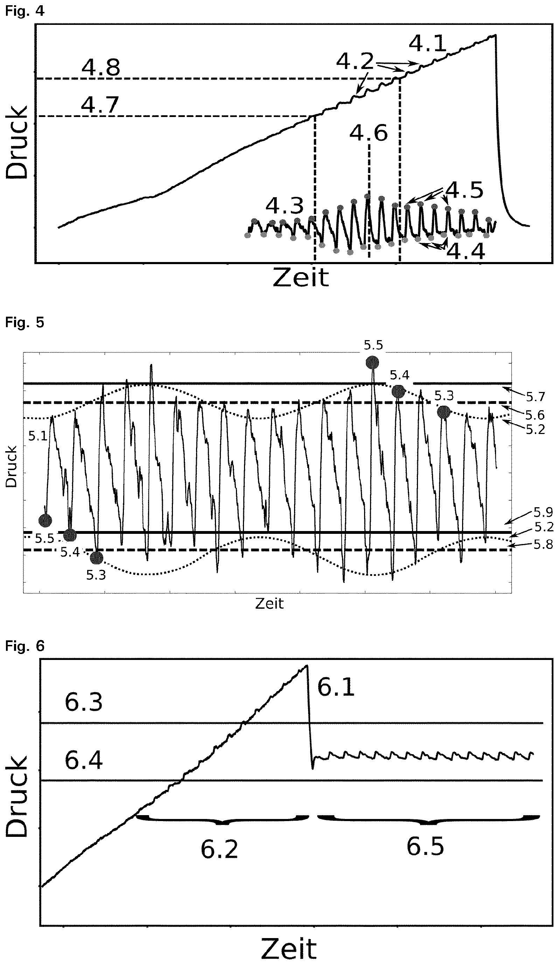

Eine heutzutage verwendete Methode zur direkten, jedoch nicht kontinuierlichen, Messung eines einzelnen Wertes des Blutdrucks beruht auf der sogenannten Riva-Rocci-Methode. Hierbei wird der Oberarm oder am Handgelenk der Arm mit einer Luftdruckmanschette abgequetscht und werden Signale des Herzpulses interpretiert. Typischerweise wird heutzutage der Druck in der Manschette gemessen. Dieser weist bei einer geringen Luftbefüllung keine rhythmischen Variationen auf, bei mittlerer Luftbefüllung ist ein Signal mit der Frequenz des Herzpulses zu erkennen, welches bei hoher Luftbefüllung wieder verschwindet. Aus den Punkten der Luftbefüllung bzw. des Luftdrucks in der Manschette an denen das Signal erscheint bzw. verschwindet, können die Werte für Diastole und Systole bestimmt werden. Die Funktionsweise ist in Fig. 4 dargestellt.

Wird heutzutage eine kontinuierliche nicht invasive Messung des Blutdrucks z.B. über 24 Stunden verordnet, so wird ein Gerät, welches nach der Riva-Rocci-Methode arbeitet, verwendet, welches in regelmäßigen Abständen eine Messung durchführt, bspw. alle 15 Minuten, und deren Werte speichert.

Ein werthaltiges Tagesprofil ist nur bedingt aus diesen Daten ableitbar. Durch die ständigen Messungen wird der Tag-Nachtrhythmus gestört, der Patient wird bei jeder Messung an die Messung erinnert und die Venen und Lymphe stehen unter enormer Belastung, zudem ist die Bewegung des Patienten durch das Gerät eingeschränkt.

Ein Verfahren zur kontinuierlichen Blutdruckmessung ist aus der US 2012/136261 bekannt.

**Darstellung der Erfindung**

Mit der hier vorgestellten Erfindung sollen diese Mängel vermieden werden, insbesondere soll ein werthaltiges Tagesprofil aufgezeichnet werden können. Auch soll eine einfache und zuverlässige und lange nutzbare Kalibration zwischen beaufschlagter Blutdruckmessung und nicht beaufschlagter Messung ermöglicht werden.

Zudem ist die bekannte Art der kontinuierlichen Messung, beruhend auf der Riva-Rocci-Methode, zeitlich zu schlecht aufgelöst für weitreichendere Diagnosen. Mit der Erfindung soll auch aufgezeigt werden, wie sich ein System zur Blutdruckmessung bauen lässt, welches kontinuierlich, nicht invasiv und direkt den Blutdruck misst. Wobei die zeitliche Auflösung es erlaubt, zu jedem Herzschlag einen Wert für Diastole und Systole zu ermitteln.

Dabei erlauben die hier vorgestellten Methoden nicht nur die kontinuierliche Messung des Blutdrucks, sondern können auch verwendet werden, um eine verbesserte Einzelwertmessung, im Vergleich zur klassischen Riva-Rocci-Methode, zu erhalten.

Die hier vorgestellte Verbesserung der Riva-Rocci umfasst insbesondere sowohl Optimierungen innerhalb der Logiken als auch Optimierungen der Luftdruckmanschette an sich.

Heutige Systeme zur kontinuierlichen, insbesondere auch nicht invasiven Messung sind einerseits nur schwierig zu kalibrieren und daher nicht für jedermann praktikabel und andererseits beruhen die Messergebnisse auf indirekten Messwerten, welche durch äußere Faktoren leicht beeinflussbar sind. Daher soll auch aufgezeigt werden, wie sich eine leichte und automatisierte Kalibration der Messwerte realisieren lässt.

In einer weiteren Ausgestaltung soll auch aufgezeigt werden, wie sich eine angenehme, kontinuierliche und/oder nicht invasive Langzeitmessung z.B. über 24 Stunden hinweg realisieren lässt.

Ein leicht zu bedienendes und schmerzfreies kontinuierliches Blutdruckmessgerät ermöglicht neue Möglichkeiten der Anwendung.

Viele Sportler im Freizeitsport und vor allem im professionellen Sport verwenden heutzutage Fitnesstracker, welche, neben anderen nicht-Vitaldaten, in der Regel nur die Daten des Herzpulses ermitteln können und dies oftmals nur gemittelt über eine Zeitspanne. Die kontinuierliche Blutdruckmessung ermöglicht es somit einen viel aussagekräftigeren Vitalwert zu erheben und ermöglicht gleichzeitig die Bestimmung des Herzpulses ungemittelt für jeden Herzschlag zu erheben.

Durch Erhebung des Blutdrucks während des Sports oder in Pausen kann zudem der Sportler vor einer Überanstrengung gewarnt werden und somit kann das Risiko einer akuten Herzkreislauferkrankung (z.B. Herzinfarkt) beim Sport reduziert werden.

Auch die Beurteilung des Stresszustands ist eine viel gefragte Anwendung eines Messsystems zur Abbildung von Herzkreislauffunktionen. Die Beurteilung erfolgt dabei über die Messung und Einstufung der Herzraten- oder Pulswellenvariabilität. Im einfachsten Fall ergeben sich diese Parameter aus der Veränderung des Herzintervalls (z.B. aus dem EKG) oder dem Pulsintervall (z.B. aus der Redtel Methode (s.u.) oder einer Plethysmographie) des aktuellen Herzschlag zum vorherigen Herzschlag. Dabei sind diese Parameter, wie in vielen anderen Publikationen beschrieben, nicht gleich.

Die Herzratenvariabilität entsteht von Schlag zu Schlag und wird direkt am Herzen gemessen, z.B. in Form eines EKGs. Die Pulswellenvariabilität ist die Variabilität aus der körperlichen Situation heraus und wird durch den veränderlichen Zustand der Gefäße und des Gewebes beeinflusst. Die Pulswellenvariabilität wird durch die Anpassung an den Alltag des Gewebes und der Gefäße bestimmt und wird ferner durch den Verlauf, die Verästelung und den Zustand der Arterien bestimmt. Sie weicht somit deutlich von den elektrischen Signalen gemessen am Herz, z.B. mittels EKG, ab.

Die genaue Abbildung des Druckverlaufs in der Arterie und dies auch über Nacht kann bei Menschen mit Risiken im Herzkreislaufsystem darüber hinaus lebensrettend sein. Ein Anwendungsgebiet kann die Schlafüberwachung sein. Die genaue Aufzeichnung der Blutdruckkurve ermöglicht die Erkennung von Abnormalitäten. Wird eine solche erkannt, kann diese aufgezeichnet und einem Arzt zur Verfügung gestellt werden. Je nach Schwere der Abnormalität kann auch der Patient oder eine Bezugsperson durch ein Warnsignal geweckt oder alarmiert werden. Die Schlafüberwachung ermöglicht es schlafbezogene Atmungsstörungen anhand der Atmung, welche anhand der Respiratorische Sinusarrhythmie (RSA) und damit auch im Blutdruckverlauf bestimmbar ist, zu erkennen. Daneben hat die Messung des Blutdrucks während der Nacht einen hohen Aussagewert über das Risiko an einer kardiovaskulären Erkrankung zu erkranken. So zeigen Studien, dass Patienten, bei denen ein schweres kardiovaskuläres Ereignis aufgetreten ist, zuvor nachts einen erhöhten systolischen Blutdruckwert von im Mittel 7 mmHg über den Werten von gesunden Patienten und einen um im Mittel 4 mmHg niedrigeren Wert des diastolischen Blutdruckwerts aufwiesen. Diese Vergrößerung des Pulsdrucks wird auch Hammerpuls genannt und kann mit einer erfindungsgemäßen Anordnung erkannt werden.

Ein weiteres Ziel der Erfindung ist es, die Blutdrucküberwachung bei Diabetes zu verbessern. Bei Diabetes kann ein Symptom Bluthochdruck sein, wobei Werte der Systole über 140 mmHg als schädlich angesehen werden. Aber auch niedriger Blutdruck mit Werten der Systole unter 105 bis 100 mmHg können auftreten. Wird ein Bluthochdruck, welcher häufig bei Typ-2-Diabetikern, aber auch nach mehreren Jahren bei Typ-1-Diabeteikern auftreten kann, nicht behandelt, so steigt dieser im weiteren Verlauf der Krankheit an. Höhere Blutdruckwerte lassen das Risiko für Arteriosklerose, Schlaganfälle und Herzinfarkte deutlich ansteigen. Daher werden blutdrucksenkende Medikamente verabreicht, welche mit Nebenwirkungen einhergehen können. Eine Nebenwirkung ist, dass der Blutdruck zu weit gesenkt wird, sodass die Durchblutung von bereits geschädigten Arterien nicht mehr gewährleistet ist.

Die kontinuierliche Blutdruckmessung kann die Medikamentenaufnahme auf ein Minimum reduzieren, indem eine erfindungsgemäße Anordnung nur dann eine Medikamenteneinnahme empfiehlt, wenn der Blutdruck zunimmt. Im weiteren Verlauf einer Diabeteserkrankung können Nerven beeinträchtigt werden, die den Kreislauf aktivieren. Es kann zu einer orthostatischen Hypotonie kommen. Dies drückt sich dadurch aus, dass der Blutdruck beim Aufstehen dramatisch fällt und dass es zu Schwindel, Benommenheit, Schwarzwerden vor den Augen oder einer Ohnmacht kommen kann.

Eine erfindungsgemäße Anordnung kann den genauen Verlauf des Blutdrucks beim Aufstehen erkennen und somit die Schwere einer solchen Erkrankung quantifizieren.

Heutige Systeme zur kontinuierlichen Messung des Blutdrucks finden bei Operationen ihre Anwendung. Da jedoch diese Messungen heutzutage invasiv durchgeführt werden müssen und da dies nicht ohne Risiken ist, wird die Messung nur bei schwerwiegenden Operationen eingesetzt. Die hier vorgestellte Blutdruckmessung kann aufgrund ihrer nicht invasiven Messung auch bei kleineren Eingriffen eingesetzt werden und diese somit sicherer machen.

Ein weiteres Ziel der Erfindung ist es die invasive Messung, wie sie auf der Intensivstation oder auf der Wachstation heutzutage Verwendung findet, weitestgehend abzulösen.

Bei einer invasiven Blutdruckmessung wird ein Sensor in Form eines Katheters in eine Arterie des Arms oder des Beins eingeführt. Dies bedeutet, dass über die Messung hinweg eine offene Wunde vorliegt und der Patient an das Bett gefesselt ist. Daher wird eine geeignete Umgebung, wie z.B. eine Intensivstation, benötigt für eine derartige Untersuchung bzw. Überwachung.

Zudem bedarf die Anwendung der invasiven Messung einer Aufsicht durch einen Arztes oder durch besonders geschulte Pflegekräfte. Die hier vorgestellte Erfindung lässt sich von einer ungeschulten Person auch zuhause benutzen und verursacht nur minimale Schmerzen, wobei gleichzeitig eine mit einer invasiven Blutdruckmessung vergleichbare Messqualität erreicht wird.

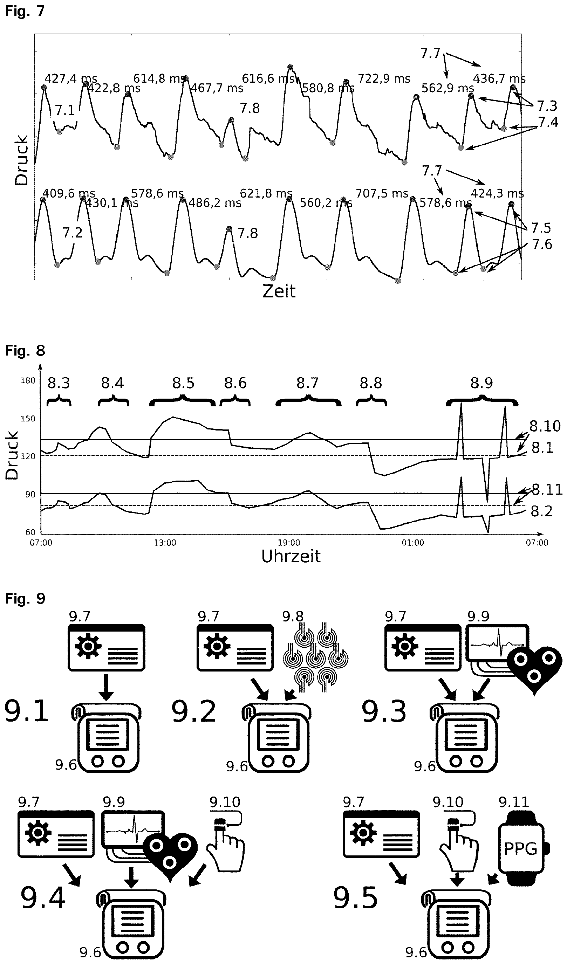

Fig. 7 zeigt eine Gegenüberstellung der Messwerte einer invasiven Messung (7.2) und der Ergebnisse der Redtel Methode (7.1) an einem Patienten, wobei die Messungen zeitgleich durchgeführt wurden, wobei die invasive Methode am linken Arm die Daten erhob und die Redtel Methode den rechten Arm verwendete. Es kann gezeigt werden, dass die Druckamplitude vergleichbar ist, dass die ermittelten RR-Intervalle vergleichbar sind und dass auch Auffälligkeiten, wie z.B. Arrhythmien erkannt werden können.

Ein weiterer Vorteil der hier vorgestellten Methoden, im Besonderen die Messung des Blutdrucks anhand der Pulswellenlaufzeit, gegenüber der invasiven Methode, ist, dass die Wahl des Messortes am Körper nicht eingeschränkt ist, und praktisch jede Hautoberfläche zur Messung verwendet werden kann. Bei der invasiven Methode werden große und einfach zugängliche Arterien benötigt. Daher werden häufig die A. Radiales in den Armen verwendet. Falls diese nicht mehr zugänglich sind, können die A. Femorales in den Beinen oder die A. Dorsales Pedes in den Füßen verwendet werden. Technisch möglich wäre die Verwendung der Halsschlagadern, da es aber aufgrund von Komplikationen durch die invasive Messung zu Okklusionen kommen kann, wird dies nicht praktiziert.

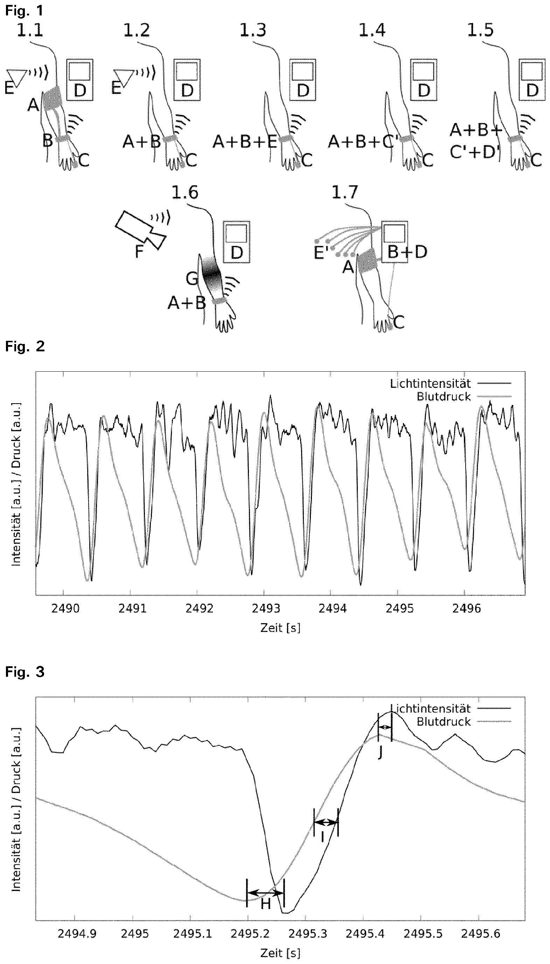

Wird die Erfindung in ein medizinisches Überwachungsgerät integriert, vgl. Fig. 1.7 und Beschreibung, so lassen sich viele heute bereits übliche Sensoren solcher Überwachungsgeräte für die Erfindung nutzen. Dies hat den Vorteil, dass zum einen es sich bei den vorhandenen Sensoren bereits um validierte Systeme handelt. Zum anderen ist eine minimale Anzahl an Sensoren erstrebenswert, da so das Gerät schneller in Betrieb genommen werden kann und eine geringere Belastung für den Patienten erreicht wird.

Die Bestimmung von Pulswellenlaufzeit bzw. der Pulswellengeschwindigkeit kann bspw. mittels EKG und Plethysmographie erfolgen. Beides sind übliche Sensoren an einem heutigen medizinischen Überwachungsgerät. Neben der Plethysmographie kann auch ein ähnlich aufgebauter Sensor zur Oximetriemessung vorhanden sein, auch dieser ermöglicht die genaue Abbildung der Pulswelle.

Bei der Verwendung der Plethysmographie sind prinzipiell zwei Methoden bekannt die Pulswellenlaufzeit bzw. die Pulswellengeschwindigkeit zu bestimmen. Die Daten einer Plethysmographie zeigen Wellen, deren Wellentäler und Wellenberge Funktionen des Herzens zugeordnet werden können. Wird ein zweiter Sensor entfernt vom ersten verwendet, dies kann eine weitere Plethysmographie, ein EKG, ein Beaufschlagungssensor oder ein System, welches auf der Redtel Methode basiert, können in den Signalen die gleichen Funktionen des Herzens erkannt werden. Der zeitliche Unterschied zwischen diesen Signalen ist die Pulswellenlaufzeit, welche mit dem Abstand zwischen den Sensoren in eine Pulswellengeschwindigkeit überführt werden kann.

Die Sensoren Plethysmographie, Beaufschlagungssensor und Redtel Methode eignen sich zur Bestimmung eines Endzeitpunktes aber auch zur Bestimmung eines Anfangszeitpunkts. Die Analyse des EKG kann nur für die Bestimmung des Anfangszeitpunkt herangezogen werden.

Soll nur ein Sensor verwendet werden so kann die sogenannte Reflexionswelle verwendet werden. Dies ist eine Welle, die der Initialwelle folgt und in der Regel bei Menschen mittleren Alters zu erkennen ist. Die Kombination von Reflexionswelle und Initialwelle heißt Pulswellenkontur. Die Reflexionswelle kann mit der Plethysmographie, einem Beaufschlagungssensor oder der Redtel Methode aufgezeichnet werden. Aus dem zeitlichen Abstand zwischen Initialwelle und Reflexionswelle kann die Pulswellenlaufzeit in der Aorta bestimmt werden. Die Pulswellengeschwindigkeit ergibt sich aus der Länge des Aortenbogens, welcher für Alter und Größer abgeschätzt werden kann.

Neben der Verwendung der Pulswellenlaufzeit bzw. Pulswellengeschwindigkeit kann auch die gemessene Spannung eines Elektrokardiogramms verwendet werden, um den Blutdruck zu bestimmen. Dabei werden die Spannungen z.B. der R-Zacken analysiert, welche mit dem Blutdruck und der Atmung korrelieren. Der Blutdruck ergibt sich aus der Funktion des Herzens. Das Herz muss je nach gefordertem Blutdruck einen stärkeren oder schwächeren Herzpuls ausführen. Die Stärke des Herzpulses wird durch das Spannungssignal vorgegeben, daher kann dieses Signal auch zur Blutdruckmessung und/oder Blutdruckveränderungsmessung verwendet werden.

Neben diesen technischen Zielen wird auch ein Umdenken in der Medizin angestrebt. Die heutige Medizin ist darauf ausgerichtet, Krankheiten zu heilen. Ein besserer Ansatz wäre es jedoch Krankheiten zu verhindern und die Lebensqualität vor allem im Alter zu steigern. Die hier vorgestellten Methoden erlauben es, im Bereich der Herzkreislaufmedizin zwei Hauptprobleme zu verbessern. Zum einem können kritische Zustände schon im Anfangsstadium erkannt werden, wodurch eine Umstellung von Lebensumständen bereits als Vorbeuge dienen kann. Zum anderen kann bei einer Medikation genau das richtige Maß zum richtigen Zeitpunkt gefunden werden.

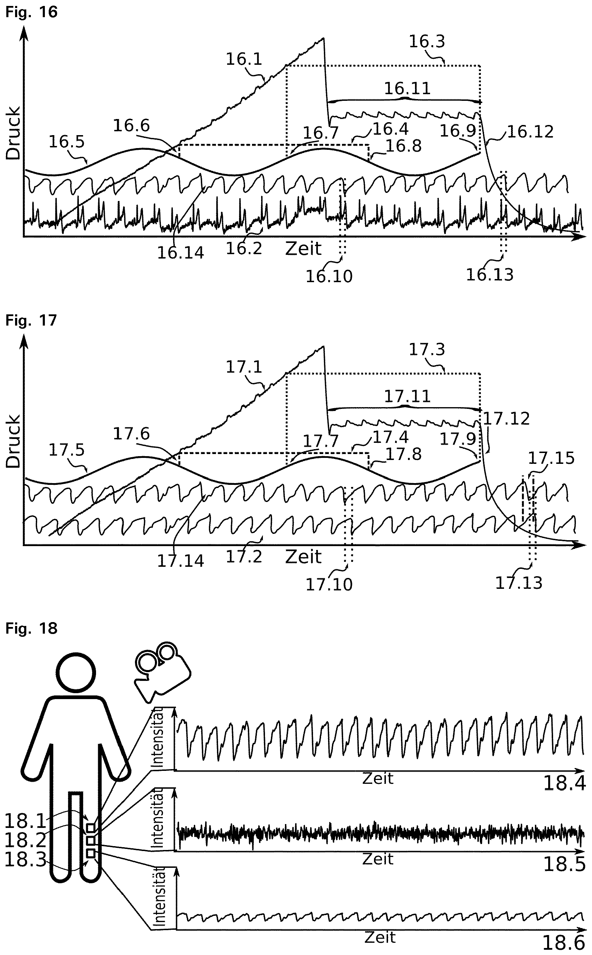

Ein Beispiel für die frühzeitige Erkennung von Krankheiten sind Verschlusskrankheiten (vgl. Fig 18-20). Tritt eine Verschlusskrankheit in einer Extremität auf, so verändert sich die Pulswellengeschwindigkeit relativ zur gesunden Extremität. Eine vergleichende Messung der Pulswellengeschwindigkeit an beiden Beinen und die Feststellung, dass diese Geschwindigkeiten nicht gleich sind, kann also einen Hinweis auf eine Verschlusskrankheit oder deren Vorstadien sein. Daher wird eine einfach zu verwendende Messmethode zur Messung der Pulswellengeschwindigkeit vorgestellt.

Eine auf die Situation maßgeschneiderte Medikation ist notwendig für die Steigerung der Lebensqualität und kann besser erfolgen, als die heute üblichen Verschreibungen, wie z.B. vor oder nach dem Essen. Eine Medikation kann nicht nur auf Zeitvorgaben basieren. Es muss auch die körperliche Konstitution, das Alter, die Größe, das Gewicht miteinbezogen werden. Darüber hinaus ermöglicht die Messung des Tagesprofils (vgl. Fig.8, 10 und 11) eine weitere maßgeschneiderte Form der Medikation. Wenn bei der Aufzeichnung des Tagesprofils sich ein Anstieg des Blutdrucks abzeichnet und dieser nach Parametern des behandelnden Arztes zu behandeln ist, kann dies dem Nutzer mitgeteilt werden, sodass dieser eine Medikation durchführt. Dies hat zur Folge, dass die Medikation weniger stark überdosiert wird und zu den Zeiten eingenommen wird, zu denen sie auch notwendig ist. Andere Parameter können bspw. eine Veränderung der Pulswellenlaufzeit oder des Pulsdrucks sein.

Heutzutage wird in der Regel eine zu hohe Dosierung der Medikation vorgenommen und der Blutdruck wird auf einen festen Wert fixiert. Diese Fixierung ist nicht in jedem Fall erstrebenswert, da eine Fixierung auch bedeutet, dass die Fähigkeit zur Freude durch die Fähigkeit des plötzlichen Blutdruckanstiegs verhindert wird, dies kann zur Folge haben, dass z.B. Depressionen auftreten. Daneben hat eine über das Notwendige hinaus gehende, zu hohe Dosierung zur Folge, dass Nebenwirkungen, wie z.B. Organschädigungen, auch über das Notwendige hinaus, auftreten. Diese Situation zeigt, dass eine Medikation gerade so geregelt werden muss, dass gilt: "so viel wie nötig, so wenig wie möglich". Wird der Blutdruck kontinuierlich erfasst, also über den gesamten Tag hinweg, so kann auch ein langsames Steigen des Blutdrucks von einem punktuellen Anstieg unterschieden werden. Eine Medikation bspw. gegen Bluthochdruck kann dann veranlasst werden, wenn diese notwendig ist, und kann darüber hinaus auch in ihrer Dosierung auf den zu senkenden Druckunterschied angepasst sein.

Für neue Produkte im Bereich der Medikation kann die kontinuierliche Messung von entscheidender Bedeutung sein. So müssen neue Größen der Dosierung entwickelt werden, die geeignet sind kleine Anpassungen des Blutdrucks durchzuführen. Da der Blutdruckverlauf bekannt ist, vor allem auch in Diastole und Systole getrennt, können Produkte entwickelt werden, die die Diastole bzw. die Systole unabhängig voneinander beeinflussen. Oder es können Medikamente entwickelt werden, die nur den gefährlichen Hammerpuls unterdrücken und den sonstigen Verlauf des Blutdrucks unverändert lassen.

Ein weiteres Problem der heutigen Medizin ist, dass alle Menschen genau gleich behandelt werden. Es werden Faktoren wie Geschlecht, Größe, Alter und Gewicht nur bedingt oder auch gar nicht beachtet. So ist der Grenzwert für einen gesunden Blutdruck nach der WHO 2017 herabgesetzt worden. Ist der systolische Wert des Blutdrucks höher als 130 mmHg oder der diastolische Wert höher als 80 mmHg, wird dies als Bluthochdruck und somit als krank eingestuft. Diese Grenzwerte sind jedoch nur für den durchschnittlichen Menschen geeignet. Ein Mensch mit einer Körpergröße über zwei Metern muss einen hohen Blutdruck haben, da sonst die Sauerstoffversorgung im Gehirn nicht gegeben ist. Genau das gleiche gilt auch für ältere Menschen, bei denen bereits die Arterien verkalkt sind. Auch hier ist der hohe Blutdruck für eine Versorgung des Gehirns (und anderer Organe) notwendig. Der hohe Blutdruck ist nicht die Erkrankung, sondern die körperliche Reaktion auf eine andere Erkrankung. Wird der Blutdruck fixiert, kommt es zu einer Unterversorgung von bspw. dem Gehirn, was weitere Krankheiten, wie z.B. Demenz zur Folge haben kann.

Genau das Gegenteil trifft für kleine Menschen zu. Ein Wert von 120/80 kann bereits ein Hinweis auf einen krankhaften Bluthochdruck sein, da jedoch die Grenzwerte dies nicht ausweisen, wird eine notwendige Medikation verweigert.

Diese Beispiele zeigen, dass ein Umdenken der Blutdruckinterpretation erforderlich ist und maßgeschneidert für den einzelnen Menschen vorgenommen werden

Ein erfindungsgemäßes Verfahren und ein erfindungsgemäßes System werden in den Ansprüchen 1 und 3 definiert.

## 3. Die Redtel Methode

In der Patentschrift der DE 10 2018 001 390, der PCT/EP2018/056275 und der DE-Anmeldung 10 2018 007 180.5, wird neben einem neun Messgerät für die beaufschlagte Blutdruckmessung eine Verbesserung der Riva-Rocci-Methode beschrieben, die basierend auf einer physischen Messung eine kontinuierliche Blutdruckmessung, hier nachfolgend benannt als "Redtel Methode", durchführt. Es wird in der einfachsten Variante aufgezeigt, dass eine kontinuierliche Messung möglich ist, indem neue Logiken zur Ansteuerung einer herkömmlichen Blutdruckmanschette angewendet werden.

Der Ablauf einer Messung mit der "Redtel Methode" teilt sich insbesondere in zwei Phasen auf (vgl. Fig. 6). In der ersten Phase wird der Blutdruck herkömmlich gemessen. Die Art der Messung ist dabei nicht entscheidend, jedoch ist eine Messung nach der Riva-Rocci-Methode vorteilhaft, da so nur ein Gerät am Körper angebracht werden muss. Die dabei ermittelten Werte für Diastole und Systole stellen Ausgangswerte für die neuen Logiken der "Redtel Methode" dar. In der zweiten Phase wird der Druck in der Blutdruckmanschette eingestellt, bzw. reduziert, und es werden die Daten des Luftdrucks in der Manschette mit Hilfe der Ausgangswerte als kontinuierliche Blutdruckwelle interpretiert.

Diese Blutdruckwelle kann verwendet werden, um weitergehende Analysen durchzuführen. So können Werte für Diastole und Systole für jeden Herzschlag unter Rückgriff auf die Ausgangswerte ermittelt werden, fehlende oder zu viele Herzschläge (Arhythmien, vgl. Fig. 7) erkannt werden oder auch die Regelmäßigkeit der Herzschläge bestimmt werden.

Diese einfachste Variante zeichnet sich dadurch aus, dass bezüglich der Ausgangswerte in den Messablauf nach der Riva-Rocci-Methode nicht eingegriffen wird.

Die Verwendung einer unveränderten Riva-Rocci Manschette oder insbesondere Methode hat jedoch den Nachteil, dass die Kalibrierung ungenau ist. Die Blutdruckkurve verändert sich nicht nur mit dem Herzschlag, sondern ist bspw. auch durch Auswirkungen der Atmung überlagert, dies wird Respiratorische Sinusarrhythmie (RSA) genannt (vgl. Fig. 5). Dies begründet, warum die Werte, welche nach der Riva-Rocci-Methode ermittelt werden, ungenau sind. Bei der Riva-Rocci-Methode sind die Werte für Diastole und Systole in der Regel nicht von einem einzigen Herzschlag, sondern können von Herzschlägen herrühren, welche bis zu 60 Sekunden zueinander versetzt sein können. In Fig. 5 wird dieser Umstand aufgezeigt. Abgebildet ist die Druckverlaufskurve mehrerer Herzschläge; im Gesamten werden zwei Atemzüge abgebildet. Wird mit der Riva-Rocci-Methode (und im speziellen beim Druckaufbau) gemessen, so kann die Riva-Rocci-Methode verschiedene Wertepaare (bspw. 5.3, 5.4 oder 5.5) finden. Hierbei liegen die Werte von Diastole und Systole zeitlich weit auseinander.

So kann die Riva-Rocci-Methode Werte liefern, die dem Nutzer einen gesunden Zustand (5.3), einen kritischen (5.4) oder auch einen erkrankten Zustand (5.5) nach WHO Einstufung attestieren, obwohl die Daten von der gleichen Druckverlaufskurve stammen.

Die Blutdruckschwankung aufgrund des Herzpulses wird als Blutdruckschwankung 1. Ordnung bezeichnet. Blutdruckschwankungen 2. Ordnung werden durch die Atmung (und andere Effekte, z.B. Einstellungen der Gefäße auf äußere Temperaturveränderungen) ausgelöst. Die Blutdruckschwankung 2. Ordnung beeinflusst den Blutdruck in der Regel in einer längeren zeitlichen Periode als der Herzschlag. Studien zeigen, dass dieser Effekt der Atmung bis zu 10 mmHg ausmacht (Sin P.Y.W., Galletly D.C., Tzeng Y.C. Influence of breathing frequency on the pattern of respiratory sinus arrhythmia and blood pressure: old questions revisited, Am J Physiol Heart Circ Physiol 298: H1588-H1599, 2010).

Um dies in Relation zu bringen zeigt ein Meta-Studie (Lewington S., Clarke R., Qizilbash N., Peto R., Collins R. Age-specific relevance of usual blood pressure to vascular mortality: a metaanalysis of individual data for one million adults in 61 prospective studie, Lancet 360: 1903-1913, 2002), dass bereits eine durchschnittliche Reduktion des systolischen Blutdrucks um 2mmHg eine Reduktion der Sterblichkeit bei koronaren Herzkrankheiten um 7% bewirkt und insbesondere bei Schlaganfällen eine Reduktion der Sterblichkeit um 10% zur Folge hat.

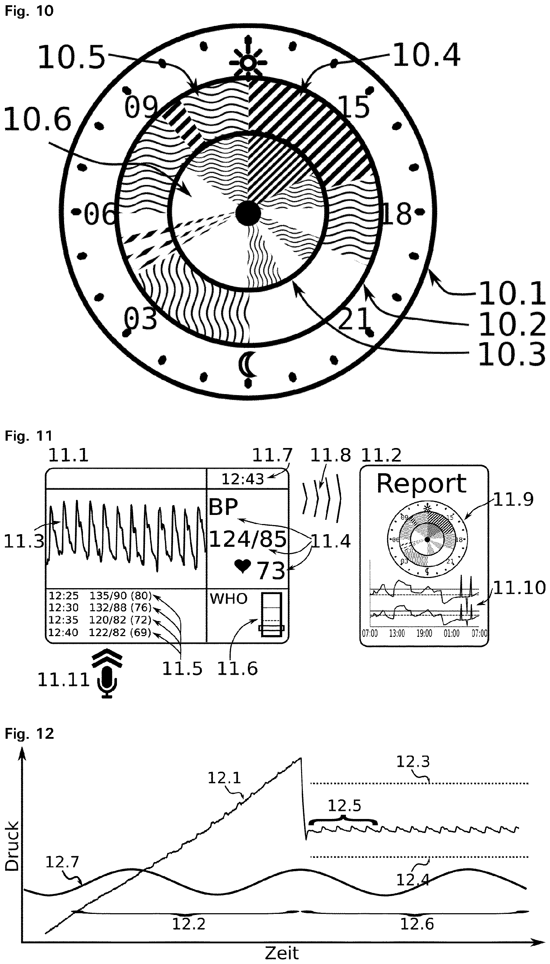

Die Messung mittels klassischer Blutdruckmessung nach Riva-Rocci hat einen systematischen Messfehler von 10%, dies bedeutet, dass bei einem wahren systolischen Blutdruck von 120 mmHg ein absoluter Messfehler von bis zu 12 mmHg auftreten kann. Eine verbesserte Kalibrierung gemäß der PCT/EP2018/056275 erfolgt, insbesondere indem die Zeitpunkte an denen der Wert für Diastole und Systole mittels Riva-Rocci-Methode erkannt werden, festgehalten werden (vgl. Fig. 12-17). Da gleichzeitig im Hintergrund die Daten für die "Redtel Methode" erfasst werden, kann eine genaue Kalibration unter Berücksichtigung der Effekte der RSA durchgeführt werden.

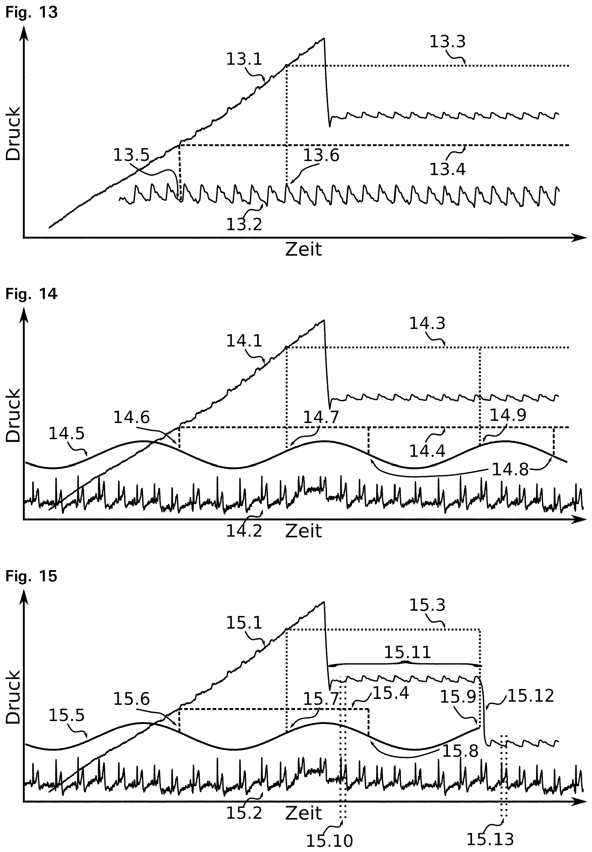

Die PCT/EP2018/056275 beschreibt weiterhin, wie der beaufschlagte Druck nach der Blutdruckmessung nach Riva-Rocci soweit reduziert wird, dass eine Blutdruckwelle ermittelt werden kann. Der dauerhaft beaufschlagte Druck kann hierbei bspw. 100 mmHg betragen. Vorteilhaft wären jedoch geringere Beaufschlagungen, da der venöse Rückstau sowie die Belastung der Lymphe nur eine begrenzte Zeit zu ertragen ist. Dafür werden, wie in der PCT/EP2018/056275 beschrieben, neue Drucksensoren verwendet, die auch bei einer geringeren Beaufschlagung bereits ein brauchbares Ausgangssignal ergeben (vgl. Fig. 9.2 und 13). Der Messablauf (siehe auch Fig. 6) mit einem Gerät nach der "Redtel Methode", unter Verwendung der integrierten Variante in einer herkömmlichen Riva-Rocci Manschette, gestaltet sich insbesondere wie folgt: Zunächst wird eine herkömmliche Riva-Rocci Messung durchgeführt, wobei vorteilhafterweise die Messung beim Aufpumpen der Manschette durchgeführt wird. Nachdem die Manschette aufgepumpt ist und die Werte für Systole und Diastole bestimmt sind, wird der durchschnittliche Druck in der Manschette reduziert, bspw. auf 100 mmHg. Bei einem solchen durchschnittlichen Druck variieren die einzelnen Druckwerte in der Manschette mit dem Herzschlag, bzw. mit der Blutdruckveränderung. Mit den aus der Riva-Rocci Messung bestimmten Werten für Systole und Diastole können diese Variationen im Druck Veränderungen im Blutdruck zugeordnet werden und die Abbildung der Blutdruckkurve ist möglich. Durch die Analyse der einzelnen Peaks in dieser Kurve kann zu jedem Herzschlag ein Wert für die Systole und ein Wert für die Diastole ermittelt werden.

Im folgenden Kapitel wird beispielhaft aufgezeigt, wie die Blutdruckvariation 2. Ordnung erkannt werden kann und somit der systematische Messfehler der klassischen Riva-Rocci-Methode verhindert werden kann.

## 4. Erweiterungen zur "Redtel Methode"

Es gilt hier ausgangs: Umso geringer der Anpressdruck, umso länger kann eine direkte kontinuierliche Blutdruckmessung durchgeführt werden.

Die Erweiterung der "Redtel Methode" besteht nun darin, dass die Beaufschlagung noch weiter reduziert werden kann, insbesondere auf oder unter 80mmHg oder 11 kPascal, insbesondere auf oder unter 60mmHg oder 8 kPascal, insbesondere auf mindestens 30 mmHg oder mindestens 4kPascal, sodass auch eine Langzeitmessung bspw. über 24 Stunden ermöglicht wird, sodass die herkömmliche Langzeitmessung beruhend auf der klassischen Riva-Rocci-Methode abgelöst werden kann.

Neben der Ermöglichung einer kontinuierlichen Messung kann auch die einfache Einzelmessung mittel Riva-Rocci durch die Logiken der Redtel Methode verbessert werden.

Die Verbesserung der Redtel Methode besteht auch darin, auch die Einflüsse der Atmung auf die Pulswelle und somit auf die Werte von Systole und Diastole zu erkennen und zu bewerten und/oder die Werte von Diastole und Systole zu vorgegebenen Punkten innerhalb der Atmung zu erfassen. Die Messung des Blutdrucks mittels Riva-Rocci-Methode kann auch verbessert werden, indem die Manschette an sich optimiert wird. Dem Fachmann ist bekannt, dass je nach Armumfang eine geeignete Manschette benutzt werden soll, um ein optimales Messergebnis zu erreichen. Je dicker der Arm, umso breiter sollte die Manschette ausfallen. Es wird daher eine verbesserte Anordnung der Luftdruckmanschette vorgestellt, die es ermöglicht für alle Armgrößen eine Manschette zu verwenden, die sich je nach Armdurchmesser anpassen kann und so eine genauere Messung ermöglicht.

Gelöst wird die Aufgabe unter anderem durch ein Verfahren zur nichtinvasiven, kontinuierlichen Blutdruckmessung bestehend aus einer Kombination aus

mindestens zwei beaufschlagten Blutdruckmessung zu unterschiedlichen Atemzuständen und/oder Höhenlagen des Messpunkts zum Herzen, insbesondere einer Blutdruckverlaufsmessung und/oder mit einem Blutdruckmanschettengerät, und

einer nicht beaufschlagten kontinuierlichen Messung der Pulswellenlaufzeit, der Pulswellengeschwindigkeit, der Pulswellenkontur und/oder des Blutdrucks, insbesondere mit einem herkömmlichen Blutdruckmanschettengerät,

wobei die beaufschlagte und die nicht beaufschlagte Messungen am selben Lebewesen, dadurch gekennzeichnet, dass spätere nicht beaufschlagte Messungen des Blutdrucks, der Pulswellenlaufzeit, der Pulswellengeschwindigkeit und/oder der Pulswellenkontur durchgeführt werden und die Messwerte der späteren nicht beaufschlagten Messungen mittels der bei der beaufschlagten Blutdruckmessung erhobenen Daten in mindestens einen Blutdruckwert umgerechnet werden.

Unter einer beaufschlagten Messung ist insbesondere eine solche zu verstehen, bei der Druck auf den Köper und/oder das blutführende Gefäß ausgeübt wird, insbesondere ein Druck der größer ist als der Lympfdruck., der typisch im Bereich von 3-5 mmHg liegt und/oder der Druck größer ist als 10 mmHg oder 1350Pascal. Unter einer nicht beaufschlagten oder unbeaufschlagten Messung ist insbesondere eine solche zu verstehen, bei der kein Druck auf den Köper und/oder das blutführende Gefäß ausgeübt wird, insbesondere maximal ein Druck der kleiner ist als der Lympfdruck, der typisch im Bereich von 3-5 mmHg liegt und/oder der Druck kleiner ist als 10 mmHg oder 1350 Pascal, insbesondere kleiner ist als 5mmHg oder 700Pascal. Dabei wird insbesondere der Druck betrachtet mit dem das Messmedium oder der Sensor auf die Haut gedrückt wird und/oder mit dem das Blutgefäß abgedrückt wird.

Erfindungsgemäß werden, insbesondere zur Kalibrierung, mindestens zwei beaufschlagten Blutdruckmessung zu unterschiedlichen Atemzuständen und/oder Höhenlagen des Messpunkts zum Herzen durchgeführt und mindestens zwei nicht beaufschlagte, insbesondere eine kontinuierliche, Messung der Pulswellenlaufzeit, der Pulswellengeschwindigkeit, der Pulswellenkontur und/oder des Blutdrucks durchgeführt. Unter kontinuierlich ist insbesondere eine Wiederholung spätestens alle 30 Sekunden, insbesondere spätestens alle 10 Sekunden, insbesondere mindestens jede Sekunde, insbesondere mindestens jede halbe Sekunde und/oder insbesondere mindestens ein, insbesondere mindestens zwei, Mal pro Herzschlag zu verstehen. Insbesondere werden bei einer kontinuierlichen Messung für jeden Herzschlag mindestens zwei Werte, beispielsweise systolischer und diastolischer Blutdruck, für den Blutdruck, die Pulswellenlaufzeit und/oder Pulswellengeschwindigkeit ermittelt. Unter kontinuierlich ist insbesondere auch mindestens eine Messung alle X Sekunden für mindestens X/10, insbesondere für mindestens X/2, Stunden zu verstehen. Wobei X insbesondere über 0,01 und/oder unter 60 liegt.

Erfindungsgemäß werden darüber hinaus danach weitere nicht beaufschlagte Messungen des Blutdrucks, der Pulswellenlaufzeit, der Pulswellengeschwindigkeit und/oder der Pulswellenkontur durchgeführt. Die Messwerte der weiteren nicht beaufschlagten Messungen werden mittels der bei der beaufschlagten Blutdruckmessungen und insbesondere auch bei der zur Kalibrierung durchgeführten nicht beaufschlagten Messungen erhobenen Daten in mindestens einen Blutdruckwert, insbesondere einen Blutdruckwert je weiterer Messung und/oder Herzschlag, umgerechnet. Insbesondere dadurch, dass eine, insbesondere lineare, Beziehung zwischen Blutdruck Pulswellenlaufzeit, der Pulswellengeschwindigkeit und/oder der Pulswellenkontur angenommen wird, kann eine Umrechnung erfolgen. So kann beispielsweise nach der Kalibrierung mittels einer nicht beaufschlagten optischen Messung der Pulswellenlaufzeit, Pulswellengeschwindigkeit und/oder der Pulswellenkontur mindestens ein Blutdruckwert errechnet werden.

Gelöst wird die Aufgabe unter anderem auch durch ein Vorrichtung zur nichtinvasiven, kontinuierlichen Blutdruckmessung aufweisend Mittel zur Durchführung einer nicht beaufschlagten kontinuierlichen Messung von Pulswellenlaufzeit, Pulswellengeschwindigkeit und/oder Pulswellenkontur und/oder Blutdruck, insbesondere mit einem herkömmlichen Blutdruckmanschettengerät, dadurch gekennzeichnet, dass die Vorrichtung eingerichtet ist, Messwerte von mindestens zwei beaufschlagten Blutdruckmessung zu unterschiedlichen Atemzuständen und/oder Höhenlagen des Messpunkts zum Herzen, insbesondere einer Blutdruckverlaufsmessung, entgegenzunehmen und die entgegengenommenen Messwerte der beaufschlagten Messungen und die nicht beaufschlagte Messungen zwecks Kalibrierung gemeinsam zu verarbeiten und

zahlreiche weitere nicht beaufschlagte Messungen des Blutdrucks, der Pulswellenlaufzeit, der Pulswellengeschwindigkeit und/oder der Pulswellenkontur durchzuführen und die zahlreichen weiteren nicht beaufschlagten Messungen des Blutdrucks, der Pulswellenlaufzeit, der Pulswellengeschwindigkeit und/oder der Pulswellenkontur jeweils mittels der gewonnen Kalibrierung in jeweils mindestens einen Blutdruckwert umzurechnen und diesen auszugeben. Die Kalibrierung wird insbesondere in vorgegebenen oder aus Messwerten ermittelten Abständen wiederholt, insbesondere frühestens nach einer Stunde, insbesondere frühestens nach sechs Stunden.

Gelöst wird die Aufgabe unter anderem durch ein Vorrichtung oder System zur nichtinvasiven, kontinuierlichen Blutdruckmessung bestehend aus einer erfindungsgemäßen Vorrichtung und Mitteln zur Erfassung und Ausgabe, insbesondere an die erfindungsgemäße Vorrichtung, von mindestens zwei beaufschlagten Blutdruckmessungen zu unterschiedlichen Atemzuständen und/oder Höhenlagen des Messpunkts der Blutdruckmessung zum Herzen, insbesondere einer Blutdruckverlaufsmessung.

Gelöst wird die Aufgabe unter anderem auch durch ein Verfahren zur Kalibrierung von Ergebnissen der Messung der Pulswellenlaufzeit, Pulswellengeschwindigkeit und/oder Pulswellenkontur bei einem Lebewesen zum Erhalt von kontinuierlichen Werten des Blutdrucks, dadurch gekennzeichnet, dass zu mindestens zwei, insbesondere mindestens vier, unterschiedlichen Blutdruckwerten, insbesondere genommen zu unterschiedlichen Atemzuständen, des Lebewesens die, insbesondere zeitlich, dazugehörigen Pulswellenlaufzeiten, Pulswellengeschwindigkeiten und/oder Pulswellenkontouren verwendet und/oder erhoben werden.

Gelöst wird die Aufgabe unter anderem durch ein Verwendung des Einflusses der Atmung und/oder unterschiedlichere Höhenlagen des Messpunkts einer Blutdruckmessung zum Herzen auf den Blutdruck zur Kalibrierung einer Messung der Pulswellenlaufzeit, der Pulswellengeschwindigkeit und/oder der Pulswellenkontur zur Berechnung eines Blutdrucks aus Messwerten der Pulswellenlaufzeit, der Pulswellengeschwindigkeit und/oder der Pulswellenkontur.

Mit Vorteil wird bezüglich eines erfindungsgemäßen Verfahrens, einer erfindungsgemäßen Vorrichtung, einer erfindungsgemäßen Verwendung oder eines erfindungsgemäßen System nachfolgendes Verfahren und/oder nachfolgende Vorrichtung, insbesondere zur beaufschlagten, Blutdruckmessung, verwendet, die auch allein eine Lösung der Aufgabe darstellen.

Gelöst wird die Aufgabe unter anderem durch ein Verfahren zur, insbesondere nicht invasiven und/oder kontinuierlichen, Blutdruckmessung, wobei mittels mindestens einem Druckaufnehmer eine durch den Blutdruck hervorgerufener Druckschwankung kontinuierlich erfasst wird, wobei mindestens zwei Blutdruckwerte aus den erfassten Druckschwankungen ermittelt werden, dadurch gekennzeichnet, dass der Einfluss der Atmung auf die ermittelten Blutdruckwerte reduziert wird, indem aus den kontinuierlich Druckschwankungen ein Einfluss der Atmung auf die Schwankung ermittelt wird und/oder Atemzustände ermittelt werden und die Blutdruckwerte aus den mittels des Druckaufnehmers erfassten Werten abgeleitet werden, die zu einem vorbestimmten und/oder gleichen Atemzustand erfasst wurden.

Gelöst wird die Aufgabe unter anderem durch ein Vorrichtung zur, insbesondere nicht invasiven und/oder kontinuierlichen, Blutdruckmessung, aufweisen mindestens einem Druckaufnehmer zur kontinuierlichen Erfassung einer durch den Blutdruck hervorgerufener Druckschwankung, wobei die Vorrichtung eingerichtet ist, mindestens zwei Blutdruckwerte aus den erfassten Druckschwankungen zu ermitteln und auszugeben, dadurch gekennzeichnet, dass die Vorrichtung eingerichtet ist, den Einfluss der Atmung auf die ermittelten Blutdruckwerte zu reduzieren, indem sie aus den erfassten kontinuierlich Druckschwankungen ein Einfluss der Atmung auf die Schwankung und/oder Atemzustände ermittelt und die Blutdruckwerte aus den mittels des Druckaufnehmers erfassten Werten ableitet, die zu einem vorbestimmten und/oder gleichen Atemzustand erfasst wurden, insbesondere die Werte aus den mittels des Druckaufnehmers erfassten Werten als die Blutdruckwerte verwendet, die zu einem vorbestimmten und/oder gleichen Atemzustand erfasst wurden.

Mit Vorteil werden bezüglich eines erfindungsgemäßen Verfahrens, einer erfindungsgemäßen Vorrichtung, einer erfindungsgemäßen Verwendung oder eines erfindungsgemäßen System Pulswellenlaufzeit, Pulswellengeschwindigkeit und/oder Pulswellenkontur für unterschiedliche Teile der Pulsdruckwelle, wie z.B. für Diastole, für Systole oder Reflexionswelle, unabhängig voneinander bestimmt, verwendet und/oder auf den Blutdruck kalibriert und/oder wird die Pulswellenlaufzeit, die Pulswellengeschwindigkeit und/oder die Pulswellenkontur mittels, Luftdruckmanschette, Plethmographieeinheit und/der oder Beaufschlagungssensor bestimmt.

Mit Vorteil erfolgen bezüglich eines erfindungsgemäßen Verfahrens, einer erfindungsgemäßen Vorrichtung, einer erfindungsgemäßen Verwendung oder eines erfindungsgemäßen System die beaufschlagten Kalibrationsmessungen und/oder Blutdruckmessungen und nicht beaufschlagten Kalibrationsmessungen, insbesondere Messungen, der Pulswellenlaufzeit, Pulswellengeschwindigkeit und/oder Pulswellenkontur, zeitnah, zeitgleich und/oder zu einem ähnlichen Atemzustand.

Die zeitnahe und/oder zeitgleiche Durchführung betrifft die Kalibrationsmessungen, insbesondere nicht erfasst sind davon die weiteren und/oder späteren Messungen, die mittels der Kalibration umgerechnet werden. Aber bezüglich aller dieser Messungen ist es von Vorteil, diese zu einem ähnlichen Atemzustand genommen werden. Dabei können und sollen unterschiedliche Messreihen zu unterschiedlichen Atemzuständen genommen werden. Insbesondere wird für jede dieser Messreihen eine eigene Kalibration zu dem jeweiligen Atemzustand vorgenommen.

Als Atemzustand kann beispielsweise die prozentuale Angabe der Einatmungsmenge oder -zeit oder Ausatmungsmenge oder -zeit oder Lungenfüllung verwendet werden. Dabei kann sich die Angabe auf ein absolutes Maximum oder ein Maximum aus einer definierten Messzeit beziehen. Bei der Bestimmung des Atemzustandes beziehungsweise eines Wertes zu einem Atemzustand wird bevorzugt eine gewisse Toleranz angewandt, weil ansonsten unverhältnismäßig lange Wartezeiten auftreten können, bis eine entsprechende Korrelation von Herzfunktion und Atemzyklus (wieder) eintritt. Diese Toleranz kann in unterschiedlichen Größen gemessen werden, beispielsweise in Prozent der Ein- und/oder Ausatemmenge, der Dauer eines Atemzyklusses und/oder der Dauer eines Herzschlages. Bevorzugt wird eine Toleranz (absolute Spanne) von maximal 1,5, insbesondere maximal 1, mal der Dauer eines Herzschlages, insbesondere RR Intervalls und/oder 30%, insbesondere 15%, der Ein- und/oder Ausatemmenge und/oder 30%, insbesondere 15% der Dauer eines Atemzyklusses. Bevorzugt wird dabei jedoch versucht, die Abweichung so gering wie möglich zu halten.

Mit Vorteil erfolgen bezüglich eines erfindungsgemäßen Verfahrens, einer erfindungsgemäßen Vorrichtung, einer erfindungsgemäßen Verwendung oder eines erfindungsgemäßen System und/oder die beaufschlagten Kalibrationsmessungen und/oder Blutdruckmessungen und nicht beaufschlagten Kalibrationsmessungen, insbesondere der Pulswellenlaufzeit, Pulswellengeschwindigkeit und/oder Pulswellenkontur an unterschiedlichen Punkten am Körper des Lebewesen, wobei die Punkte insbesondere so gewählt sind, dass eine sich von oder zum Herzen erstreckende Blutbahn die Punkte nacheinander erreicht.

Mit Vorteil wird bezüglich eines erfindungsgemäßen Verfahrens, einer erfindungsgemäßen Vorrichtung, einer erfindungsgemäßen Verwendung oder eines erfindungsgemäßen System nach einer Kalibration die Beaufschlagung abgebaut und werden weitere unbeaufschlagte, insbesondere kontinuierliche, Messungen, insbesondere der Pulswellenlaufzeit, Pulswellengeschwindigkeit und/oder Pulswellenkontur, durchgeführt, insbesondere für mindestens 30 min, insbesondere mindestens 1 Stunde, insbesondere mindestens 6 Stunden, insbesondere mindestens 12 Stunden, insbesondere mindestens 24 Stunden, insbesondere mindestens alle fünf Minuten, insbesondere mindestens alle zwei Minuten, insbesondere mindestens alle 60 Sekunden, insbesondere mindestens alle 20 Sekunden, insbesondere ohne zwischenzeitlich eine Beaufschlagung oder beaufschlagte Messung durchzuführen.

Mit Vorteil wird bezüglich eines erfindungsgemäßen Verfahrens, einer erfindungsgemäßen Vorrichtung, einer erfindungsgemäßen Verwendung oder eines erfindungsgemäßen System eine Veränderung der Lage eines Messpunkts zum HIP und/oder zum Herzen durch einen Lage- und/oder einen Beschleunigungssensor erfasst und insbesondere zur Korrektur der Messungen verwendet.

Mit Vorteil wird bezüglich eines erfindungsgemäßen Verfahrens, einer erfindungsgemäßen Vorrichtung, einer erfindungsgemäßen Verwendung oder eines erfindungsgemäßen Systems die Pulswellenlaufzeit aus der Pulswellenkontur ermittelt.

Mit Vorteil wird bezüglich eines erfindungsgemäßen Verfahrens, einer erfindungsgemäßen Vorrichtung, einer erfindungsgemäßen Verwendung oder eines erfindungsgemäßen System zur beaufschlagten Blutdruckmessung eine Luftdruckmanschette verwendet wird, die einen Sensor zur Bestimmung des Armdurchmessers aufweist, insbesondere einen Biegesensor, einen kapazitiven und/oder induktiven Sensor und/oder ein Sensor verwendet, welcher auf der Technologie capacitive touch beruht und/oder ist die Luftdruckmanschette so ausgebildet, dass sie stufenweise geschlossen wird und/oder in regelmäßigen Abständen Elemente in die Luftdruckmanschette eingebracht sind, die von dem Sensor eindeutig identifiziert werden können und/oder ist ein Luftsack der Luftdruckmanschette in mehrere Kammern aufgeteilt und wird insbesondere eine aktive Fläche, insbesondere Andruckfläche, des Luftsacks der Luftdruckmanschette durch Hinzu- oder Wegschalten von Kammern mittels elektrisch schaltbaren Ventilen auf den Armdurchmesser angepasst, wobei nicht geschaltete Kammern während der Messung nicht mit Luft befüllt werden und/oder ist die Luftdruckmanschette so ausgebildet, dass eine aktive Fläche, insbesondere Andruckfläche, des Luftsacks der Luftdruckmanschette durch zwei Kammern, die insbesondere durch Riemen aneinander gehalten werden, eingestellt werden kann, indem eine erste der zwei Kammer zur Blutdruckmessung verwendet wird und eine zweite der zwei Kammer zur Verformung der ersten Kammer verwendet wird, insbesondere indem diese durch Druckänderung die Abschnürung der ersten Kammer durch die Riemen verändert.

Mit Vorteil werden bezüglich eines erfindungsgemäßen Verfahrens, einer erfindungsgemäßen Vorrichtung, einer erfindungsgemäßen Verwendung oder eines erfindungsgemäßen System auf Basis der Messungen von Herzaktionen, insbesondere des Blutdrucks und/oder insbesondere des Pulses, insbesondere unbeaufschlagten Messungen, insbesondere weiteren Messungen Geräte gesteuert und/oder Steuer- und/oder Handlungsanweisungen ausgegeben werden, diese Geräte können bspw. automatisierte Medikationssysteme, wie Medikamentenpumpen, Beatmungsmaschinen, Notrufsysteme oder auch, Verkehrsmittel sein, die dann einen automatisierten Notruf absetzten können und/oder auch autonom fahrende Verkehrsmittel, insbesondere Fahrzeug, sein, die insbesondere bei kritischen Herzzuständen autonom reagieren. können, indem insbesondere eine Warnung an den Nutzer ausgegeben wird und/oder insbesondere ein Notruf ausgelöst wird, insbesondere das Fahrzeug an den Fahrbahnrand gefahren wird und/oder insbesondere eine Fahrt zu einem, insbesondere zum nächst gelegenen, Krankenhaus eingeleitet wird, wobei vorteilhaft die zulässige Höchstgeschwindigkeit überschritten wird, was insbesondere durch Signalisierung des Fahrzeugs an andere Verkehrsteilnehmer, insbesondere durch Licht-, Ton- und/oder Funksignale risikoarm gestaltet werden kann.

Gelöst wird die Aufgabe auch durch ein entsprechend ausgestattetes, insbesondere autonomes, Verkehrsmittel, insbesondere Fahrzeug sowie durch ein System aus mindestens einer erfindungsgemäßen Vorrichtung und einem solchen Verkehrsmittel.

Eine Recheneinheit zur Auswertung der gemessenen Rohdaten kann dabei beispielsweise wahlweise im Verkehrsmittel und/oder im Mittel zur Messung der Herzaktionen angeordnet sein. Die Mittel zur Messung können insbesondere in dieser Schrift beschriebene Geräte und/oder Gerätekombinationen sein.

Neben der Aufzeichnung von einzelnen Werten des Blutdrucks kann ein Tagesprofil (vgl. Fig. 8, 10 und 11) erstellt werden. Ein solches Tagesprofil kann auch zur weiteren Analyse verwendet werden. So zeigen sich die Auswirkungen von Medikation (8.4, 8.6 und 8.8) und ob die Medikation die gewünschte Reaktion hervorruft (8.8 zeigt diesbezüglich eine Überdosierung). Auch die Effekte von Kaffee (8.3), Essen (8.5) oder Sport (8.7) können dargestellt werden. Kommt es zu Auffälligkeiten (z.B. 8.9) können diese auch zeitlich gut aufgelöst dargestellt werden.

Die Methodik zur Bildung des Tagesprofil kann nicht nur zur Aufzeichnung des Zustands des Herzkreislaufsystems verwendet werden, sondern kann auch genutzt werden, um Signale für Anweisungen an den Nutzer auszugeben (vgl. Beschreibung und Fig. 11). Diese Signale umfassen bspw. die Aufforderung, eine (vorher mit einem Arzt vereinbarte) Medikation durchzuführen, Flüssigkeit zu sich zu nehmen, eine sportliche Aktivität einzuschränken, die Nahrungsaufnahme zu regulieren oder weitere durch den Nutzer definierte Aktionen. Diese Signale für Anweisungen können dem Nutzer aufgrund der Blutdrucksituation ausgegeben werden und können durch Ton, Vibration oder visuelle Darstellung auf ein Display erfolgen. Ferner ist eine Information als (Push)Nachricht ans Smartphone möglich.

Heutige Systeme der indirekten Blutdruckmessung z.B. von Dräger oder Somnomedics verwenden die Pulswellenlaufzeit. Diese Systeme müssen mit einer aufwendigen Kalibration verwendet werden, bei der die zu untersuchende Person zunächst in Ruhe und danach im belasteten Zustand, also während oder nach einer sportlichen Aktivität, untersucht wird. Es ergibt sich bereits hier eine Fehlerquelle, da der Zustand Ruhe und Belastung nicht genau erreicht werden können bzw. nur vage definiert sind. Nach der Kalibration kann die Pulswellenlaufzeit, welche bspw. mittels EKG und Plethysmographie ermittelt wird, zu einem Blutdruckpaar, also beispielsweise einem Zahlentupel von systolischem und diastolischem Blutdruck zugeordnet werden.

Auch eine Verbesserung der Kalibration kann durch den Einsatz der "Redtel Methode", wie in der PCT/EP2018/056275 Anmeldung beschrieben, erreicht werden.

Ziel der Kalibration anhand eines Zustands in Ruhe und in Belastung ist es, unterschiedliche Blutdrücke zu erhalten und die Auswirkungen auf die Ausgangswerte der Plethysmographie daraufhin zu untersuchen. Die "Redtel Methode" erlaubt es auch kleinste Änderungen im Blutdruck, welche natürlich auftreten, z.B. durch die RSA (Atmung), zu erkennen. Diese kleinsten Änderungen sind ausreichend für eine Kalibration. Anstatt der Kalibrierungen des Blutdrucks in Ruhe und in Belastung, können Blutdruckwerte, welche mit der "Redtel Methode" ermittelt werden, insbesondere von einzelnen Herzschlägen während der Einatmung bzw. Ausatmung verwendet werden. Dies ist insbesondere möglich, da eine Vielzahl von Blutdruckwertepaaren gemessen werden, sodass auch eine Mehrfachkalibration möglich ist. Da die RSA erkannt werden kann, kann eine automatische Kalibration durchgeführt werden, sodass keine aufwendige Berechnung oder Abschätzung durch den Nutzer nötig ist. Die Kalibrierung wird nicht nur einfacher, sie wird insbesondere präziser und erzeugt damit ein verbesserte Blutdruckmessung/ Abschätzung über die Messung der Pulswellenlaufzeit. Auch ist es möglich einzelne Blutdruckwerte für die Kalibration zu nutzen und zum Beispiel für systolischen Blutdruck und diastolischen Blutdruck separat zu kalibrieren. Dabei können auch unterschiedliche Abschnitte der Pulswellenkontur verwendet und/oder systolischem und diastolischem Blutdruck unterschiedliche Pulswellenkonturen, Pulswellenlaufzeiten und/oder -geschwindigkeiten zugeordnet werden.

## 5. weitere Beschreibung der Erfindung

Das Problem der oben beschrieben PCT/EP2018/056275 Patentanmeldeschrift hier genannt "Redtel Methode" besteht darin, dass eine präzise kontinuierliche Blutdruckmessung mit der benötigten Beaufschlagung nur zeitlich befristet durchgeführt werden kann.

Eine venöse Stauung, vielmehr jedoch der Lymphdruck, verhindern eine durchgehende Blutdruckmessung über 24 Stunden. Folgend wird aufgezeigt, wie die Redtel Methode verwendet werden kann um über einen längeren Zeitraum präzise kontinuierlich und mit minimalen Schmerzen für den Nutzer den Blutdruck zu messen.

## 5.1 Ermittlung des Blutdrucks aus der Pulswellenlaufzeit

Die in dieser Patentschrift beschriebene Erfindung beschreibt auch eine Kombination aus der Methode der kontinuierlichen Beaufschlagung mit einer herkömmlichen Blutdruckmanschette, auch mittels Armband oder Uhr und der Messung mit Hilfe von Licht, wie bspw. bei einer Plethysmographie oder mit smarten Geräten, wie Uhren bspw. von Polar, Apple oder auch einfachen Smartphonegeräten, die dieses Problem lösen.

Die Erfindung besteht beispielsweise aus zwei Teilen, welche entweder getrennt oder in einem Gerät vereint sind.

Dies ist zum einen ein Mittel zur Messung des Blutdrucks nach der "Redtel Methode" und zum anderen ein Mittel zur, insbesondere nicht beaufschlagten, Messung der Pulswellenlaufzeit, - geschwindigkeit und/oder -kontur.

Die Wahl der Messorte für die nicht beaufschlagte der Pulswellenlaufzeit, -geschwindigkeit und/oder -kontur ergibt unterschiedliche Ergebnisse, welche bezüglich der Position interpretiert werden soll. Vom Herzen aus nimmt die Pulswellengeschwindigkeit mit der Entfernung zu. So liegt die Geschwindigkeit in der Aorta bei gesunden Menschen zwischen 4 und 6 m/s und in den Fingern zwischen 10 und 12 m/s.

Eine Messung der Pulswellengeschwindigkeit ist also immer eine Mittlung über die Geschwindigkeiten, welche sich in den Arterien zwischen den Messpunkten einstellen.

Folgende konkreten Ausgestaltungen zur Bestimmung der Pulswellenlaufzeit sind beispielsweise möglich:

- zwei Geräte beruhend auf der, insbesondere verbesserten, Redtel Methode
- insbesondere verbesserte, Redtel Methode und EKG
- insbesondere verbesserte, Redtel Methode und Plethysmographie
- insbesondere verbesserte, Redtel Methode und beabstandete Messung der Pulswelle

• EKG und Plethysmographie
• zwei Plethysmographien

Einige dieser möglichen Ausgestaltungen sind in Fig. 1 und 9 dargestellt.

Eine Messung mit Licht (Plethysmographie oder beabstandete Messung) benötigt keine Druck Beaufschlagung.

Die Daten der weiteren Messung zur Bestimmung der Pulswellenlaufzeit und/oder - geschwindigkeit und die Daten der, insbesondere verbesserten, "Redtel Methode" werden in einer Recheneinheit zusammengeführt. Die Daten müssen dabei zeitlich aufeinander abstimmbar sein. Dabei kann die Recheneinheit auch in einem medizinischen Überwachungsgerät integriert sein. Auch die Verwendung anderer Recheneinheiten, wie z.B. ein Smartphone sind möglich.

Diese neue erfinderische kontinuierliche Blutdruckmessmethode kann an einer oder an mehreren Extremitäten in mehrfacher Ausführung befestigt werden und bspw. zur Erkennung von Stenosen oder auch zur Erkennung von Arteriosklerose eingesetzt werden. Ferner ist auch die Früherkennung möglich, da die Pulswellenlaufzeit auch durch Gefäßsteifigkeiten aufgrund von Plaque Ablagerungen, welche sich zu den Verschlusskrankheiten entwickeln können, verändert wird.

Bei einer beabstandeten Messung, beispielsweise mit einer Kamera, können Befestigungen am Körper entfallen. Auch kann damit flexibel an unterschiedlichen Orten untersucht werden, bei Aufnahme ausreichend großer Areale kann auch eine Umpositionierung der Kamera entfallen, da ganzheitlich jede Fläche gemessen werden kann, die als Haut zur Blutdruckmessung frei liegt.

### 5.1.1 Zwei Geräte beruhend auf der, insbesondere verbesserten, Redtel Methode

Werden zwei Geräte beruhend auf der, insbesondere verbesserten, Redtel Methode an einem Arm oder an einem Bein angebracht so können zwei unabhängige Blutdruckwellen aufgezeichnet werden, welche je nach Abstand zu einander einen Laufzeitunterschied aufweisen. Dieser Laufzeitunterschied ist die Pulswellenlaufzeit und kann mit dem Abstand der Geräte untereinander in eine Pulswellengeschwindigkeit umgeformt werden.

Der Vorteil dieser Kombination ist, dass die erhobenen Wellen dem Druckverlauf in der Arterie entsprechen, so kann für jede Amplitude der Wellen eine unabhängige Pulswellengeschwindigkeit, -laufzeit und/oder -kontur ermittelt werden. Werden die lokalen Minima der beiden Wellen verglichen, so ergibt sich die Pulswellenlaufzeit, -geschwindigkeit und/oder -kontur für die Diastole, der Vergleich der lokalen Maxima ergibt die Laufzeit und/oder Geschwindigkeit der Systole. Ferner können auch die Ausprägungen der Reflexionswelle verfolgt werden und somit deren Geschwindigkeit bestimmt werden.

Nachteilig bei dieser Kombination ist die aufgrund der Belastung kurze Messzeit und die auf die Gliedmaßen beschränkten Messorte.

### 5.1.2 insbesondere verbesserte, Redtel Methode und EKG

Durch die Verwendung eines EKGs kann die Einschränkung der Messorte aufgehoben werden. Bei der Messung mittels EKG ergibt sich die Pulswellenlaufzeit vom Herzen (Startzeitpunkt) zur Manschette des Systems zur Messung des Blutdrucks nach der, insbesondere verbesserten, "Redtel Methode" (Endzeitpunkt).

Die Manschette kann beliebig an den Extremitäten befestigt werden.

Die Kombination der, insbesondere verbesserten, Redtel Methode mit einem EKG ist in Fig. 9.3 beschrieben und ein typischer Messverlauf ist in Fig 14 gezeigt.

Vorteilhaft bei dieser Verwendung ist, dass medizinisch relevante Informationen einfach erhoben werden können.

Die Pulswellenlaufzeit kann bspw. am rechten und am linken Fußgelenk entweder gleichzeitig mit zwei Manschetten oder nacheinander mit einer Manschette bestimmt werden.

Ergeben sich Unterschiede in der Laufzeit zwischen zwei Extremitäten, so ist dies ein Hinweis auf eine Verschlusskrankheit oder deren Entwicklung, wie z.B. Arteriosklerose oder eine Stenose.

Vorteilhaft ist die Verwendung eines EKGs, da nicht nur die Pulswellenlaufzeit bestimmt werden kann, sondern es auch möglich ist eine bessere Messung mit der Riva-Rocci Messung durchzuführen, indem die Atmung berücksichtigt wird.

Ist die Atmung bekannt können die Werte von Diastole und Systole der Phase der Atmung zugeordnet werden und eine verbesserte Kalibration ist möglich.

Wird eine Anordnung ohne EKG nur basierend auf der, insbesondere verbesserten, Redtel Methode verwendet, kann bereits die Atmung auch während einer Riva-Rocci Messung festgestellt werden. Fig. 4 und Fig 12 zeigen typische Druckverläufe in der Manschette bei einer Messung. Es ist bereits bei der Messung mit Riva-Rocci zu erkennen, dass kleine Variationen auf der ansteigenden Druckverlaufskurve zu erkennen sind. Diese rühren von der Herzaktion her und die Pulsintervalllänge kann auch hier für jeden Herzschlag bestimmt werden. Die Veränderung der Pulsintervalllänge rührt von der Atmung her (RSA - respiratorische Sinusarhythmie) und somit kann die Atmung festgestellt werden.

Vorteilhaft ist jedoch die Analyse des EKGs auf die Atmung hin, vgl. Fig. 14. Auch hier wird die Herzintervalllänge von z.B: R-Zacke zu R-Zacke für jeden Puls bestimmt, es ergeben sich die RR-Intervalle. Die Veränderung zwischen den einzelnen RR-Intervallen rühren von der Atmung her, sodass diese bestimmt werden kann.

Die Messung mittels Riva-Rocci bestimmt die Werte für Diastole und Systole jedoch zufällig in der Atmung (vgl. Fig. 5). Die Verbesserung der Messung erfolgt nun beispielsweise indem dieser Umstand protokolliert wird (vgl. Fig. 12-17). Es werden nicht nur die Werte für Diastole und Systole durch die Riva-Rocci Messung bestimmt sondern auch deren Messzeitpunkte. Somit ist eine Zuordnung der Werte von Diastole und Systole auf die Phase der Atmung möglich.

Wird nun die, insbesondere verbesserten, Redtel Methode mit den Daten der Riva-Rocci-Methode kalibriert unter der Rücksichtnahme der Atmung kann eine bessere Kalibration erreicht werden. Dazu werden die Werte für die Diastole/Systole in der gleichen Phase in der Atmung, wie bei Riva-Rocci Messung, mit den (ausgangs) Messwerten der Redtel Methode zur Kalibration verwendet.

Im Rückkehrschluss bedeutet dies auch, dass die Messung durch Riva-Rocci verbessert wurde. Eine Einzelwertmessung, vergleichbar zur heutigen Messung mittels Riva-Rocci, würde wie folgt ablaufen:

Messung mittels (heutiger) Riva-Rocci, danach Kalibration der Redtel Methode unter Berücksichtigung der Atmung und Messung mit der Redtel Methode (inklusive Kalibrationsphase) von mindestens einem Atemzug.

Es können jetzt für jeden Herzschlag in der Messphase ein Wertepaar von Diastole und Systole bestimmt werden.

Einfach darzustellende Ergebnisse wären bspw. die höchste Systole und die niedrigste Diastole in der Atmung, die Werte mit dem höchsten Pulsdruck oder die Werte von Systole und Diastole zu einem festen Punkt innerhalb der Phase der Atmung.

### 5.1.3 insbesondere verbesserte, Redtel Methode und Plethysmographie

Darüber hinaus kann jedoch auch alleinig der Plethysmographiesensor zur Blutdruckmessung verwendet werden. Ein Plethysmographiesensor strahlt Licht in das Gewebe ein, wobei ein Teil des Lichtes gestreut und zum Sensor zurückgestrahlt wird. Die Intensität des zurückgestreuten Lichts wird mit einem Lichtsensor erfasst. Dabei hängt die Lichtintensität von mehreren Faktoren ab, unter anderem auch vom Blutdruck. Wird die absolute Intensität des zurückgestreuten Lichts bestimmt, kann, mit einer Kalibrierung, der Blutdruck bestimmt werden. Wird die relative Intensitätsänderung des zurückgestreuten Lichts bestimmt, kann, eine Pulswellenkontur erfasst werden aus der ebenfalls mittels einer Kalibrierung, der Blutdruck bestimmt werden kann. In einer solchen Anordnung ist also nur das Gerät für die "Redtel Methode" und ein Plethysmographiesensor notwendig (vgl. Fig 1.1 und 1.2).

Die Plethysmographieeinheit kann dabei zum Beispiel auf zwei Arten verwendet werden. Zum einen kann die Pulswellenlaufzeit zwischen dem Ort der, insbesondere verbesserten, Redtel Methode und dem Ort der Plethysmographie bestimmt werden, dabei ergibt sich die Pulswellenlaufzeit von dem System zur Messung des Blutdrucks (bspw. Oberarm) nach der, insbesondere verbesserten, "Redtel Methode" (Startzeitpunkt) zum Ort der Plethysmographie, welche üblicherweise am Finger ist (Endzeitpunkt).

Zum Anderen können die Messwerte der Plethysmographie verwendet werden, um die Pulsdruckkurve abzubilden

### 5.1.4 insbesondere verbesserte, Redtel Methode und beabstandete Messung der Pulswelle

Bei der beabstandeten Messung wird aus einzelnen Bildern oder Bilderserien, bzw. "Live"-Videos anhand von kleinsten, dem menschlichen Auge unsichtbaren, Farbveränderungen der Haut eine Pulswelle erkannt. Diese Welle ist zeitlich über den Körper verschoben, je nach Pulswellenlaufzeit. Dies erlaubt die Analyse hinsichtlich Pulswellengeschwindigkeit aus der Pulswellenlaufzeit und dem Ort auf dem Körper.

Eine genaue Vorgehensweise einer beabstandeten Messung der Pulswellenlaufzeit kann in der Patentanmeldung mit dem Aktenzeichen DE 10 2018 002 268.5 entnommen werden. Eine mögliche Ausgestaltung mit beabstandeter Messung ist in Fig. 1.6 dargestellt.

Bei dieser Methode kann die Pulswellenlaufzeit für jeden Punkt auf der Körperoberfläche bestimmt werden und daher ist auch keine Mittelung über eine Wegstrecke nötig.

Die flächendeckende Messung der Pulswellenlaufzeit bzw. -geschwindigkeit erlaubt es durch eine Kalibration auch den Blutdruck flächendeckend zu bestimmen.

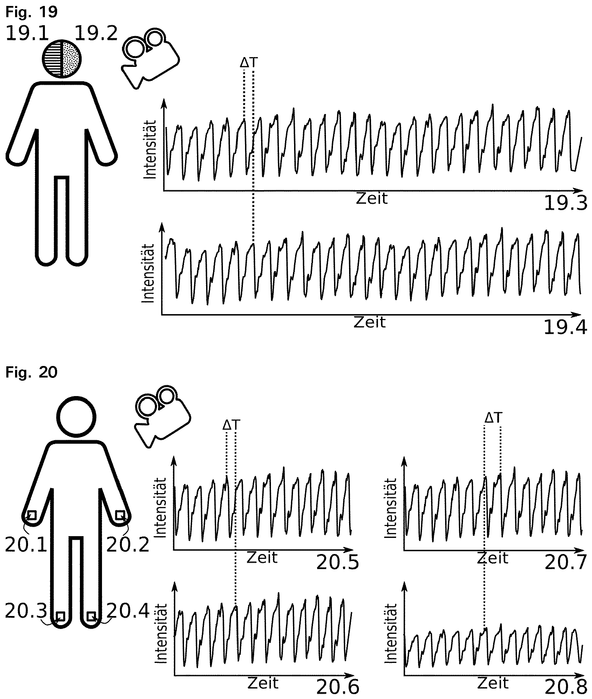

Unterschiede im Blutdruck, in der Pulswellengeschwindigkeit oder im Pulsdruck sind Hinweise auf Erkrankungen oder deren Entwicklung, wie z.B. Stenosen (vgl. Fig. 19) oder Arteriosklerosen (Fig. 18 und 20), deren Ort genau lokalisiert werden kann und deren Schwere abgeschätzt werden kann durch den Druckunterschied örtlich vor und nach der Verschlusskrankheit (vgl. Fig 18).

Eine Anordnung kann durch eine feststehende Kamera erfolgen, jedoch ist eine Verwendung einer Smartphonekamera vorteilhaft. Das Smartphone ist in einer solchen Anordnung die zentrale Steuereinheit und ist gleichzeitig für die beabstandete Messung zuständig. Die Daten anderer Messgeräte zur Abbildung von Herzfunktionen und/oder zur Durchführung einer beaufschlagten Messung, wie z.B. der, insbesondere verbesserten, Redtel Methode oder eines EKG werden mittels Funk, Schall und/oder optisch und/oder kabelgebunden an das Smartphone übertragen.

Dieser Anordnung ermöglicht es z.B. Verschlusskrankheiten einfach zu detektieren. Es wird bspw. am Handgelenk ein Gerät zur beaufschlagten Blutdruckmessung, insbesondere Messung nach, insbesondere verbesserter, Redtel Methode, angebracht. Dies ermöglicht die Kalibrierung der gemessenen Pulswellenlaufzeit auf den Blutdruck. Mit dem Smartphone werden potenzielle Regionen gescannt. Diese Regionen können bspw. die Beine und Füße sein. So können bspw. Diabetiker ihre Füße kontrollieren und die Bildung eines diabetisches Fußsymptom frühzeitig erkennen.

Im speziellen beim Diabetiker kann die Pulswellenlaufzeit für jeden Zeh im einzelnen bestimmt werden. Bei einem gesunden Menschen sind diese Laufzeiten nahezu gleich. Bei einer Verschlusskrankheit in einem Zeh kann diese durch eine unterschiedliche Pulswellenlaufzeit erkannt werden. Zur Bestimmung der Laufzeit kann die Position des jeweiligen Bildes und/oder des Messpunkts der beaufschlagten Messung erfasst werden, beispielsweise aus einem Bild, das mittels des Smartphones erstellt wird, durch Eingabe ins Smartphone und/oder durch eine Abstandsmessung, insbesondere funkbasiert.

### 5.1.5 Zwei oder mehr Messpunkte aus einer beabstandeten Messung

Bei einer beabstandeten Messung, wie im vorherigen Abschnitt beschrieben, kann nicht nur ein Punkt der Hautoberfläche vermessen werden. Es kann vielmehr jeder im Bild erfasste Punkt der Haut unabhängig von einander vermessen werden.

Zu jedem Punkt ergibt sich eine Welle, die den Pulsverlauf abbildet. Diese Wellen sind aufgrund der Pulswellenlaufzeit gegeneinander verschoben. Wird ein Punkt als Ausgangspunkt gewählt, so kann die Pulswellenlaufzeit zu jeden anderen Punkt durch die Verschiebung der Wellen zum Ausgangspunkt bestimmt werden.

In den Figuren 18-20 sind verschiedene Anwendungsbeispiele gezeigt, die verschiedene Analysemöglichkeiten aufweisen. Ziel dieser Analysen ist es Verschlusskrankheiten aufzudecken oder sogar bevor sie merklich auftreten zu erkennen.

Fig. 18 zeigt die Erkennung einer Stenose im Bein. Ein solcher Verschluss ist dadurch zu erkennen, dass keine Pulsung zu sehen ist.

Jedoch ist es sinnvoll eine solche Erkrankung nicht erst im Endstadium zu erkennen. Die Entwicklung einer Verschlusskrankheit beginnt mit der Veränderung der Gefäßsteifigkeit, was bspw. durch Plaque Ablagerung erfolgt. Die Veränderung der Gefäßsteifigkeit hat zur Folge, dass sich die Pulswellenlaufzeit verändert. Daher kann eine vergleichende Messung der Pulswellenlaufzeit dies aufdecken.

Fig. 19 zeigt diesbezüglich eine Messung der Gesichtshälften. Aufgrund der versorgenden Halsschlagadern sind die Pulswellen der Gesichtshälften gegeneinander zeitlich verschoben. Dies ist damit begründet, dass die beiden Halsschlagadern an unterschiedlichen Stellen aus der Aorta abzweigen. Die Verschiebung beträgt in der Regel 10 ms. Wird ein deutlich anderer Wert ermittelt, so kann dies auf die Entwicklung einer Verschlusskrankheit hinweisen.

Fig. 20 zeigt ein ähnliches Verfahren für die Extremitäten. Wird die Welle, gemessen an der Hand, mit der Welle, gemessen am Fuß, verglichen, so ergibt sich eine zeitliche Verschiebung. Bei einem gesunden Zustand der Arterien sollte dieser Unterschied auf beiden Seiten gleich sein. Wenn nicht, so ist dies wieder ein Hinweis auf eine sich entwickelnde Verschlusskrankheit.

Der Aufschluss, wo diese Erkrankung ist, kann erfolgen, indem die Wellen bspw. entlang der Beine (vergleichbar zu Fig. 18) analysiert werden. Es kann die Pulswellenlaufzeit im Bein vom Oberschenkel ausgehend Punkt zu Punkt hinab bis zu den Zehen gemessen werden. Durch den Abstand des Ortes des Punktes auf dem Bein zum Ort des ersten Punktes auf dem Oberschenkel, kann die Pulswellengeschwindigkeit bestimmt werden. Diese sollte kontinuierlich vom Oberschenkel zum Zeh zunehmen. Weißt der Verlauf auf einem Bein im Vergleich zum anderen eine oder mehrere unstetige Stellen auf, so sind diese Stellen höchstwahrscheinlich die Orte an denen Verschlusskrankheiten droht.

### 5.1.6 EKG und Plethysmographie

Die Messung der Pulswellenlaufzeit mit einem EKG und der Plethysmographie ist eine medizinisch validierte Methode, deren Ergebnisse durch Kalibration einem Blutdruck zugeordnet werden können. Diese beiden Messungen sind seit Jahrzehnten Alltag in der medizinischen Anwendung.

Die Anmeldung stellt dabei eine Verbesserung der Kalibration dar (vgl. Fig 9.4 und 16).

Eine heutige Kalibration besteht darin, dass zwei unterschiedliche Blutdrücke im Nutzer durch z.B. sportliche Aktivität eingestellt werden müssen. Durch die Lehre der vorliegenden Anmeldung reichen jedoch schon die Variationen des Blutdrucks aufgrund der Atmung (vgl. Fig. 5) oder durch die Bewegung der Messstelle bezüglich des HIPs, beispielsweise das Heben der Hand, wenn die Messung an der Hand erfolgt, zur Kalibration aus.

### 5.1.7 zwei Plethysmographien

Bei den bisher vorgestellten Methode (Abschnitt 5.1.2) wurde die Messung des Blutdrucks insbesondere mittels Riva-Rocci-Methode durch die Verwendung eines EKGs verbessert, mit dem die Atmung erfasst wurde. Jedoch kann mit der gleichen Methodik diese Verbesserung auch mit einem Plethysmographiesensor erreicht werden.

Ein möglicher Aufbau ist in Fig. 9.5 gezeigt und die Funktionsweise ist in Fig. 17 beschrieben.

Die Werte der Plethysmographie können hinsichtlich dem RR-Intervall untersucht werden. Wie beim EKG variiert das RR-Intervall von Schlag zu Schlag mit der Atmung, wodurch die Frequenz der Atmung ermittelbar wird.

Die Kalibration erfolgt insbesondere wieder im Bezug auf die Atmung, wie es bereits im Abschnitt 5.1.2 beschrieben wurde.

Da die beiden Plethysmographien voneinander beabstandet am Körper angebracht sind, so sind beide Kurven zueinander verschoben aufgrund der Pulswellenlaufzeit.

Darüber hinaus sind die beiden Kurven zueinander verformt. Diese Verformung kommt, neben Verzweigungen und Verengungen der Arterien, dadurch zustande, dass die Pulswellenlaufzeit eine vom Druck abhängige Größe ist. Somit bewegt sich der Wellenanteil der Systole schneller als der der Diastole, wodurch diese sich mit Entfernung vom Herzen immer weiter voneinander trennen.

Dies gilt auch für andere Komponenten der Welle, wie z.B. die Reflexionswelle.

Es kann also eine Pulswellenlaufzeit für die Diastole unabhängig gegenüber einer für die Systole bestimmt werden. Somit ist auch eine unabhängige Kalibration der Blutdruckwerte, insbesondere systolischer und diastolischer, möglich. Dadurch können anschließend kontinuierlich auch unabhängig voneinander systolsischer und diastolischer Blutdruck ermittelt werden.

### 5.1.8 Kalibrierung einer Photo-Plethysmographieeinheit

Die Abbildung der Pulsdruckwelle mit einer Plethysmographieeinheit beruht darauf, dass Licht einer bestimmten Wellenlänge oder eines Spektrums in Gewebe gesendet wird. Dort wird das Licht teilweise reflektiert und absorbiert.

Vorteilhafterweise wird eine Lichtwellenlänge gewählt, deren Reflektivität bzw. Absorbtionseigenschaften im Gewebe sich durch die Menge an Bluts in den Arterien verändert. Heutige Systeme verwenden bevorzugt die Farben infrarot, grün, rot und blau.

Dabei ist zu erkennen, dass die erhobenen Wellen an einem festen Ort am Körper nicht gleich (bzw. linear zueinander) sind und sich mit der Lichtwellenlänge verändern.

So zeigen Wellen aufgenommen mit grünem oder blauem Licht Wellenberge an Stellen, an denen eine Welle mit rotem Licht aufgenommen Wellentäler anzeigt.

Daneben ist auch zu erkennen, dass sich die Wellen der Redtel Methode auch von denen der Plethysmographieeinheiten unterscheiden.

Im generellen unterschieden sich alle Wellen, zu mindestens im Detail, von den verschiedenen Möglichkeiten, die die Herzfunktion abzubilden, und sogar Geräte, die auf dem gleichen Prinzip beruhen, können unterschiedliche Wellen ausgeben.

Die Verwendung der gleichen Lichtwellenlänge bei der Verwendung von zwei Plethysmographieeinheiten ist dabei sinnvoll, wenn die Einheiten getrennt voneinander am Körper angebracht werden. Die Wellen unterscheiden sich hierbei insbesondere nur durch die Unterschiede im Blutfluss von der einen zur anderen Messstelle, wobei die Erkennung dieser Unterschiede genau das Ziel einer Messung ist.

Es kann jedoch auch vorteilhaft sein, zwei verschiedene Lichtwellenlängen in zu verwenden. Wird eine kleine Baugröße benötigt, z.B. ein Armband ohne weitere Anbauten, so müssen die Plethysmographieeinheiten dicht beieinander liegen. Das Problem ist nun, dass bei einer gleichen Lichtwellenlänge sich die Signale überlagern können und dann beide Einheiten kein gutes Signal erfassen können.

Diesem Problem kann entgegengewirkt werden, wenn zwei verschiedene Lichtwellenlängen verwendet werden, es wird jedoch nun vorteilhafterweise ein Abgleich, Vergleich oder eine Kalibration zwischen den unterschiedlichen Messungen benötigt.

Wird eine Kombination aus verschiedenen Geräten gewählt, so sollte zunächst analysiert werden welche Bestandteile der Wellen miteinander zu vergleichen sind, um die Pulswellenlaufzeit zu bestimmen.

Prinzipiell gibt es hierfür zwei Herangehensweisen. Eine Möglichkeit ist es beide Geräte am gleichen Ort am Körper zu benutzen. So ergibt sich eine Pulswellenlaufzeit von Null. Werden die beiden Kurven nun übereinander gelegt, so liegen die zu vergleichenden Einflüsse der Herzfunktion auf die Messwertwellen auch übereinander. Es werden nun Punkte im Herzzyklus gesucht, die automatisiert zu erkennen sind. Dies sind bspw. lokale Minima und Maxima oder auch Wendepunkte.

Die andere Methode ist ähnlich, jedoch werden die Geräte an den Stellen angebracht an denen sie sich in der Anwendung auch befinden. Zusätzlich wird die Pulswellenlaufzeit mit einer anderen bereits abgeglichenen Messmethode gemessen, wobei die gleichen Messorte verwendet werden. Die Messwertwellen der beiden abzugleichenden Messgeräte werden nun wieder übereinandergelegt und nun um die Pulswellenlaufzeit gegeneinander verschoben. Es werden wieder automatisiert findbare Punkte herausgenommen und für die weitere Berechnung verwendet.

Eine andere Möglichkeit der Abstimmung speziell für Plethysmographieeinheiten, bzw. Kameras ist die Verwendung von Farbmusterkarten. Wird eine solche Karte abgefilmt, oder mittels Plethysmographie vermessen, kann anhand der Farben eine Zuordnung eine Empfindlichkeit der Messeinheit zu einer Lichtwellenlängen erfolgen. Durch hinterlegte Profile für Lichtwellenlängen kann ein Abgleich erfolgen.

Ist das Messsystem abgeschlossen, so muss dieser Abgleich nur in der Entwicklung erfolgen. Jedoch können diese Logiken auch bei offenen Systemen angewendet werden.

Ein offenes System besteht bspw. aus der Kombination eines Messsystems nach der, insbesondere verbesserten, Redtel Methode und einer Kamera eines Smartphones.

Mit der Kamera des Smartphone kann durch Auflegen eines Fingers auf Kamera und Blitzlicht die Pulswelle ermittelt werden, indem gleichzeitig das Blitzlicht im Dauermodus betrieben wird, also konstant angeschaltet ist. Das Licht durchdringt den Finger und wird, je nach Blutfluss, absorbiert und reflektiert und gelangt dann teilweise in die Kamera. Es ist ein Helligkeitsvariation mit der Pulswelle zu erkennen. Dabei sollte die Anordnung des Fingers auf dem Smartphone nicht verändert werden.

Jedoch ist die Anzahl von Kamera- oder Blitzlichtmodulen für Smartphones unüberschaubar, wobei jedes Modul leicht andere Eigenschaften und Empfindlichkeiten aufweist. Daher ist auch der Abgleich der Geräte durch den Nutzer sinnvoll.

### 5.1.9 Kalibrierung der Pulswellenlaufzeit auf den Blutdruck

Für die Kalibrierung der Pulswellenlaufzeit zur Ermittlung des Blutdrucks aus der Pulswellenlaufzeit wird die Pulswellenlaufzeit bei unterschiedlichen und bekannten Blutdrücken benötigt.

Heutige Verfahren lassen die zu vermessende Person eine sportliche Aktivität durchführen. Es werden der Blutdruck und die Pulswellenlaufzeit vor und während bzw. kurz nach der sportlichen Aktivität vermessen. Dabei werden dann Wertepaare von systolischem und diastolischem Druck mit der jeweiligen Pulswellenlaufzeit vergleichen.

Die hier vorgestellten Methoden benötigen keine sportliche Aktivität, da der Blutdruck sich auch natürlich immerwährend verändert und dies durch die, insbesondere verbesserte, Redtel Methode erkennbar ist.

Der Blutdruck verändert sich durch eine Höhenänderung der Messstelle. Wird eine Messung am Unterarm und im Stehen durchgeführt, kann der Blutdruck und die Pulswellenlaufzeit im gesenkten und bei in die Höhe gestrecktem Arm gemessen werden. Durch die Änderung der Höhe der Messstelle gegenüber dem HIP verändert sich der Blutdruck bzw. die Pulswellenlaufzeit. Derzeitig ist empirisch belegt, dass sich der Blutdruck für die Diastole mit $a\_d = 0.5$mmHg/cm und für die Systole mit $a\_s = 1$ mmHg/cm verändert.

Die Höhenänderung kann aufgrund der Armlänge, welche z.B. aus der Körpergröße gefolgert werden kann, bestimmt werden. Vorteilhafterweise werden jedoch die Methoden zur Bestimmung der richtigen Lage im nächsten Kapitels verwendet.

Ein- und Ausatmung verändern den RR-Intervall (RSA - respiratorische Sinusarrhythmie) bzw. die Pulsfrequenz, den Blutdruck sowie den Pulsdruck.

Die Einatmung beschleunigt den RR-Intervall, erhöht die Systole und senkt gleichzeitig die Diastole. Dadurch erhöht sich der Pulsdruck.

Das Gegenteil geschieht, wenn ausgeatmet wird.

Die Auswirkungen der RSA auf die Druckverlaufskurve des Blutdrucks ist in Fig. 5 gezeigt.

Vorteilhafterweise werden nicht Wertepaare aus systolischem und diastolischem Druck betrachtet sondern einzelne Druckwerte aus dem Verlauf der Blutdruckkurve.

Insbesondere durch die Messung jeder einzelnen Pulsdruckkurve für jeden Herzpuls über eine kurze Zeitspanne, insbesondere im Bereich der Dauer eines Atemzyklus und/oder von 1 bis 3 Atmenzyklen kann das System kalibriert werden. Dies geschieht insbesondere ohne die Fehlerquelle Mensch.

Heutige Methoden zur Ermittlung der Pulswellenlaufzeit betrachten die Verschiebung zweier Wellen zueinander, wie schon beschrieben z.B. vom EKG-Signal und der Welle eines Plethysmog rap hiesensors.

Dies stellt eine Mittlung dar. Die Pulswellenlaufzeit ist aber nicht über den gesamten Pulszyklus konstant, sondern ändert sich mit der Druckvariation innerhalb des Pulses. Dies erklärt unter anderem, warum die Druckkurven gemessen an unterschiedlichen Arterien sich unterscheiden. Der punktuelle Vergleich zweier Wellen z.B. anhand markanter Punkte ist in vielen Fällen ausreichend, jedoch kann eine bessere Kalibration erfolgen, wenn mehrere markante Punkte verglichen werden.

In Fig. 2 sind beispielhafte Messwerte einer Blutdruckkurve, welche mittels verbesserter "Redtel Methode" aufgenommen wurden und beispielhafte Messwerte eines Plethysmographiesensors gezeigt. Dabei war das Gerät, welches nach der verbesserten "Redtel Methode" arbeitet am Handgelenk angebracht und der Plethysmographiesensor an einem Finger der gleichen Hand angebracht. In Fig. 3 ist ein vergrößerter Ausschnitt der Daten gezeigt. Es zeigt sich, dass die Pulswellenlaufzeit mit steigendem Druck innerhalb eines Herzpulses von Diastole (Wellental) zur Systole (Wellenberg) abnimmt.

Heutzutage ist die Pulswellenlaufzeit ein Wert, der abhängig ist vom Messort und bestenfalls für jeden Puls bestimmt wird. Unter der Verwendung der, insbesondere verbesserten, "Redtel Methode" ist die Pulswellenlaufzeit nun auch ein kontinuierlich erfassbarer Wert.

Medizinisch validiert ist, dass die Pulswellengeschwindigkeit linear zu den Blutdruckwerten von Systole und Diastole ist. Dieser Zusammenhang ist jedoch von Mensch zu Mensch unterschiedlich und wird durch Medikamente, Erkrankungen, Flüssigkeiten und Nahrungsaufnahme, bzw. fehlende Flüssigkeiten und fehlende Nahrung und durch die Tagesform verändert.

Daher ist eine Kalibration nur für einen begrenzten Zeitraum ausreichend genau und sollte bei einer Veränderung wiederholt werden.

Für eine, insbesondere lineare, Kalibration müssen mindestens zwei unterschiedliche Wertepaare von Pulswellengeschwindigkeit Druck, beispielsweise die Werten von Systole und Diastole des Blutdruck und die Pulswellengeschwindigkeiten bei der Systole und Diastole ermittelt werden. Um jedoch die Genauigkeit zu erhöhen ist es vorteilhaft viele Wertepaare zu verwenden.

Die Wertepaare werden insbesondere linear genähert oder gefittet, insbesondere mittels, insbesondere linearer, Regression, es ergeben sich die Parameter A_s, B_s, A_d, B_d der linearen Gleichungen:

$$S\_BD = A\_s * PWG + B\_s$$

$$D\_BD = A\_d * PWG + B\_d,$$

wobei S_BD der systolische Wert, D_BD der diastolische Wert und PWG die Pulswellengeschwindigkeit sind.

Anstatt der Pulswellengeschwindigkeit kann auch der Kehrwert der Pulswellenlaufzeit verwendet werden, es ergeben sich lediglich andere Parameter (A_s, B_s, A_d, B_d ) bei der linearen Näherung.

Die heutzutage verwendete Kalibration erhebt ein Wertepaar in Ruhe und eins unter Belastung, wobei die Wertepaare jeweils aus drei Zahlen bestehen, nämlich zum einen dem systolischen und diastolischen Druck und zum anderen einer (gemittelten) Pulswellengeschwindigkeit oder - laufzeit. Da somit nur zwei Werte für die Kalibration vorliegen, schlägt sich jeder Messfehler extrem auf die Qualität der Kalibration aus. Zudem wird nicht zwischen der Pulswellengeschwindigkeit von Diastole und der von Systole unterschieden. Die zwei Werte Systole und Diastole werden heutzutage einem einzelnen Wert der Pulswellenlaufzeit zugeordnet und so kalibriert.

Bei der Verwendung von Pulswellenkonturen oder Blutdruckkonturen kann aus diesen eine Pulswellengeschwindigkeit und/oder -laufzeit, insbesondere an Hand der Reflexionswelle bestimmt werden und damit dann wie bezüglich der Kalibrierung der Pulswellengeschwindigkeit und/oder -laufzeit beschrieben verfahren werden.

Die in dieser Patentschrift vorgeschlagene Verwendung der Werte, die während der RSA vorliegen und für jeden Herzpuls erhoben werden können, stellen jedoch eine Vielzahl an Wertepaaren dar, wodurch der Messfehler an einem Wertepaar sich weniger stark auf das Ergebnis der Kalibration auswirken kann. Es gilt je länger kalibriert wird, umso besser wird die Kalibrierung, in der Praxis reichen Dauern im Bereich der Dauer eines Atemzyklus und/oder im Bereich von mindestens 1 Sekunde und/oder maximal 3s meistens aus.

Zur Kalibration kann ein Wert der Pulswellenlaufzeit bzw. Pulswellengeschwindigkeit für den jeweiligen Puls verwendet werden. Jedoch ist es vorteilhaft, wenn die Pulswellenlaufzeit bzw. Pulswellengeschwindigkeit zum Zeitpunkt der Systole und zum Zeitpunkt der Diastole für die Kalibration und bei der anschließenden Berechnung von Systole bzw. Diastole verwendet wird. Die Pulswellenlaufzeit ist auch eine sich kontinuierlich, innerhalb des Herzpulses veränderliche Variable, wie bereits beschrieben und in Fig. 3 dargestellt.

### 5.1.10 Problem der richtigen Lage

Bei der Wahl der Druckluftmanschette für die Riva-Rocci-Methode sind heutzutage zwei Varianten üblich.

Oberarm- und Handgelenk-Blutdruckmessgeräte besitzen dabei einen großen qualitativen Unterschied.

Der Oberarm kann sich gegenüber den HIP (hydrostatischer Indifferenzpunkt) nur begrenzt entfernen.

Der HIP ist ein Punkt, der sich unterhalb des Herzens befindet. Zur Einschätzung des Wertes muss der Blutdruck auf dieser Höhe bestimmt werden bzw. auf diese Höhe abgestimmt sein.

Bei Handgelenkblutdruckmessgeräten können

Unterschiede weit über 100mmHg durch Bewegung der Hand mit Messgerät von oberhalb nach unterhalb des eigentlichen Blutdrucks entstehen.

Dieses Problem bei Handgelenkmessungen, ob physisch oder mit Licht gemessen, kann durch Lage- und Beschleunigungssensoren ausgeglichen werden.

Während der Kalibrierung wird der Arm bzw. die Hand mit dem Messgerät bspw. vom HIP an den Oberschenkel gelegt und anschließend in einer halbkreisähnlichen Bewegung mit ausgestreckten Arm vom Oberschenkel über den Kopf gehoben.

Anschließend wieder zum HIP zurück. Nun ist die Lage des Messgerätes kalibriert und bekannt. Weitere Bewegungen können erkannt werden und der Wert des Blutdrucks kann unter Kenntnis der Lage zum HIP bestimmt werden.

Eine vorteilhaftere Bestimmung der Lage zum HIP ist gegeben, wenn die Entfernung vom HIP zum Messgerät bestimmt wird und die Orientierung des Messgeräts bekannt ist.

Die Orientierung ergibt sich beispielsweise aus den Daten eines Beschleunigungssensor. Wird dieser nicht bewegt, so zeigt dieser dennoch eine Beschleunigung an und zwar die Erdbeschleunigung, es ist also erkennbar, wie das Gerät (bei Stillstand) im Raum gedreht ist.

Da der Messort am Körper bekannt ist, z.B. am linken Handgelenk mit der Anzeige auf der Seite der Handinnenseite, ergibt sich bereits aus der Orientierung im Raum eine eingeschränkte Menge an möglichen Lagen zum HIP.

Weiter wird die Menge eingeschränkt durch die Armlänge, die über die Körpergröße aus statistischen Daten ermittelt werden kann.

Für eine Messung bei einem bewussten Nutzer sind diese Daten bereits ausreichend, um eine Plausibilitätsprüfung durchzuführen, um entsprechende Anweisungen zur richtigen Benutzung zu geben.

Jedoch ist es auch ein Ziel dieser Patentschrift aufzuzeigen, wie eine Anordnung während des Schlafens oder bei bewusstlosen Nutzern verwendet werden kann.

Um die genaue Lage zu bestimmen kann ein zusätzliches Gerät verwendet werden, welches auf die Brust an einer definierten Stelle geklebt wird.

Dieses Gerät verfügt über ein bzw. zwei Funkeinheiten und je nach Ausführung auch ein oder zwei Ultraschalleinheiten, außerdem ist ein Beschleunigungssensor zur Orientierungsbestimmung des zusätzlichen Geräts integriert.

Die Funk bzw. die Ultraschalleinheit wird verwendet, um die Entfernung zum Messgerät zu bestimmen. Im Falle der Verwendung der Funkeinheit, dies kann bspw. ein Sender auf dem ISM-Band 13.56 Mhz sein, wie z.B. eine RFID Einheit, wird z.B. die Intensität des Funksignals durch die Messeinheit gemessen. Dabei ist die Intensität abhängig von der Entfernung zwischen den Geräten und die Entfernung kann bestimmt werden.

Bei der Verwendung von Ultraschall wird, dem zusätzlichen Gerät eine Aufforderung durch das Messgerät geschickt, wodurch das zusätzliche Gerät einen kurzen Schallimpuls abgibt. Das Messgerät misst die Zeit t zwischen Aufforderung und dem Eintreffen des Schallimpulses. Die Entfernung d ergibt sich mit der Schallgeschwindigkeit c zu d = c*t.

Zwei Funkeinheiten bzw. Ultraschalleinheiten können werden verwendet, damit die Richtung zum Messgerät messbar wird.

Zusätzlich muss die Orientierung des zusätzlichen Geräts bekannt sein, daher übersendet das zusätzliche Gerät die Daten des Beschleunigungssensors, welche im Stillstand der Orientierung zum Erdboden entsprechen.

Der Abstand und die Richtung des Messgeräts zum Zusatzgerät kann aus den Abständen und der Orientierung des Zusatzgeräts zum Erdboden bestimmt werden.

Die bisherige Beschreibung des zusätzlichen Geräts ist insbesondere zu verwenden bei vollständiger Unkenntnis der Daten des Nutzers.

Sind jedoch die Daten Armlänge (z.B. über eine statistische Abschätzung aus der Körpergröße), Körperumfang an verschiedenen Stellen und Ort der Befestigung der Manschette bekannt und darüber hinaus die Art der Benutzung bekannt (z.B. bei Schlafen im liegen), kann eine einfachere Variante benutzt werden.

Diese Variante verfügt nur über einen Beschleunigungssensor, der die Orientierung und somit die Drehung des Körpers bei Schlafen erkennt.

Durch den Körperumfang z.B. an der Schulter kann die Höhe des HIPs zur Matratze bestimmt werden.

Darüber hinaus verfügt das Messgerät auch über einen Beschleunigungssensor. Die Orientierung des Messgeräts hängt von der Armhaltung ab und diese ist durch die Körperdrehung eingeschränkt, sodass sich aus der Körperdrehung nur eine mögliche Armhaltung ergibt, die die gemessene Orientierung des Messgeräts hervorrufen kann. Durch die Armhaltung und die Armlänge, kann auch die Höhe über der Matratze bestimmt werden. Die Differenz beider Höhen ergibt den Höhenunterschied des Messgeräts zum HIP.

**5.2 Bestimmung der Druckkurve aus Plethysmographie**

Diese bisher vorgestellte Kalibrierung ordnet der Pulswellengeschwindigkeit einen Wert für den Blutdruck zu. Diese Kalibrierung ist nicht vorteilhaft, um ein Abbild der Blutdruckwelle zu erhalten.

Dies kann erreicht werden, indem die, insbesondere absolute, Intensität von nach in das Gewebe eingestrahltem Licht empfangenem Licht ermittelt und mit einer Blutdruckwelle verglichen wird. Da der Ort der Lichtmessung und der Blutdruckmessung in der Regel unterschiedlich sind, ergibt sich auch ein zeitlicher Versatz von Wellenbergen bzw. -tälern in den Pulsdruckwellen/-kurven an den Messstellen.

Eine Kalibration erfolgt indem die Wellen zeitlich, um den Wert der Pulswellenlaufzeit zwischen den Messpunkten, aufeinander geschoben werden. Je nach Lichtfrequenz kann die Welle zur Blutdruckwelle umgekehrt sein und an den Stellen von Wellenbergen Täler in der Blutdruckwelle anzeigen.

Dieser Umstand ist jedoch irrelevant für die Kalibration.

Im Rahmen der möglichen auftretenden Blutdrücke ist eine lineare Kalibration möglich, sodass durch die Messwertpaare die Parameter C und D in der folgenden Gleichung durch lineare Regression bestimmt werden können:

$$P(t - PWL) = C * L(t) + D,$$

wobei P(t - PWL) der Druck in der Arterie zum Zeitpunkt als dieser Druckpunkt den Ort der Manschette passierte und L(t) die Lichtintensität zum Messzeitpunkt ist, P(t - PWL) und L(t) liegen zeitlich um den Wert der Pulswellenlaufzeit (PWL) zwischen den Messpunkten auseinander.

Nachdem eine oder beide Kalibrationen durchgeführt wurden, kann der Druck in der Luftdruckmanschette abgesenkt werden, sodass keine Beaufschlagung mehr vorliegt. Im weiteren Verlauf kann der Blutdruck über die Pulswellengeschwindigkeit, Pulswellenlaufzeit bzw. über die Lichtintensität mittels der Kalibration bestimmt werden.

Die Kalibration kann, wenn nötig, jederzeit wiederholt werden.

Ein Grund für eine Wiederholung kann eine zu starke Bewegung sein. Ein anderer kann eine über die normale Veränderung hinaus erfolgte Änderung der Lichtintensität sein.

Jedoch ist eine in regelmäßigen zeitlichen Abständen wiederholte Kalibration vorteilhaft.

**5.3 Ermittlung des Blutdrucks aus Elektrokardiogrammen**

Der absolute Wert der Spannungen von markanten Teilen eines EKGs beruht auf vielen Faktoren. Dabei lassen sich die Faktoren in zwei Gruppen einteilen. Zum einen beeinflussen die verwendeten Elektroden und die verwendete Hardware den Wert. Darüber hinaus hat die genaue Position der Elektroden auf der Haut und die Beschaffenheit der Haut großen Einfluss auf die Werte der Spannung.

Zum anderen hängt der absolute Wert der Spannung von dem aktuellen Wert anderer Vitalparameter ab. Hervorzuheben sind die Atmung und der Blutdruck.

Während eines Atmungszyklus unterliegt die absolute Spannung einer periodischen Veränderung und nimmt zu bzw. wieder ab. Die Absenkung geht bei schlechter Positionierung oder schlechter Hardware soweit herab, dass keine Funktionen des Herzens aus dem EKG ableitbar sind.

Diese Variation mit der Atmung kann herausgefiltert werden, indem z. B. nur eine Funktion des Herzens (z.b. R-Zacke) analysiert wird. Insbesondere werden dann während der Atmung nur solche Messwerte verwendet, die, welche sich in etwa (vergleiche die obenstehenden Angaben zur Toleranz) oder genau an der gleichen Position innerhalb der Atmung befinden, z.B. im vollständig eingeatmeten Zustand. Als praxistaugliche Näherung oder alternativ können auch in Bezug auf einen Atemzug ausgewählte Extremwerte verwendet werden, beispielsweise das Maxima der Spannung in einem Atemzyklus oder das Maxima der Spannung in einer R-Zacke in einem Atemzyklus. Dies funktioniert insbesondere, wenn die Atemfrequenz klein im Vergleich zur Herzfrequenz ist, was in der Regel gegeben ist. Natürlich können gleichzeitig aber separat mehrere verschiedene Funktionen und Positionen innerhalb der Atmung analysiert werden.

Nun Hängt die Veränderung der Spannung der ausgewählten Messwerte in einer Reihe, gebildet aus Messwerten einer Funktion und zu einem Atemzustand und/oder ein Extremwert in einem Atemzyklus in aufeinanderfolgenden Atemzyklen) von einem Atmungszyklus zum nächsten nur vom einer möglichen Blutdruckänderung ab.

Da mit der, insbesondere verbesserten, Redtel Methode für jeden Herzschlag, auch für jeden Herzschlag in einem Atmungszyklus, der systolische Blutdruckwert bestimmt werden kann, kann die Absolute Spannung einer Funktion des Herzens innerhalb einer festen Position des Atmungszyklus einem Blutdruckwert zugeordnet werden oder darauf kalibriert werden. Insbesondere wird zunächst der Blutdruck und gleichzeitig das EKG vermessen. Nun wird bspw. die absolute maximale Spannung der R-Zacke bestimmt. Ist diese bspw. die höchste in einem Atemzyklus so wird eine Vielzahl solcher Spannungen (maximale Spannung der R-Zacke in jedem Atemzyklus) mit der gleichen Anzahl von dazugehörigen systolischen Druckwerten, insbesondere linear, kalibriert, insbesondere mittels, insbesondere linearer, Regression.

Anschließend wird die Redtel Methode abgestellt und der Blutdruck kann über das EKG alleinig bestimmt werden.

**5.4 Optimierte Anordnung einer Luftdruckmanschette**

Für eine Blutdruckmessung nach dem Prinzip der Riva-Rocci-Methode wird eine Luftdruckmanschette um eine Gliedmaße gelegt. Die Breite und Länge der Manschette sollte jedoch auf den Armdurchmesser angepasst gewählt werden. Es werden heutzutage folgende Manschette für den Armdurchmesser empfohlen

    Armdurchmesser: (Breite x Länge)
    Unter 24 cm: 10x18 cm
    24-32 cm: 12-13x24 cm
    33-41 cm: 15x30 cm
    Über 41 cm: 18x36 cm

Häufig wird jedoch eine falsche Manschette gewählt. Wird eine zu kleine Manschette gewählt, so kann der Blutdruck überschätzt (bis zu 30 mmHg sind möglich) werden, es wird ein zu hoher Blutdruck ausgegeben.

Wird die Blutdruckmanschette zu groß gewählt eine Unterschätzung des tatsächlichen Blutdrucks (Fehler im Bereich von 10-30 mmHg sind möglich), es wird ein zu geringer Blutdruck ausgegeben.

Eine Optimierung erfolgt indem diese menschlichen Fehler bei der Wahl der Manschette durch eine technische Vorrichtung ausgeschlossen wird. Zusätzlich kann durch eine geeignete Vorrichtung auch die Anzahl der zur Verfügung stehenden Größenabstufungen der Manschette erweitert werden oder sogar eine kontinuierliche Einstellung erreicht werden.

Eine heutige Manschette besteht aus einem Luftsack, der in ein Gewebe eingebracht ist, sodass diese an einem Arm angebracht werden kann.

Die Optimierung, die eine eigenständige Erfindung darstellt, mit den anderen beschriebenen Lösungen und Ausgestaltungen aber auch vorteilhaft kombiniert werden kann, wird erreicht, indem in das Gewebe ein Sensor integriert wird, der den Armdurchmesser erfassen kann und/oder durch einen Aufbau des Luftsacks aus mehreren Kammern, welche von einem mittleren Sack hinzugefügt oder abgetrennt werden können.

Der Armdurchmesser (D) wird näherungsweise anhand des Armumfangs (U) gemessen. Der Umfang kann anhand der Kreisformel $D = U/Pi$ in einen Durchmesser umgeformt werden. Sensoren, die den Armumfang ermitteln können sind bspw. Biegesensoren, die indirekt über die Krümmung basierend auf Foliendrucksensoren, je nach Biegung ihren elektrischen Widerstand ändern, welcher einem Umfang zugeordnet werden kann. Dieser Sensor kann beliebig innerhalb des Gewebes eingebracht werden, jedoch so, dass dieser senkrecht zur Achse der Gliedmaße verläuft.

Vorteilhaft aufgrund der mechanischen Beanspruchung einer Manschette ist jedoch ein Sensor oder eine Sensoranordnung, der/die keinen analogen Wert (wie z.B. die Änderung eines elektrischen Widerstands), sondern diskrete Werte ausgibt, dies wird insbesondere durch konstruktive Maßnahmen ermöglicht.

Die Manschette wird insbesondere so gestaltet, dass nur diskrete Stufen der Festigkeit der Anbringung möglich sind. Bspw. kann die Manschette in Schritten von einem Zentimeter weiter geschlossen werden.

Dies kann mit einem Lochband und einem Verschluss, wie bei einer Armbanduhr erfolgen. Vorteilhaft geschieht dies jedoch, wie bei heutigen Manschetten, indem ein Teil des Gewebes durch eine Lasche gezogen wird und an der Manschette mittels Klettverschluss befestigt wird. Nun sind im Gewebe in festen Abständen harte bzw. nur schwer verformbare Elemente eingebracht, sodass das Gewebe nur Stück für Stück für jedes Element durch die Lasche zogen werden kann. Für jedes Element ist eine Länge des Armumfangs bekannt und es wird insbesondere erfasst, welche Elemente durch die Lasche gezogen wurden und/oder welche nicht.

Insbesondere in der Lasche, im Gewebe unter der Lasche oder im Klettverschluss ist ein Sensor eingebracht. Dieser Sensor kann ein kapazitiver und/oder induktiver Sensor sein oder ein Sensor, der die Technologie "capacitive touch" ausnutzt.

Insbesondere sind für einen induktiven Sensor die harten Elemente aus einem magnetischen Material geformt, sodass insbesondere jedes Element eine andere und unterscheidbare Magnetisierung aufweist. Bei einem Sensor, der die Technologie "capacitive touch" ausnutzt, sind bevorzugt in den harten Elementen verschieden große Metallstücke oder -gewebe eingebracht, bei einem kapazitiven Sensor sind bevorzugt verschiedene und/oder verschiedene Mengen Dielektrika und/oder in unterschiedlichen Abständen zur Auflagefläche in den Elementen angeordnet.

Wird die Manschette geschlossen, so befindet sich über dem Sensor ein hartes Element, welches durch den Sensor identifiziert werden kann und somit ist der Umfang bestimmt.

Bevorzugt kann/wird der Luftsack in seiner aktiven Größe verändert werden. Dazu ist der Luftsack insbesondere in mehrere Kammern aufgeteilt. Diese Kammern sind insbesondere konzentrisch um eine zentrale Kammer aufgebaut. Die Größen und Anzahl der Kammern können bspw. nach den heutigen Empfehlungen gewählt sein. Dann wäre die Anzahl der Kammern auf vier festgelegt. Die zentrale Kammer hat damit eine Größe (Breite x Länge) von 10x18cm. Diese rechteckige Kammer wird von weiteren Kammern umgeben, die in der Mitte eine Aussparung in der Größe der kleineren Kammern aufweisen. Die Außenmaße der zweiten Kammer ist demnach 12-13x24 cm, der dritten 15x30 cm und der vierten 18x36 cm.

Jedoch ist es vorteilhaft mehr als vier Stufen zu verwenden, um eine feinere Anpassung zu ermöglichen.

Die Kammern sind mit elektrisch schaltbaren Ventilen untereinander und/oder mit Luftleitungen verbunden. Im ersten Fall kann ein Ventil eine Kammer mit der nächst kleineren verbinden und die zentrale Kammer, so wie heutige Manschetten, an das Messgerät angeschlossen sein.

Der Luftsack kann jedoch auch so gestaltet werden, dass die aktive Fläche stufenlos geregelt werden kann. Dabei besteht der Luftsack insbesondere aus zwei Kammern die übereinander angeordnet sind. Die unterere Kammer wird zur Messung des Blutdrucks verwendet und die andere zur Einstellung der aktiven Fläche.

Die Kammer zur Einstellung der aktiven Fläche kann mittels Luft oder Flüssigkeit befüllt werden und dient zur Verformung der unteren Kammer.

Die obere Kammer ist insbesondere ringförmig oder angepasst auf die untere Kammer rechteckig, jedoch mit einer Aussparung in der Mitte. Zusätzlich ist die obere Kammer, insbesondere mit Riemen, an der unteren Kammer fixiert. Wird die obere Kammer aufgeblasen so überträgt sich deren Größenänderung, insbesondere mittels Riemen, auf die untere Kammer, die beispielsweise abgeschnürt wird. Die Abschnürung bewirkt, dass die aktive Fläche kleiner wird. Der Grad der Abschnürung kann mit Hilfe des Drucks in der oberen Kammer geregelt werden.

Durch die Kenntnis des Armumfangs ist auch eine Lösung möglich, die die Manschette in ihrer Funktion (bis auf das Einbringen des Sensors zur Messung des Armdurchmessers) nicht verändert.

Dabei wird eine Manschette mittlerer Größe verwendet. Es ergeben sich bei der Messung Fehler, wenn der Armdurchmesser nicht zur Manschettengröße passt. Diese Art des Fehlers ist jedoch systematisch und nur vom Armdurchmesser abhängig. Es ist also möglich den Fehler näherungsweise zu bestimmen und somit näherungsweise aus dem Messergebnis herauszurechnen.

Der Messfehler kann anhand von statistisch erhobenen Listen und Bestimmung des Armumfangs oder anderer Größenmerkmale bestimmt werden. Diese Listen werden insbesondere für in Frage kommende Armdurchmesser oder andere Größenmerkmale angefertigt und enthalten zu einem möglichen Messergebnis ein korrigiertes Ergebnis.

Bei einer Blutdruckmessung wird nach dem Schließen der Manschette der Armdurchmesser oder eine damit näherungsweise korrelierende Größe bestimmt und damit werden aufgrund einer im Gerät abgespeicherten Zuordnung von Armdurchmesser und geeigneter Manschettengröße die Ventile geöffnet bzw. geschlossen und/oder werden die Messwerte korrigiert. Diese Einstellung der Manschettengröße wird während der Blutdruckmessung nicht verändert. Eine Kombination von Anpassung und Korrektur ist insbesondere dann vorteilhaft, wenn die Anpassung nur in Stufen erfolgt/erfolgen kann. Die geringen Abweichungen zur optimalen Größe lassen sich dann rechnerisch im Messwert korrigieren.

## 6. Mögliche konkrete Umsetzungen der Erfindung

Mögliche Formen der Ausgestaltung der Erfindung sind rein exemplarisch und nicht beschränkend in den rein schematischen Fig. 1 und 9 dargestellt. Eine Ausgestaltung verfügt insbesondere immer über eine Luftdruckmanschette, welche mit der, insbesondere verbesserten, "Redtel Methode" betrieben wird. Zusätzlich wird eine weitere Einheit zur Messung der Pulswellenlaufzeit, bzw. Pulswellengeschwindigkeit benötigt. Dies kann durch die Kombination eines EKGs mit einem Plethysmographiesensor erfolgen, oder mittels zweier Plethysmographiesensoren. Es sind jedoch auch andere Methoden der Bestimmung der Pulswellenlaufzeit möglich, wie bereits beschrieben, insbesondere kann auch die Luftdruckmanschette dabei genutzt werden, beispielsweise zusammen mit einem weiteren Gerät wie EKGs oder Plethysmographiesensor.

Die folgenden Beschreibungen sollen mögliche Anwendungsgebiete für den Einsatz der Erfindung als Produkt aufzeigen.

## 6.1 Fitnessüberwachung/Schlafüberwachung

Diese Ausformung dient der kontinuierlichen Aufzeichnung des Pulses und dabei auftretenden Unregelmäßigkeiten bzw. Arhythmien und zur punktuellen Ermittlung des Blutdrucks. Dabei ist das Einsatzgebiet die medizinische Selbstüberwachung, wie z.B. während des Sports, bspw. einer Leistungsdiagnostik oder der Nacht, bspw. zur Überwachung hämodynamischer Effekte einer positiven Überdruckbeatmung bei Patienten mit schlafbezogener Atmungsstörung und Herzinsuffizienz.

Die Ausformung der Erfindung besteht aus einer Luftdruckmanschette für den Unterarm und einem Plethysmographiesensor, welcher bspw. am Finger angebracht wird. Beide Geräte sind mit einem Steuergerät auf der Manschette verbunden (vgl. Fig 1.3).

Vorteilhaft ist jedoch, zwei Plethysmographieeinheiten zu verwenden, wobei eine am Finger und eine am Armband sich befindet und mit der Steuereinheit verbunden sind.

Dabei ist das Signal der Einheit am Armband das Startsignal und das des

Plethysmographiesensors am Finger das Endsignal einer Pulswellenlaufzeitmessung (vgl. Fig. 1.4 bzw. Fig. 9.5 und deren Funktionsweise Fig. 17).

Darüber hinaus ist ein Beschleunigungssensor in dem Steuergerät integriert.

Die Messung erfolgt, indem eine Kalibrierung mit der Riva-Rocci-Methode oder, insbesondere verbesserten, Redtel Methode durchgeführt wird. Anschließend wird der Druck in der Manschette abgelassen und die Blutdruckmessung erfolgt über die Lichtintensitätsänderungen des Plethysmographiesensors bzw. über die kalibrierte Pulswellenlaufzeit (vgl. Fig. 17).

Darüber hinaus gibt der Plethysmographiesensor eine Welle aus, die den Herzpuls wieder spiegelt, sodass die Frequenz des Pulses bzw. das RR-Intervall und Fehler, z.B. Arhythmien, bestimmt werden können.

Da jedoch eine Bewegung Messartefakte bzgl. der Intensität verursacht, kann so eine kontinuierliche Blutdruckmessung in der Bewegung meist nicht zuverlässig erfolgen, jedoch ist die Frequenz des Pulses bzw. das RR-Intervall bestimmbar.

Der integrierte Beschleunigungssensor registriert Bewegungen. Wenn keine Bewegung erkannt wird und wenn der Sensor auf der Höhe zum Herzen sich befindet, welche bei der Kalibrierten verwendet wurde, kann der Blutdruck bestimmt werden. Wenn eine Lagekorrektur durch ein Zusatzgerät gegeben ist, kann die Veränderung des Blutdrucks aufgrund einer anderen Höhe zum HIP als bei der Kalibration ausgeglichen werden.

Die Werte von der Frequenz des Pulses bzw. die RR-Intervalle, Fehler, z.B. Arhythmien und Blutdruck werden gespeichert und können mithilfe einer Funkschnittstelle ausgelesen werden. Darüber hinaus kann auch ein Tagesprofil angefertigt werden (vgl. Fig. 8, 10 und 11).

Bei der Aufzeichnung des Tagesprofils können bereits auch Warnungen ausgegeben werden. Bei einer sportlichen Aktivität kann vor einem zu hohen Blutdruck gewarnt werden, dazu kann die Blutdruckmessung insbesondere erfolgen während einer Pause des sportlich Aktiven und/oder einem kurzzeitigen Ruhen der Messstelle, oder wenn der Puls zu gering ist kann eine Motivation ausgegeben werden höhere Leistungen zu erbringen.

Bei der Schlafüberwachung können kritische Situationen erkannt werden, z.B. Atemaussetzer oder Herzflimmern. Auf diese Situationen kann dann in geeigneter Weise reagiert werden, z.B. durch Wecken der Person oder einer Bezugsperson oder auch durch die Regulierung einer Beatmungsmaschine oder Änderung einer Medikation, beispielsweise mittels Infusionspumpe.

Bereits heute gibt es Systeme, z.B. von Omron eine Armbanduhr "HeartGuide fitness watch", welche mehrere Vitaldatensensoren vereinen. In diesem Beispiel ist die Blutdruckmessung nach Riva-Rocci integriert. Durch das Hinzufügen der Logiken der, insbesondere verbesserten, "Redtel Methode" in eine solche Uhr, kann diese bereits zu einer kontinuierlichen Blutdruckmessung befähigt werden.

In einer Ankündigung von Omron soll die nächste Version dieser Uhr auch ein EKG-Signal erfassen können.

Durch die Analyse der Form des EKG-Signals kann die Pulswellengeschwindigkeit bestimmt werden und somit auf den Blutdruck kalibriert werden, sodass eine Messung mit geringer Beaufschlagung möglich wird (vgl. die Funktionsweisen Fig. 14 und Fig 15).

Genauso gut kann ein Plethysmographiesensor, zur Bestimmung der Pulswellenlaufzeit, in eine solche Uhr integriert werden, wie es bereit in einer Vielzahl anderer Smartwatches der Fall ist, es ergeben sich die Möglichkeiten für die Funktionsweise Fig. 16.

Die Überwachung des Blutdrucks und des Herzpulses während der Nacht kann von hohem therapeutischen Nutzen sein.

Während der Nacht kann es zu Atemaussetzungen kommen. Dies betrifft vor allem auch Herz erkrankte Menschen. Durch die Veränderungen des Blutdrucks und des Pulses anhand der RSA kann die Atmung bzw. deren Frequenz erkannt werden. Zusätzlich treten bei Atemaussetzern auch Phasen an erhöhten und erniedrigten Pulsfrequenzen auf.

Wenn Atemaussetzer vorliegen, so kann der Körper oftmals nicht in die Ruhephase eintreten und somit bleibt der Blutdruck auf dem Niveau des Tages und die normale Nachtabsenkung des Blutdrucks kann nicht eintreten.

Abhilfe kann ein Beatmungsgerät bringen. Dieses Gerät würde die Atmung unterstützen, wenn ein Atemaussetzer erkannt wird oder anhand von Vorzeichen sich ein Atemaussetzer abzeichnet.

Für die Überwachung des Blutdrucks in der Nacht kann eine intermittierende kontinuierliche Messung durchgeführt werden.

Wird ein Gerät basierend auf den Funktionsweisen Fig. 12 bis 15 verwendet, so wird während der Messung der Arm beaufschlagt und eine lange ununterbrochene Messung ist nicht ohne Schmerzen möglich. Daher ist eine intermittierende Messung sinnvoll. Diese kann bspw. darin bestehen alle 15 Minuten eine Messung von 3 Minuten durchzuführen.

Werden Geräte verwendet die auf der Kalibration der Intensität von Plethysmographie oder EKG basieren, so kann eine kontinuierliche Messung durchgeführt werden. Für eine zuverlässige Kalibration wird auch hierbei bevorzugt diese dauerhafte Messung unterbrochen, wobei eine erneute Kalibration durchgeführt wird. Diese Kalibration wird in regelmäßigen Abständen bspw. 30 Minuten durchgeführt oder, wenn es zu starken Bewegungen, gemessen z. B. durch den Beschleunigungssensor, kommt.

Werden Geräte verwendet, die die Pulswellenlaufzeit ermitteln (Funktionsweisen Fig. 16 oder 17) kann eine kontinuierliche Messung erreicht werden.

Auch die Kalibration der Pulswellenlaufzeit muss in regelmäßigen Abständen erfolgen, jedoch können diese in größeren zeitlichen Abständen erfolgen, wobei die geforderte Qualität der Messwerte die Kalibrationsintervalle bestimmt. Eine weitaus qualitativ bessere Messung als die Messung mittels Riva-Rocci ergibt sich bereits bei Kalibrationsintervallen von 2 Stunden.

Da die Pulswellenlaufzeit bevorzugt auch während einer Kalibration bestimmt werden kann, kann auch während der Kalibration ein Blutdruck bestimmt werden.

### 6.2 Einfachste Kurzüberwachung / Bestimmung der Pulswellenlaufzeit

Eine der einfachsten Umsetzungen erlaubt es die Pulswellenlaufzeit zu bestimmen. Dabei besteht die Ausformung der Erfindung aus einer Riva-Rocci Manschette, welche auch die, insbesondere verbesserte, "Redtel Methode" durchführt, und einem Smartphone, welches über eine Kamera mit Beleuchtungseinheit verfügt.

Alle heutigen modernen Smartphones besitzen eine Kamera und eine Beleuchtungseinheit (Blitz).

Durch das Berühren und Abdecken der Kamera und insbesondere der Beleuchtungseinheit mit einem Finger, bei eingeschaltetem Blitzlicht als Dauerlicht, gelangt Licht vom Blitzlicht zur Kamera. Dabei ist die Intensität veränderlich mit dem Blutdruck und dem Puls. Es kann die Frequenz des Pulses bzw. das RR-Intervall und eine Pulswellenkontur bestimmt werden.

Die Kamera insbesondere die Beleuchtungseinheit kann auch mit einem anderen Körperteil abgedeckt werden, wodurch sich weitere Messmöglichkeiten ergeben, siehe im Folgenden.

Die Pulswellenlaufzeit ergibt sich aus dem Vergleich der Blutdruckwelle aus der Manschette und der Lichtintensitätswelle aus der Kamera des Smartphones. Wobei die Blutdruckwelle den Startzeitpunkt einer Messung markiert und die Lichtwelle den Endzeitpunkt.

Über die Länge von der Position der Manschette am Körper bis zur Position der Kamera am Körper, kann auch die Pulswellengeschwindigkeit angegeben werden.

Lichtwellen im sichtbaren Bereich besitzen unterschiedliche Eindringtiefen in die Haut. Jegliche Lichtquellen strahlen somit nicht immer die für die Blutdruckmessung benötigte Lichtwellenlänge aus. Smartgeräte besitzen unterschiedliche Lichtquellen. Weißes Licht im Smartphone zur Photographie geeignet, beinhaltet bspw. Lichtwellen von blau 460nm bis rot 680nm.

Der Smartphonehersteller wie Samsung verwendet bspw. bei seiner Smartwatch Gear S3 grünes Licht.

Um auch mit dem Smartphone den Blutdruck messen zu können, sollte die Lichtquelle bekannt sein.

Definiertes Licht, bspw. 535nm, lässt sich auf das Minima und Maxima jeder einzelnen durchlaufenden Pulswelle kalibrieren.

Eine mögliche Anwendung ist die Erkennung von Verschlüssen der Arterien (Stenosen) oder von Gefäßwandverkalkung (Arteriosklerose). Wird eine große Manschette verwendet, kann eine Druckwellenbestimmung am Oberschenkel durchgeführt werden und die Messung der Lichtwelle an einem Zeh. Wird dies für beide Beine durchgeführt und ergeben sich unterschiedliche Pulswellenlaufzeiten, so ist dies ein Hinweis auf einen Verschluss oder Gefäßwandverkalkung in einem Bein.

Grundsätzlich gilt, liegt eine Stenose vor, so ist die Pulswellenlaufzeit länger und bei einer Arteriosklerose sind die Laufzeiten kürzer im Vergleich zum gesunden Zustand.

**6.3 Integration in ein medizinisches Überwachungsgerät**

Heutige Systeme zur medizinischen Kreislaufüberwachung verfügen über eine Vielzahl an Sensoren. Darunter sind Plethysmographiesensoren oder Oximetriesensoren und Sensoren zur Aufnahme des EKG Signals Standard.

Derartige Systeme werden bspw. von Dräger oder Siemens angeboten.

Der Blutdruck wird typischerweise anhand einer von zwei Methoden bestimmt.

Dabei ist die invasive Methode zwar sehr genau, jedoch benötigt diese einen Eingriff in den Körper und weitere externe Geräte, neben dem eigentlichen Überwachungsgerät, wodurch die Transportfähigkeit z.B. vom Unfallort zum Krankenhaus, des Patienten eingeschränkt ist.

Daher gibt es an medizinischen Überwachungsgeräten die Möglichkeit den Blutdruck über die Pulswellenlaufzeit zu bestimmen. Dies wird mittels EKG und Plethysmographie realisiert. Jedoch kann bisher nur in wenigen Fällen eine geeignet Kalibrierung erfolgen, um der Laufzeit einen absoluten Wert für den Blutdruck zuordnen. In der Regel werden lediglich Veränderungen der Pulswellenlaufzeit als Warnung ausgegeben.

Wird nun zusätzlich ein medizinisches Überwachungsgerät mit einer Luftdruckmanschette und den Logiken der, insbesondere verbesserten, "Redtel Methode" ausgestattet, kann eine kontinuierliche Langzeitmessung des Blutdrucks erfolgen.

Dabei wird insbesondere die Pulswellenlaufzeit aus dem Signal des EKGs und des Plethysmographiesensors kalibriert (Funktionsweise Fig. 16) oder aus den Signalen von zwei Plethysmographieeinheiten (Funktionsweise Fig. 17). Diese Kalibration kann völlig automatisiert auf Knopfdruck erfolgen und bedarf keines menschlichen Eingriffs.

Die Integration erlaubt es auch auf die invasive Messung zu verzichten, da die Werte der Messung nach der, insbesondere verbesserten, "Redtel Methode" vergleichbar sind in der Qualität. Sie zeigen Abweichungen zur invasiven Messung unter optimalen Bedingungen von 3 - 4% und sind dabei deutlich unanfälliger gegenüber äußeren Einflüssen oder Fehlbedinungen.

**6.4 Ferndiagnose**

Die Kombination eines Geräts zur Messung des kontinuierlichen Blutdrucks nach der, insbesondere verbesserten, "Redtel Methode" mit einer beabstandeten Messung, wie sie z.B. in der Patentanmeldung mit dem Aktenzeichen DE 10 2018 002 268.5 zu entnehmen ist, ermöglicht es Arterienverschlüsse zu erkennen.

Die beabstandete Messung kann erfolgen, indem ein übertragenes (Live) Videosignal analysiert wird. Dieses Videosignal kann mit einer stationären Kamera aufgenommen werden, jedoch ist auch eine Videoaufzeichnung und -analyse mit einem Smartphone möglich. Die Ergebnisse dieser Messung ist die Pulswellengeschwindigkeit an jeder im Bild zu erkennenden Hautoberfläche (vgl. Fig. 18).

Ein Verschluss einer Arterie nahe zur Hautoberfläche hat einen Einfluss auf die Pulswellengeschwindigkeit.

Mit Hilfe eines Geräts zur Messung des kontinuierlichen Blutdrucks nach der, insbesondere verbesserten, "Redtel Methode", das insbesondere die Daten ebenfalls überträgt, beispielsweise zunächst an ein Smartphone, dass die Daten dann, insbesondere zusammen mit dem Videosignal zur Analyse, insbesondere über das Internet, überträgt kann nun jedem Ort auf der Hautoberfläche anhand der Pulswellengeschwindigkeit ein Blutdruck zugeordnet werden.

Diese Zuordnung erlaubt es die Schwere eines möglichen Arterienverschlusses durch den Vergleich des Blutdrucks vor und nach dem Verschluss einzuschätzen.

Es können auch beginnende Stenosen und Arteriosklerosen erkannt werden, aufgrund von Gefäßsteifigkeiten bzw. durch Plaque Ablagerung, die zu Unterschieden in der Pulswellenlaufzeit führen. Hierzu werden vergleichende Analysen an verschiedenen Körperpositionen und/oder - hälften durchgeführt.

Wird die rechte mit der linken Gesichtshälfte verglichen, so ist ein Unterschied in der Pulswellenlaufzeit zwischen linker und rechter Seite erkennbar (vgl. Fig. 19). Dies ist begründet in der Länge des Aortenbogens, von dem die beiden Halsschlagadern an verschiedenen Stellen abzweigen. Unterscheidet sich nun die Laufzeit erheblich von einem normalen Zustand (10 ms von linker zur rechten Seite), so ist dies ein Hinweis auf eine Stenose oder deren Entwicklung in den Halsschlagadern.

Eine ähnliche Vorgehensweise kann für die Erkennung von Arteriosklerose in den Extremitäten durchgeführt werden. Es können die Pulswellengeschwindigkeiten zwischen der linken und rechten Extremität analysiert oder die Pulswellenverschiebungen zwischen den Armen und Beinen auf beiden Seiten verglichen (vgl. Fig. 20) werden. Ergibt sich ein Unterschied zwischen rechter und linker Seite, so ist dies ein Hinweis auf eine Verschlusskrankheit oder deren Entwicklung. Eine Früherkennung ist möglich, da die Pulswellenlaufzeit aufgrund von Gefäßsteifigkeiten. z.B. durch Plaque Ablagerung, bereits verändert wird.

**6.5 Weitere Einsatzgebiete**

Da die Ausgestaltungen der Erfindung schmerzfrei und einfach zu bedienen sind, ergeben sich, aufgrund der täglichen Nutzung, neue Einsatzmöglichkeiten.

So kann der Alltag eines Nutzers gelenkt werden.

In einfachster Weise ist dies eine Warnung an den Nutzer oder eine Bezugsperson bei Herzkreislaufproblemen. Wird ein Problem erkannt, kann der Nutzer darauf aufmerksam gemacht werden, dieser kann dann je nach vorheriger Diagnose oder Anweisung eines Arztes Medikamente zu sich nehmen oder seine jetzigen Aktionen einschränken (z.B. Sport) und/oder sein aktuelles Verhalten ändern. Dies wird insbesondere ermöglicht, wenn ein Tagesprofil (siehe Fig. 8, 10 und 11) aufgezeichnet wird.

Die kontinuierliche Überwachung des Blutdrucks, kann dem Patienten eine auf die Situation angepasste Medikamentenaufnahme ermöglichen. Wobei die Notwendigkeit und die Dosierung anhand der momentanen Aufzeichnung und/oder des aufgezeichneten Profils, z.B. Tagesprofils, ermittelt werden kann.

Die Überwachung des Alltags ermöglicht es den Belastungs- und Gefühlszustand besser zu erkennen. Schnell steigender hoher Blutdruck mit geringer Variabilität und flacher Atmung ist ein Zeichen für Stress. Bei sportlichen Aktivitäten ist ein erhöhter Blutdruck gewünscht, jedoch wenn der Blutdruck oder die Pulsfrequenz über ein gesundes Maß steigt, kann dies erkannt werden und rechtzeitig durch eine Warnung verhindert werden.

Eine weitere Möglichkeit der Warnung ist bei falscher Ernährung. Will ein Nutzer z.B. abnehmen, so ist dies oft ein Prozess, der erhöhte Willensstärke erfordert. Die Probleme sind, dass zunächst der Appetit hoch bleibt und bei ersten Fortschritten sich das Gewebe langsam zurückbildet, was meist als unattraktiv empfunden wird. Damit diese Probleme nicht zum Abbruch des Vorhabens führen, kann eine quantifizierte Aussage zum Essverhalten eine Hilfe sein. Eine Hilfe besteht dabei in der Gamifizierung des Vorhabens, so kann das Erreichen von Leveln (z.B. Suppenwoche abgeschlossen) und Rekorden (z.B. 10 Tage jeweils unter 2000 kcal) motivierend wirken. Die Erfindung erlaubt es hierbei das Essverhalten zu dokumentieren. Wird etwas gegessen, so steigt der Blutdruck an. Dieser Anstieg hängt vom Essen und der Menge ab. Es gilt je mehr sich der Völlerei zum Essen hingegeben wird, umso mehr steigt der Blutdruck. Die Erfindung würde bei einem zu hohen Blutdruckanstieg während des Essens eine Warnung ausgeben, sodass das Essen rechtzeitig eingestellt werden kann, um das Abnehmvorhaben nicht zu gefährden.

Eine weitere Möglichkeit der Warnung bei falscher Ernährung ist die Erkennung von Flüssigkeitsmangel. Gerade ältere Menschen verlieren das Durstempfinden und nehmen daher weniger Flüssigkeit zu sich. Der Blutdruck ist ein Indikator für den Flüssigkeitshaushalt. Bei anhaltendem Flüssigkeitsmangel steigt der Blutdruck. Daher kann eine Langzeitmessung verwendet werden, um die Einhaltung von Trinkplänen und der Qualität von Pflege quantitativ beurteilen zu können.

Neben der Messgröße Blutdruck ist auch der Herzpuls ein wichtiger Informationswert. Abnormale Veränderungen des Pulses und des Blutdrucks weisen auf Herzkreislaufprobleme hin. Bei Menschen in Risikogruppen könnte daher eine dauerhafte kontinuierliche Messung verwendet werden, um gegebenenfalls sofort einen automatischen Notruf absetzten zu können.

Die gleiche Überwachung kann auch bei Menschen an sicherheitsrelevanten Stellen, z.B. beim Personentransport oder bei der Bedienung von Maschinen, ihren Einsatz finden.

Bspw. könnten Lokomotivführer vermessen werden. Tritt nun ein kardiologisches Ereignis auf, z.B. ein Herzinfarkt, so kann der Zug gestoppt werden, ohne dass es zu einem Unfall kommt. Gleichzeitig kann ein automatischer Notruf für den Lokomotivführer abgesendet und die Leitstelle informiert werden, sodass diese weitere Maßnahmen, wie Umleiten anderer Züge, veranlassen kann.

Auf großen Transportschiffen wird heutzutage nur eine minimale Mannschaft eingesetzt, die auch über das Schiff verteilt ihren Tätigkeiten nachgeht, sodass ein persönlicher Kontakt nur selten am Tag vorhanden ist. Kommt es zu einem kardiologischen Ereignis so kann dies von der restlichen Mannschaft viele Stunden unbemerkt bleiben. Werden die Mannschaftsmitglieder vermessen können diese Ereignisse der restlichen Mannschaft mitgeteilt werden.

Heutige Entwicklungen zeigen, dass zukünftig der Personentransport mit teil- oder voll autonom fahrenden Fahrzeugen realisiert werden kann. In einem solchen Fahrzeug kann die Erkennung von Herzproblemen genutzt werden, damit das Fahrzeug rechtzeitig an den Fahrbahnrand fährt, sodass kein Unfall passiert, wobei gleichzeitig ein automatischer Notruf abgesetzt wird. Bei voll autonomen Fahrzeugen kann auch die Fahrt in ein nahegelegenes Krankenhaus eingeleitet werden. Durch die Kommunikation des autonomen Fahrzeugs mit anderen sich auf der Straße befindlichen autonomen Fahrzeugen, kann das Fahrzeug ähnlich einem Rettungswagen auch schneller als normalerweise erlaubt fahren, da es die anderen Fahrzeuge entsprechend informiert. Als Beispiel sei eine autonome Fahrt von Berlin nach München genannt. Zu Beginn der Fahrt wird das Ziel eingegeben und das Auto fährt ohne weitere Nutzerinteraktion zum Ziel. Tritt nun ein kardiologisches Ereignis auf oder der Fahrer verstirbt, so fährt das Auto Stunden lang mit dem verstorbenen oder ohnmächtigen Fahrer zum Ziel. Am Ziel angekommen parkt das Auto autonom auf einem Parkplatz, wo der Fahrer unter Umständen mehrere Tage unbemerkt bleibt.

Die Vermessung des Fahrers kann daher lebensrettend sein, da eine rechtzeitige Fahrt ins Krankenhaus organisiert werden kann, oder, falls dies nicht mehr möglich ist, es mindestens ein würdehaftes Umgehen mit dem verstorbenen Fahrer erlauben.

**Beschreibung der Abbildungen**

*Figure 1*

Die Abbildung zeigt exemplarisch mögliche Positionen und Ausgestaltungen der Erfindung. In einer einfachen Ausgestaltung (1.1) sind alle Teileelemente als separate Einheiten ausgeführt. Die Luftdruckmanschette (A), welche mittels der Recheneinheit nach der, insbesondere verbesserten, "Redtel Methode" arbeitet, ist über einen Schlauch an die Recheneinheit (B) angeschlossen. Darüber hinaus ist an die Recheneinheit ein Plethysmographiesensor (C) per Kabel angeschlossen. Die Recheneinheit sendet per Funkübertragung oder kabelgebunden die Daten der Manschette und der Plethysmographie an eine Auswerte- und Darstellungseinheit (D). Die Auswerte- und Darstellungseinheit kann bspw. ein Smartgerät oder ein medizinisches Überwachungsgerät z.B. ein Drägermonitor sein. Zur Ermittlung der Pulswellenlaufzeit wird ein separates EKG (E) verwendet, welches seine Daten zur gleichen Auswerte- und Darstellungseinheit (D) sendet.

In einer weiteren Ausgestaltung (1.2) kann auch ein Unterarmmanschettengerät (A + B) verwendet werden, in das bereits eine Recheneinheit integriert ist.

Auch die Integration eines EKGs in das Unterarmmanschettengerät (A + B + E) ist in einer weiteren Ausgestaltung (1.3) möglich. Jedoch kann die Aufzeichnung eines EKG-Signals nur erfolgen, wenn der Cabrerakreis geschlossen wird, indem ein Finger der anderen Hand auf eine äußere Elektrode der Ausgestaltung gelegt wird.

Daher ist es in einer weiteren Ausgestaltung (1.4) sinnvoll den Startzeitpunkt für die Ermittlung der Pulswellenlaufzeit nicht anhand eines EKG-Signals zu ermitteln, sondern über einen weiteren Plethysmographiesensor, welcher auch in das Unterarmmanschettengerät (A + B + C') integriert ist.

Die Auswerte- und Darstellungseinheit muss nicht zwingend eine externe Einheit sein, so kann in einer weiteren Ausgestaltung (1.5) die Auswerte- und Darstellungseinheit in das Unterarmmanschettengerät (A + B + C' + D') integriert werden. Dabei können, wobei dies nicht zwingend notwendig ist, die Daten auch an eine externe Auswerte- und Darstellungseinheit (D) übermittelt werden.

Neben der herkömmlichen Messung der Pulswellenlaufzeit bzw. Pulswellengeschwindigkeit können auch moderne Verfahren, wie z.B. die Analyse von Bewegtbildern, verwendet werden. In einer weiteren Ausgestaltung (1.6) wird eine Kamera (F) auf den Patienten gerichtet. Durch geeignete Analyse kann die Pulswelle (G) und deren Bewegung auf dem Videobild sichtbar gemacht werden. Es lassen sich Pulswellenlaufzeit und Pulswellengeschwindigkeit ermitteln. So kann auf einen Plethysmographiesensor und eine Messanordnung zur Aufzeichnung des EKG verzichtet werden.

Auf heutigen Intensiv- und Wachstationen wird ein medizinisches Überwachungsgerät, welches die Daten unterschiedlichster Sensoren bearbeitet und Messwerte anzeigt, verwendet. Die hier vorgestellte Erfindung kann in einer weiteren Anordnung (1.7) in ein solches Überwachungsgerät (B + D) integriert werden. Die Integration ist von daher vorteilhaft, da bereits notwendige Sensoren, wie z.B. ein medizinisches EKG (E'), ein Plethysmographiesensor (C) oder eine Manschette (A) vorhanden sein können.

*Figure 2*

Die Abbildung zeigt beispielhafte Daten einer Messung, welche mit einem Gerät durchgeführt wurde, welches mit den Logiken der verbesserten "Redtel Methode" ausgestattet ist (Blutdruckverlauf), und unverarbeitet Daten eines Plethysmographiesensors (Verlauf der Lichtintensität). Die Daten wurden mit einer Ausgestaltung, wie sie in Fig. 1.1 dargestellt wird, erhoben. Wobei die Luftdruckmanschette am Unterarm befestigt war und die Plethmographieeinheit am Zeigefinder der Hand aufgesetzt wurde.

Es ist zu erkennen, dass die Kurve der Lichtintensität, der Kurve des Blutdrucks zeitlich hinterher läuft, erkennbar an den zeitlichen Positionen der Minima.

*Figure 3*

Die Darstellung zeigt einen vergrößerten Ausschnitt aus Fig. 2. Es zeigt sich, dass die Pulswellenlaufzeit nicht konstant über den Puls hinweg ist. Die Abstände ergeben sich wie folgt: H: 80 ms (Wellental zu Wellental bzw. Diastole); I: 40 ms (Wendepunkt zu Wendepunkt) und J: 20 ms (Wellenberg zu Wellenberg bzw. Systole)

*Figure 4*

Gezeigt ist der Druckverlauf als Funktion der Zeit (4.1), welcher bei einer herkömmlichen Messung nach Riva-Rocci in der Manschette vorliegt. Hierbei ist die Messvariante bei steigendem Druck gezeigt. Der Druckverlauf zeigt kleine Variationen (bspw. Markierung 4.2), welche auf den Herzpuls zurückzuführen sind. Wird der generelle Druckanstieg von der Druckkurve subtrahiert, so ergibt sich die Druckvariation, welche alleinig auf den Herzpuls zurückzuführen ist (4.3).

In dieser Kurve werden lokale Minima (z.B. Markierung 4.4) und lokale Maxima (z.B. Markierung 4.5) ermittelt. Der Abstand in Amplitude zwischen einem lokalen Minimum und einem darauf folgenden lokalen Maximum wird bestimmt und der größte Abstand wird gefunden (4.6). Ausgehend vom größten Abstand wird in Richtung zeitlich früheren Abständen ein Abstand gesucht, welcher einem empirischen prozentualem Anteil vom größten entspricht. Der Zeitpunkt an dem dieser Abstand gefunden wird, ist der Zeitpunkt an dem in der Manschette der diastolische Blutdruck (4.7) vorlag. Vergleichbar wird für den systolischen Blutdruck (4.8) vorgegangen, indem zu zeitlich späteren Abständen ein Abstand gesucht wird der einen (anderen) empirischen prozentualen Anteil zum größten Abstand aufweist.

*Figure 5*

Darstellung der Einflüsse von Atmung auf die Messergebnisse der Riva-Rocci-Methode. Gezeigt ist ein zeitlicher Verlauf des arteriellen Blutdrucks über zwei volle Atemzuge hinweg (5.1) aufgenommen mit der verbesserten Redtel Methode. Die Kurve weißt für jeden Herzpuls eine Druckvariation auf, die zwischen dem diastolischen und dem systolischen Blutdruckwert für diesen Herzpuls variiert. Dabei variiert die Amplitude der Druckvariationen mit der Atmung (schematisch anhand der Linien 5.2 gezeigt).

Würde nun eine Messung mit der Riva-Rocci-Methode durchgeführt, so werden Werte für den diastolischen und den systolischen Wert ermittelt, die den lokalen Minima und den lokalen Maxima entsprechen. Hierbei liegen die Werte für Diastole und Systole, aufgrund der Messmethode, zeitlich mehrere Pulsschläge auseinander.

Beispiele für mögliche Wertepaare sind die Markierungen 5.3, 5.4 und 5.5.

Derzeit gelten nach der WHO für den Blutdruck folgende Grenzwerte (angedeutet durch Linien):

Systole: Größer 130 mmHg: Hypertonie Stadium 1 (5.6), Größer als 140 mmHg Hypertonie Stadium 2 (5.7)

Diastole: Größer 80 mmHg: Hypertonie Stadium 1 (5.8), Größer als 90 mmHg Hypertonie Stadium 2 (5.9)

Danach würde der Messpunkt 5.3 als gesund bewertet, der Messpunkt 5.4 als Hypertonie Stadium 1 und der Messpunkt 5.5 als Hypertonie Stadium 2 bewertet werden.

Dieses Beispiel zeigt, dass die Messergebnisse der Riva-Rocci-Methode eigentlich von ihrer Qualität her nicht verwendet werden können, um eine genaue Diagnose zu stellen, da bei gleichem Druckverlauf unterschiedliche Ergebnisse ermittelt werden.

*Figure 6*

Darstellung des Druckverlaufs in der Manschette bei der Verwendung der Redtel Methode (6.1). Bei der Redtel Methode wird zunächst eine vergleichbare Messung (6.2), wie bei der Riva-Rocci-Methode durchgeführt. Für eine genaue Kalibrierung der verbesserten Redtel Methode werden jedoch die Messzeitpunkte der ermittelten Systole (6.3) und der Diastole (6.4) aufgezeichnet. Danach wird der Druck in der Manschette reduziert bspw. auf 100 mmHg, andere Werte sind auch möglich.

Mit den Werten für Systole, Diastole und deren Messzeitpunkte kann der weitere Verlauf des Drucks in der Manschette (6.5) kalibriert werden und als Ergebniswelle angezeigt und zur weiteren Analyse ausgegeben werden.

*Figure 7*

Vergleich von Druckkurven, welche mit der verbesserten Redtel Methode (7.1, am rechten Arm) und mit der invasiven Methode (7.2, am linken Arm, Dräger System) ermittelt wurden. Von diesen Kurven wurden die lokalen Minima (bspw. verbesserte Redtel Methode 7.4, invasive Methode 7.6) und die lokalen Maxima (bspw. verbesserte Redtel Methode 7.3, invasive Methode 7.5) bestimmt. Mit diesen Werten ist es möglich neben Blutdruck auch das RR-Intervall (bspw. 7.7) für jeden Herzschlag zu bestimmen.

Neben den Vitaldaten können auch Auffälligkeiten, wie z.B. eine Arrhythmie (7.8) erkannt und dargestellt werden.

Der Vergleich zeigt, dass die verbesserte Redtel Methode in Qualität und Genauigkeit vergleichbare Daten erheben kann, wie der derzeitige Goldstandard der Blutdruckmessung, die invasive Methode.

*Figure 8*

Gezeigt ist ein mögliches Tagesprofil des systolischen (8.1) und des diastolischen (8.2) Werts des Blutdrucks, welches mit der verbesserten Redtel Methode erhoben werden kann. Außerdem sind die Grenzwerte der WHO eingezeichnet (siehe auch Fig. 5, Systole 8.10, Diastole 8.11).

Der Verlauf zeigt typische Veränderungen des Blutdrucks durch alltägliche Situationen und durch Medikation.

8.3: Auswirkung von Kaffeetrinken.

8.4: Einnahme von blutdrucksenkenden Medikamenten

8.5: Mittagessen

8.6: Eine weitere Einnahme von blutdrucksenkenden Medikamenten

8.7: Rehasport unter Flüssigkeitsmangel

8.8: Eine weitere Einnahme von blutdrucksenkenden Medikamenten, jedoch sinkt der Blutdruck zu weit, wahrscheinlich aufgrund einer falschen Dosis

8.9: Auffälligkeiten während der Nacht

*Figure 9*

Darstellung verschiedener Geräteanordnungen und deren Komponenten zur Messung des kontinuierlichen Verlaufs des Blutdrucks mit der verbesserten Redtel Methode.

Die Variante 9.1 besteht nur aus einer herkömmlichen Blutdruckmanschette (9.6), welche durch die neuen Logiken der verbesserten Redtel Methode (9.7) erweitert wird. Die Funktionsweise ist anhand des Druckverlaufs in Fig. 12 dargestellt.

Bei der Variante 9.2 wird zusätzlich ein oder mehrere Beaufschlagungssensoren (9.8) eingebracht. Diese Sensoren sind in der Manschette so angebracht, dass diese zur Hautoberfläche zeigen und sich über einer Arterie befinden, diese Arterie ist bei einer Unterarmmanschette die A. Radialis und bei einer Oberarmmanschette die A. Brachialis. Die Funktionsweise ist anhand des Druckverlaufs in Fig. 13 dargestellt.

Bei der Variante 9.3 wird die herkömmliche Blutdruckmanschette um ein EKG (9.9) erweitert.

Dieses EKG dient zur Verbesserung der Kalibration der verbesserten Redtel Methode durch die Messwerte basierend auf der Riva-Rocci-Methode. Die genaue Ausgestaltung des EKGs kann je nach Anwendungsgebiet erfolgen. In einer mobilen Variante können zwei Elektroden in der Manschette zur Haut zeigend eingebracht sein. Diese Elektroden können bspw. aus einem Edelstahlgewebe bestehen. Eine dritte Elektrode kann auf dem Gehäuse der Steuer- und Anzeigeeelektronik angebracht sein. Berührt der Nutzer diese dritte Elektrode mit einem Finger der Hand an dessen Gliedmaße nicht gemessen wird, so ist der Cabrerakreis geschlossen und ein EKG kann dargestellt werden.

Auch die Verwendung von kabelgebundenen Elektroden ist möglich. Diese sind bei einer mobilen Anwendung mit der Steuerelektronik verbunden und können am Körper an entsprechenden Stellen aufgeklebt werden. In der klinischen Anwendung kann die Blutdruckmanschette an einem Patientenmonitor angebracht oder mit ihm verbunden sein, dabei wird das EKG-Signal vom Patientenmonitor bereitgestellt, da diese Monitore in der Regel über eine EKG-Funktion verfügen. Die Funktionsweise zur kontinuierlichen Abbildung der Druckwelle ist anhand des Druckverlaufs in Fig. 14 dargestellt. Eine weitere Verwendungsweise über die Kalibration des Blutdrucks durch die Pulswellenlaufzeit ist in Fig. 15 dargestellt.

Die Variante 9.4 ist eine erweiterte Form von Variante 9.3, wobei diese Variante nach der Kalibration ohne einen Anpressdruck durch die Manschette auskommt und daher für eine Langzeitmessung auch über 24 Stunden hinweg geeignet ist. Durch die Verwendung einer Photo-Plethysmographie (9.10) kann der Pulsverlauf nachvollzogen werden. Es kann also die Pulswellenlaufzeit aus dem EKG-Signal (Startzeitpunkt) und der Plethysmographie (Endzeitpunkt) bestimmt werden. Die Pulswellenlaufzeit kann durch Kalibration genutzt werden, um die Werte des Blutdrucks zu bestimmen.

Die Plethysmographieeinheit kann unterschiedlich ausgeprägt sein. Es kann eine Fingereinheit verwendet werden, jedoch ist auch eine Einheit in der Blutdruckmanschette, welche zur Hautoberfläche gerichtet ist, möglich. Ferner ist eine von der Manschette unabhängige Einheit als separates Band um den Arm denkbar.

Die Funktionsweise ist anhand des Druckverlaufs in Fig. 16 dargestellt.

Die Variante 9.5 verzichtet auf ein EKG und verwendet stattdessen zwei Photo-Plethysmographieeinheiten (9.10 und 9.11). Die Plethysmographieeinheiten können, wie bei der Variante 9.4 beschrieben, ausgeprägt sein, jedoch sollten die Einheiten separat voneinander am Körper angebracht sein. Durch den Abstand der Einheiten zueinander ergibt sich ein zeitlicher Versatz in den Messwertkurven der beiden Einheiten. Neben diesem Versatz sind die beiden Messwertwellen zueinander verformt. Diese Verformung beruht auf der unterschiedlichen Geschwindigkeit der Pulswelle für die Diastole und die Systole. Die Auswertung der beiden Messwertwellen erlaubt es eine Pulswellengeschwindigkeit für die Diastole und eine, die die Systole unabhängig voneinander, zu ermitteln. Somit kann die Diastole unabhängig von der Systole auf die Pulswellenlaufzeit kalibriert werden. Die Funktionsweise ist anhand des Druckverlaufs in Fig. 17 dargestellt.

*Figure 10*

Eine weitere Darstellungsmöglichkeit eines Tagesprofils (zu Fig. 8). Ziel dieser Darstellung ist es kritische und unkritische Zustände auf einen Blick darzustellen. Diese Darstellungsmöglichkeit eignet sich besonders als Display in einer Uhr oder auf dem Smartphone. Diese Art der Darstellung ist nicht nur für die Darstellung des Blutdrucks geeignet, sondern kann auch andere Arten von Tagesprofilen darstellen; hier wird jedoch nur auf die spezielle Anwendung beim Blutdruck eingegangen.

Gezeigt ist eine konzentrische Anzeige, welche von außen nach innen verschiedene Segmente aufweist. Im Falle der Anzeige eines Blutdrucktagesprofils sind diese vorteilhafterweise: Ein Ziffernblatt einer 12 oder 24 Stunden analog Uhr (10.1) zur Anzeige der Uhrzeit, der Verlauf der Systole (10.2) und der Verlauf der Diastole (10.3).

Die Verläufe von Systole und Diastole sind durch eine Farbgebung gegeben. Die Farbgebung richtet sich danach wie kritisch der Zustand ist. Für eine Beurteilung nach WHO bedeutet dies, dass ein gesunder Zustand gegeben ist, wenn der Wert der Systole geringer als 120 mmHg ist und der der Diastole geringer als 80 mmHg. Ein erhöhter Blutdruck ist gegeben mit Blutdruckwerten zwischen 120 und 140 für die Systole oder einer Diastole zwischen 80 und 90 mmHg. Ein erkrankter Zustand ist gekennzeichnet durch Blutdruckwerte höher als 140 mmHg für Systole oder 90 mmHg für die Diastole. In der Darstellung werden gesunde Tagesbereiche mit grün dargestellt (hier nicht schraffierte Bereiche, z.B. 10.6), erhöhte Blutdrucksbereiche im Tagesprofil mit orange dargestellt (hier wellige Schraffur, z.B. 10.5) und als krankhaft eingestufte Bereiche mit rot dargestellt (hier streifige Schraffur, z.B. 10.4). Bei einer 12 Stunden Uhr können Farben und/oder konzentrische Ringe mit unterschiedlichen Innen- und/oder Außendurchmessern a.m. und p.m. kennzeichnen.

*Figure 11*

Darstellungsmöglichkeit des Blutdrucks und dessen Verlauf, welcher nach der verbesserten Redtel Methode erfasst wurde, und Nutzerinteraktionsmöglichkeit, welche für den (interessierten) Privatnutzer gedacht ist.

Gezeigt ist eine Anzeige 11.1, welche entweder auf einem Smartphone, welches mit einer Blutdruckmanschette, die mit der verbesserten Redtel Methode arbeitet, per Funkverbindung verbunden ist, oder direkt auf der Anzeige eines entsprechenden

Blutdruckmanschettenautomatens angezeigt werden kann.

Die Anzeige 11.1 kann unter anderem folgende Komponenten darstellen:

11.3: Darstellung des zeitlichen Verlaufs des Drucks in den Arterien unter der Manschette.
11.4: Blutdruckwert in Form von Systole und Diastole mit der Pulsfrequenz zum aktuellen Herzschlag.
11.5: Vergangene Blutdruck- und Pulsfrequenzwerte mit Messzeitpunkten
11.6: Eine Einordnung des Blutdrucks z.B. nach den WHO Kriterien, auf einer Farbskala. Dabei kann entweder der aktuelle Blutdruck des aktuellen Herzschlags zur Einordnung verwendet werden, oder die Gesamtheit der gemessenen Werte.
11.7: Aktuelle Uhrzeit

Außerdem kann zwischen Anzeigemöglichkeiten gewechselt werden, so kann z.B. das aktuelle Tagesprofil, wie z.B. in Fig. 10 oder in Fig. 8 dargestellt angezeigt werden.

Die Blutdruckmessung kann darüber hinaus mit einer Nutzerinteraktionsmöglichkeit (11.11) gesteuert werden. Im einfachsten Falle sind dies Druckknöpfe oder Touchfelder auf dem Display, jedoch sind auch weitere Steuerungsmöglichkeiten z.B. die Sprachsteuerung, möglich. Diese Steuerung ermöglicht es neben der eigentlichen Messsteuerung auch Anmerkungen des Nutzers einzugeben. Diese Anmerkungen dienen der Einstufung einer Veränderung im Tagesprofil. Nimmt der Nutzer blutdrucksenkende Medikamente zu sich kann dies so festgehalten werden und entsprechend im Tagesprofil markiert werden. Andere Anmerkungen können bspw. sportliche Aktivität, Therapie und medizinische Maßnahmen, akute Erkrankungen, Nahrungsaufnahme, Stress, Unwohlsein, Schwindel, Schmerzen oder auch plötzliche Gemütsveränderungen (z.B. "hat sich erschreckt") sein.

Für eine weitere Analyse durch eine medizinisch geschulte Fachperson kann ein Bericht (11.2) automatisch oder auf Eingabe des Nutzers erzeugt werden. Dieser Bericht wird in einer Datenbank abgelegt oder elektronischer versendet (11.8), sodass die Fachperson darauf Zugriff hat.

Der Bericht kann unter anderem folgende Komponenten enthalten:

11.9: Ein Tagesprofil in Form einer konzentrischen Darstellung z.B. als Uhr (siehe auch Fig. 10), sodass die kritischen Zeitabschnitte auf einen Blick erkennbar werden.
11.10 Ein detailliertes Tagesprofil, welches die Werte für Diastole und Systole getrennt voneinander zu jeder Tageszeit darstellt und farblich markiert einordnet (siehe auch Fig. 8). Darüber hinaus können dem Bericht Druckverlaufskurven angehängt sein, die den Verlauf von Schlag zu Schlag zeigen (vergleichbar zu 11.3) und eine Auffälligkeit aufweisen.

Neben der reinen Anzeige, Ausgabe und Aufzeichnung des aktuellen Zustands des Nutzers kann die Nutzerinteraktionsmöglichkeit auch genutzt werden, um Signale für Anweisungen an den Nutzer auszugeben. Diese Signale umfassen bspw. die Aufforderung eine (vorher mit einem Arzt vereinbarte) Medikation durchzuführen, Flüssigkeit zu sich zu nehmen, eine sportliche Aktivität einzuschränken, die Nahrungsaufnahme zu regulieren oder weitere vordefinierte Aktionen. Diese Signale für Anweisungen werden dem Nutzer aufgrund der Blutdrucksituation ausgegeben und können durch Ton, Vibration oder visuelle Darstellung auf dem Display erfolgen. Ferner ist eine Information als Pushnachricht ans Smartphone möglich.

*Figure 12*

Typischer Luftdruckverlauf bei einer kontinuierlichen Blutdruckmessung mit einer um die Logiken der Redtel Methode erweiterten aber sonst nicht weiter modifizierten herkömmlichen Blutdruckmanschette. Gezeigt ist der Druckverlauf in der Manschette als Funktion der Zeit (12.1).

Wird eine Messung durchgeführt, so wird zunächst eine Messung nach Riva-Rocci durchgeführt (Zeitabschnitt 12.2). Die Messung nach Riva-Rocci ergibt die Werte für die Systole (12.3) und für die Diastole (12.4). Mit diesen Werten werden in einer Kalibrationsphase (12.5) Skalierungsfaktoren zum Erhalt einer Blutdruckkurve aus dem Luftdruck in der Manschette bestimmt. Danach wird der zeitliche Verlauf der Luftdruckwerte verwendet, um skaliert als Blutdruckkurve dargestellt zu werden. Die Kalibrationsphase zusammen mit der Darstellungsphase (12.4) nutzt die Redtel Methode.

In einer weiteren Entwicklungsstufe kann die Atmung (12.7) aus den Pulsintervallen bestimmt werden. Dabei werden die einzelnen Intervalllängen bestimmt, welche von Puls zu Puls mit der Atmung variieren, wodurch die Atmung messbar wird. Dies kann auch während der Riva-Rocci Phase erfolgen, Wodurch die Einordnung der Blutdruckwerte auf die Phase der Atmung ermöglicht wird (vgl. Beschreibung Fig. 14 dazu).

*Figure 13*

Typischer Luftdruckverlauf und Beaufschlagungsdruckverlauf bei einer kontinuierlichen Blutdruckmessung mit einer um die Logiken der verbesserten Redtel Methode erweiterten herkömmlichen Blutdruckmanschette, welche neben den Messergebnissen auch Daten zur Zeiterfassung dieser Daten ausgibt. Gezeigt ist der Luftdruckverlauf in der Manschette (13.1)

und die Rohdaten eines Beaufschlagungssensors (13.2). Die Messung nach Riva-Rocci ergibt die Werte für die Systole (13.3) und die Werte für die Diastole (13.4). Neben diesen Werten werden auch die Zeitpunkt der Erhebung ausgegeben. Diese können verwendet werden, damit die Werte des Beaufschlagungssensors eindeutig kalibriert werden können. Die Kalibration findet zu diesen Zeitpunkten in den beiden Kurven statt (13.5 für den diastolischen Wert und 13.6 für den systolischen Wert).

Die Kalibrierung ist somit mit dem Ende der Riva-Rocci Messung abgeschlossen.

*Figure 14:*

Ein Problem bei den zuvor gezeigten Kalibrationsmethoden ist die Annahme, dass der Blutdruck ein (zumindest kurzzeitig) konstanter Wert sei. Dies ist nicht der Fall, besonders die Atmung führt zu einer Variation des Blutdrucks zwischen jedem Herzschlag, siehe Fig. 5.

Um die Atmung zu berücksichtigen, wird neben der Luftdruckkurve (14.1) auch ein EKG (14.2) aufgezeichnet. Das EKG kann bzgl. den einzelnen Intervalllängen und deren Unterschiede untereinander untersucht werden. Werden die Intervalllängen als zeitliche Funktion aufgetragen, so kann eine Welle erkannt werden (idealisiert dargestellt als 14.5). Die Wellenlänge entspricht einem Atemzug.

Bei der Messung nach Riva-Rocci werden bevorzugt neben dem systolischen Wert (14.3) und dem diastolischen Wert (14.4) auch deren Messzeitpunkte ausgewertet. Diese Zeitpunkte werden einer Phase in der Atmung (14.6 für die Diastole und 14.7 für die Systole) zugeordnet. Bei der anschließenden Messung nach verbesserten Redtel Methode kann nun eine Kalibration durchgeführt werden, wenn sich die Atmung in der gleichen Phase befindet, wie bei den Messungen von Diastole (z.B. 14.8) und Systole (z.B. 14.9). Diese Kalibration erlaubt es die Ungenauigkeiten der Messung nach Riva-Rocci durch die Atmung, wie sie in Fig. 5 beschrieben werden, auszugleichen.

Somit kann diese Methode verwendet werden, um eine verbesserte einzel- bzw statische Messung gegenüber der herkömmlichen Riva-Rocci-Methode durchzuführen. Der Messverlauf wäre genauso, jedoch wird nach der Kalibrierung der Druck vollständig abgelassen und die Messung wäre abgeschlossen. Da die Kalibration über einen Atemzyklus durchgeführt wurde, sind auch alle Blutdruckwerte für den Atemzyklus bekannt, und eine Angabe von maximalen und minimalen Werten für Diastole und Systole ist möglich.

*Figure 15*

In einer weiteren Entwicklungsstufe soll der Anpressdruck der Luftdruckmanschette weiter reduziert werden. Ausgehend von der Methode, welche in Fig. 14 beschrieben wurde (die Punkte 15.1 bis 15.9 entsprechen den Punkten 14.1 bis 14.9), wird der Blutdruck über die Pulswellenlaufzeit (z.B. 15.10 oder z.B. 15.13) bestimmt. Eine Kalibration der Pulswellenlaufzeit zu Werten des Blutdrucks ist nur möglich, wenn unterschiedliche Blutdrücke untersucht werden. Dies ist durch die Variation des Blutdrucks durch die Atmung gegeben. Daher werden in einer Kalibrationsphase (15.11), welche sich z.B. über einen Atemzyklus erstreckt, zahlreiche oder alle Werte für den Blutdruck erfasst und den entsprechenden Werten zum jeweiligen Herzschlag der Pulswellenlaufzeit (z.B. erste Herzschlag in der Kalibrationsphase 15.10) zugeordnet.

Nach der Kalibrationsphase kann der Druck reduziert werden (15.12). Danach wird die Pulswellenlaufzeit für jeden Herzschlag bestimmt (erster Herzschlag 15.13) und damit der Blutdruck errechnet.

*Figure 16*

Die Methode in Fig. 15 kann nicht komplett auf einen Anpressdruck verzichten. Dies liegt daran, dass die Bestimmung der Pulswellenlaufzeit zwei zu vergleichende Kurven der Aktivitäten des Herzens benötigt. Die Analyse des EKGs (16.2) ergibt die Startzeitpunkte. Die Endzeitpunkte wurden bei Fig. 15 aus der Luftdruckkurve (15.1) ermittelt. Bei einer kompletten Reduzierung des Luftdrucks muss also eine andere Kurve der Aktivitäten des Herzens mit aufgezeichnet werden.

Die hier vorgestellte Methode basiert auf Fig. 15 (die Punkte 16.1 bis 16.9 entsprechen den Punkten 15.1 bis 15.9).

Für die Bestimmung des Endzeitpunkte zur Ermittlung der Pulswellenlaufzeit werden die Daten einer Plethysmographieeinheit (16.14) verwendet. Die Pulswellenlaufzeiten (z.B. erster Herzschlag in der Kalibrationsphase 16.10 oder während der Messung, erster Herzschlag während der Messung 16.13) werden nun über die Werte des EKGs und der Plethysmographieeinheit ermittelt und entsprechen somit der gängigen Methode. Hierbei ist die Kalibration wieder aufgrund der Variationen durch die Atmung des Blutdrucks möglich.

Diese Methode erlaubt es den Blutdruck für jeden Herzschlag zu bestimmten, wobei darüber hinaus auch kein Anpressen durch die Manschette mehr notwendig ist.

*Figure 17*

Eine höhere Genauigkeit bei der Bestimmung von Diastole und Systole als bei der Methode in Fig. 16 kann erreicht werden, wenn statt eines EKGs die Daten einer weiteren Plethysmographieeinheit (17.2) verwendet werden. Die hier vorgestellte Methode ist eine Erweiterung der Methode aus Fig. 16, die Punkte 17.1, 17.3 bis 17.9, 17.11, 17.12, 17.14 entsprechen den jeweiligen Punkten in Fig. 16.

Die Pulswellenlaufzeit wird nun zwischen den beiden Datensätzen der Plethysmographieeinheiten ermittelt. Dabei kann bspw. zwischen den lokalen Minima oder den lokalen Maxima die Laufzeit bestimmt werden.

Es ergeben sich Pulswellenlaufzeiten für die Diastole und die Systole unabhängig voneinander, sodass auch eine unabhängige Kalibration durchgeführt werden kann. Die anschließend ermittelten Werte für Diastole und Systole aus der Pulswellenlaufzeit können somit anders als bei der heutigen Methode (vgl. Fig 16) sich gegeneinander und unabhängig voneinander verändern.

*Figure 18*

Darstellung der beabstandeten Messung mittels Kamera. Die beabstandete Messung ermöglicht es an jeden Punkt (z.B. 18.1-18.3) des Körpers gleichzeitig eine Messung des Pulswellengeschwindigkeit, bzw. der Pulswellenkontur (18.4-18.6) durchzuführen. In diesem Beispiel liegt eine Verschlusskrankheit vor, was an der Messwertwelle 18.5 zu erkennen ist, da hier keine Pulsung zu erkennen ist.

*Figure 19*

Darstellung der beabstandeten Messung. Die beabstandete Messung kann verwendet werden, um eine vergleichende Messung durchzuführen. In diesem Fall wird die rechte (19.1) und die linke (19.2) Gesichtshälfte mit einander verglichen. Die Pulswellen (19.3 und 19.4) weisen bei gesunden Menschen einen zeitlichen Versatz zueinander auf, welcher im Bereich von 10 ms liegt. Liegt eine Verschlusskrankheit oder deren Frühstadium, z.B. eine Stenose der Halsschlagadern vor, können sich andere zeitliche Versätze ergeben. Die Messung des Versatz kann also Hinweise auf diese Krankheiten geben.

*Figure 20*

Darstellung der beabstandeten Messung. Eine weitere Möglichkeit der Erkennung von Verschlusskrankheiten ist die Messung an unterschiedlichen Extremitäten und der Vergleich zwischen linker und rechter Körperhälfte. Wird zwischen Hand (20.1, 20.2) und Fuß (20.3, 20.4) gemessen, so ergibt sich ein zeitlicher Versatz der Kurven zueinander (20.5 zu 20.6 bzw. 20.7 zu 20.8), aufgrund der unterschiedlichen Längen von Arterien. Jedoch sollte sich im gesunden Zustand kein Unterschied zwischen der linken und rechten Körperhälfte ergeben. Auch der Versatz zwischen den Messungen an den Händen bzw. an den Füßen sollte keinen bzw. nur geringen Versatz aufweisen im gesunden Zustand.

Ein Unterschied in beiden Fällen ist ein Hinweis auf eine Erkrankung.

**Patentansprüche**

**1.** Verfahren zum Erhalt von kontinuierlichen Werten des Blutdrucks wobei zu mindestens zwei, insbesondere mindestens vier, unterschiedlichen Blutdruckwerten des Lebewesens die zeitlich zu den jeweiligen Blutdruckwerten der Pulsdruckwelle gehörigen Pulswellenlaufzeiten, Pulswellengeschwindigkeiten, Pulswellenkontouren und/oder elektrische Aktivität des Herzens erhoben werden,
wobei die unterschiedlichen Blutdruckwerte zu unterschiedlichen Atemzuständen mittels eines Blutdruckmanschettengeräts genommen werden und die mindestens zwei zu unterschiedlichen Atemzuständen genommenen unterschiedlichen Blutdruckwerte unterschiedliche Werte aufgrund der natürlichen Variation aufgrund der Unterschiede in der Respiratorischen Sinusarrhythmie zwischen den zwei unterschiedlichen Atemzuständen aufweisen und wobei die Ergebnisse der Messung der Pulswellenlaufzeit, Pulswellengeschwindigkeit, Pulswellenkontur und/oder der elektrischen Aktivität des Herzens mittels der Messungen der mindestens zwei Blutdruckwerten kalibriert werden, wobei zur Kalibrierung die natürliche Variation verwendet wird

**2.** Verfahren nach Anspruch 1 zur nichtinvasiven und kontinuierlichen Blutdruckmessung, **dadurch gekennzeichnet, dass** spätere nicht beaufschlagte Messungen des Blutdrucks, der Pulswellenlaufzeit, der Pulswellengeschwindigkeit und/oder der Pulswellenkontur durchgeführt werden und die Messwerte der späteren nicht beaufschlagten Messungen mittels der bei der beaufschlagten Blutdruckmessung erhobenen Daten nach Anspruch 1 in mindestens einen Blutdruckwert umgerechnet werden.

**3.** System zur nichtinvasiven und kontinuierlichen Blutdruckmessung, aufweisend Mittel zur Durchführung einer nicht beaufschlagten kontinuierlichen Messung von Pulswellenlaufzeit, der Pulswellengeschwindigkeit, der Pulswellenkontur, der elektrischen Aktivität des Herzens und/oder Blutdruck, wobei

das System eingerichtet ist, Messwerte von mindestens zwei beaufschlagten Blutdruckmessungen zu unterschiedlichen Atemzuständen entgegenzunehmen, wobei die mindestens zwei zu unterschiedlichen Atemzuständen genommen beaufschlagten Blutdruckwerte unterschiedliche Werte aufgrund der natürlichen Variation aufgrund der Unterschiede in der Respiratorischen Sinusarrhythmie zwischen den zwei unterschiedlichen Atemzuständen aufweisen, die entgegengenommenen Messwerte der beaufschlagten Messungen und die nicht beaufschlagte Messungen zur Kalibrierung der nicht beaufschlagten Messungen auf Basis der beaufschlagten Blutdruckmessung gemeinsam zu verarbeiten, wobei das System eingerichtet ist, zur Kalibrierung die natürliche Variation zu verwenden,

die nicht beaufschlagten Messungen auf Basis der beaufschlagten Blutdruckmessung zu kalibrieren und

zahlreiche weitere nicht beaufschlagte Messungen des Blutdrucks, der Pulswellenlaufzeit, der Pulswellengeschwindigkeit und/oder der Pulswellenkontur durchzuführen und die zahlreichen weiteren nicht beaufschlagten Messungen des Blutdrucks, der Pulswellenlaufzeit, der Pulswellengeschwindigkeit und/oder der Pulswellenkontur jeweils auf Basis der Kalibrierung in jeweils mindestens einen Blutdruckwert umzurechnen und diesen auszugeben.

4. Verfahren nach Anspruch 1 oder 2, wobei für die beaufschlagte Blutdruckmessung mittels mindestens eines Druckaufnehmers durch den Blutdruck hervorgerufene Druckschwankungen kontinuierlich erfasst werden, wobei mindestens zwei Blutdruckwerte aus den erfassten Druckschwankungen ermittelt werden, **dadurch gekennzeichnet, dass**, zum Reduzieren des Einflusses der Atmung auf die ermittelten Blutdruckwerte, aus den kontinuierlichen Druckschwankungen ein Einfluss der Atmung auf die Schwankung ermittelt wird und/oder Atemzustände ermittelt werden und die Blutdruckwerte aus den mittels des Druckaufnehmers erfassten Werten abgeleitet werden, die zu einem vorbestimmten und/oder gleichen Atemzustand erfasst wurden.

5. System nach Anspruch 3, aufweisend mindestens einen Druckaufnehmer zur kontinuierlichen Erfassung einer durch den Blutdruck hervorgerufenen Druckschwankung, wobei der Druckaufnehmer eingerichtet ist, die mindestens zwei Blutdruckwerte aus den erfassten Druckschwankungen zu ermitteln und auszugeben, **dadurch gekennzeichnet, dass** der Druckaufnehmer eingerichtet ist, zur Reduzierung des Einflusses der Atmung auf die ermittelten Blutdruckwerte, aus den erfassten kontinuierlichen Druckschwankungen einen Einfluss der Atmung auf die Schwankung zu ermitteln und/oder Atemzustände zu ermitteln und die Blutdruckwerte aus den mittels des Druckaufnehmers erfassten Werten abzuleiten, die zu einem vorbestimmten und/oder gleichen Atemzustand erfasst wurden.

6. Verfahren nach einem der Ansprüche 1, 2 oder 4, wobei Pulswellenlaufzeit, Pulswellengeschwindigkeit, die Pulswellenkontur und/oder elektrische Aktivität des Herzens für unterschiedliche Teile der Pulsdruckwelle, wie z.B. der Diastole, der Systole oder der Reflexionswelle, unabhängig voneinander bestimmt, verwendet und/oder auf den Blutdruck kalibriert werden und/oder wobei Pulswellenlaufzeit, Pulswellengeschwindigkeit, Pulswellenkontur und/oder elektrische Aktivität des Herzens mittels Luftdruckmanschette, Plethysmographieeinheit, EKG und/oder Beaufschlagungssensor bestimmt werden.

7. Verfahren nach einem der Ansprüche 1, 2, 4 oder 6, wobei die beaufschlagten Blutdruckmessungen und nicht beaufschlagten Messungen der Pulswellenlaufzeit, Pulswellengeschwindigkeit, Pulswellenkontur und/oder elektrische Aktivität des Herzens zeitnah, zeitgleich und/oder zu einem ähnlichen Atemzustand erfolgen und/oder wobei die beaufschlagten Blutdruckmessungen und nicht beaufschlagten Messungen der Pulswellenlaufzeit, Pulswellengeschwindigkeit und/oder Pulswellenkontur an unterschiedlichen Punkten am Körper des Lebewesen erfolgen, wobei die Punkte so gewählt sind, dass eine sich von oder zum Herzen erstreckende Blutbahn die Punkte nacheinander erreicht.

8. Verfahren nach einem der Ansprüche 2 oder 4, 6 oder 7, soweit diese einen Rückbezug zu Anspruch 2 aufweisen, wobei nach einer Kalibration die Beaufschlagung abgebaut wird und die späteren unbeaufschlagten Messungen der Pulswellenlaufzeit, Pulswellengeschwindigkeit, Pulswellenkontur und/oder elektrische Aktivität des Herzens, durchgeführt werden, insbesondere für mindestens 30 min, insbesondere mindestens 1 Stunde, insbesondere mindestens 6 Stunden, insbesondere mindestens 12 Stunden, insbesondere mindestens 24 Stunden, insbesondere mindestens alle fünf Minuten, insbesondere mindestens alle zwei Minuten, insbesondere mindestens alle 60 Sekunden, insbesondere mindestens alle 20 Sekunden.

9. Verfahren nach einem der Ansprüche 1, 2, 4, oder 6 bis 8, wobei eine Veränderung der Lage eines Messpunkts zum hydrostatischer Indifferenzpunkt (HIP) und/oder zum Herzen durch einen Lage- und/oder einen Beschleunigungssensor erfasst und insbesondere zur Korrektur der Messungen verwendet wird.

10. Verfahren nach einem der Ansprüche 1, 2, 4 oder 6 bis 9, wobei die Pulswellenlaufzeit aus der Pulswellenkontur ermittelt wird.

11. Verfahren nach einem der Ansprüche 1, 2, 4 oder 6 bis 10 oder System nach einem der Ansprüche 3 oder 5, wobei zur beaufschlagten Blutdruckmessung eine Luftdruckmanschette verwendet wird, die einen Sensor zur Bestimmung des Armdurchmessers aufweist, insbesondere einen Biegesensor, einen kapazitiven und/oder induktiven Sensor und/oder einen Sensor, welcher auf der Technologie capacitive touch beruht, und/oder wobei die Luftdruckmanschette so ausgebildet ist, dass sie stufenweise geschlossen wird und/oder in regelmäßigen Abständen Elemente in die Luftdruckmanschette eingebracht sind, die von dem Sensor eindeutig identifiziert werden können, und/oder dass ein Luftsack der Luftdruckmanschette in mehrere Kammern aufgeteilt ist und insbesondere eine aktive Fläche, insbesondere Andruckfläche, des Luftsacks der Luftdruckmanschette durch Hinzu- oder Wegschalten von Kammern mittels elektrisch schaltbaren Ventilen auf den Armdurchmesser angepasst werden kann, wobei nichtgeschaltete Kammern während der Messung nicht mit Luft befüllt werden und/oder die Luftdruckmanschette so ausgebildet ist, dass eine aktive Fläche, insbesondere Andruckfläche, des Luftsacks der Luftdruckmanschette durch zwei Kammern, die insbesondere durch Riemen aneinander gehalten werden, eingestellt werden kann, indem eine erste der zwei Kammern zur Blutdruckmessung verwendet wird und eine zweite der zwei Kammern zur Verformung der ersten Kammer verwendet wird, insbesondere indem diese durch Druckänderung die Abschnürung der ersten Kammer durch die Riemen verändert.

12. Verfahren nach einem der Ansprüche 1, 2, 4 oder 6 bis 11, wobei auf Basis der Messungen von Herzaktionen, insbesondere des Blutdrucks und/oder insbesondere des Pulses, insbesondere unbeaufschlagten Messungen, insbesondere weiteren Messungen, Geräte gesteuert und/oder Steuer- und/oder Handlungsanweisungen ausgegeben werden, wobei diese Geräte bspw. automatisierte Medikationssysteme, wie Medikamentenpumpen, Beatmungsmaschinen, Notrufsysteme, Verkehrsmittel, die dann einen automatisierten Notruf absetzten können und/oder auch autonom fahrende Verkehrsmittel, insbesondere Fahrzeug, sein können, die insbesondere bei Erkennen von kritischen Herzzuständen autonom reagieren können, indem insbesondere eine Warnung an den Nutzer ausgegeben wird und/oder insbesondere ein Notruf ausgelöst wird, insbesondere das Fahrzeug an den Fahrbahnrand gefahren wird und/oder insbesondere eine Fahrt zu einem, insbesondere zum nächst gelegenen, Krankenhaus eingeleitet wird, wobei vorteilhaft die zulässige Höchstgeschwindigkeit überschritten wird, was insbesondere durch Signalisierung des Fahrzeugs an andere Verkehrsteilnehmer, insbesondere durch Licht-, Ton- und/oder Funksignale risikoarm gestaltet werden kann.

13. Verfahren nach einem der Ansprüche 1, 2, 4 oder 6 bis 12, wobei die Erkennung des Atemzustandes, der Atmung und/oder Atemfrequenz

mittels Erfassung periodischer Änderungen von Diastole und Systole, deren Abstand und/oder Ausprägung und/oder
mittels Erfassung periodischer Änderungen des Pulsdrucks und/oder
mittels Erfassung periodischer Änderungen des RR-Intervalls und/oder
mittels Erfassung periodischer Änderungen des Sauerstoffgehalts im Blut, insbesondere
mittels der Erfassung der Veränderung der Reflexionseigenschaft von Licht, beispielsweise mittels Pulsoxymieter oder Kamera, erfolgt.

**Claims**

1. A method for obtaining continuous values of blood pressure, wherein the pulse wave transit times, pulse wave velocities, pulse wave contours and/or electrical activity of the heart associated with the respective blood pressure values of the pulse pressure wave are determined for at least two, in particular at least four, different blood pressure values of the living organism, wherein the different blood pressure values are taken at different breathing states by means of a blood pressure cuff device and said at least two different blood pressure values taken at different breathing states have different values due to the natural variation due to the differences in respiratory sinus arrhythmia between the two different breathing states, and wherein the results of the measurement of the pulse wave transit time, pulse wave velocity, pulse wave contour and/or the electrical activity of the heart are calibrated by means of the measurements of said at least two blood pressure values, wherein the natural variation is used for calibration.

2. A method according to claim 1 for non-invasive and continuous blood pressure measurement, **characterised in that** subsequent non-stressed measurements of the blood pressure, the pulse wave transit time, the pulse wave velocity and/or the pulse wave contour are carried out and the measured values of the subsequent non-stressed measurements are converted into at least one blood pressure value by means of the data collected during the stressed blood pressure measurement according to claim 1.

3. A system for non-invasive and continuous blood pressure measurement, comprising means for performing a non-invasive continuous measurement of pulse wave transit time, pulse wave velocity, pulse wave contour, electrical activity of the heart and/or blood pressure, wherein the system is arranged to receive measured values from at least two pressurised blood pressure measurements for different breathing states, wherein said at least two pressurised blood pressure values taken for different breathing states have different values due to the natural variation due to the differences in respiratory sinus arrhythmia between the two different breathing states, to jointly process the received measured values of the pressurised measurements and the non-pressurised measurements for calibrating the non-pressurised measurements on the basis of the pressurised blood pressure measurement, wherein the system is set up to use the natural variation for calibration, to calibrate the non-pressurised measurements on the basis of the pressurised blood pressure measurement and to carry out numerous further non-pressurised measurements of the blood pressure, the pulse wave transit time, the pulse wave velocity and/or the pulse wave contour and to convert the numerous further non-pressurised measurements of the blood pressure, the pulse wave transit time, the pulse wave velocity and/or the pulse wave contour into at least one blood pressure value in each case on the basis of the calibration and to output this.

4. A method according to claim 1 or 2, wherein pressure fluctuations caused by the blood pressure are continuously recorded for the pressurised blood pressure measurement by means of at least one pressure transducer, wherein at least two blood pressure values are determined from the detected pressure fluctuations, **characterised in that**, in order to reduce the influence of respiration on the determined blood pressure values, an influence of respiration on the fluctuation is determined from the continuous pressure fluctuations and/or respiratory states are determined and the blood pressure values are derived from the values detected by means of the pressure transducer, which were detected for a predetermined and/or identical respiratory state.

5. A system according to claim 3, comprising at least one pressure transducer for continuously detecting a pressure fluctuation caused by the blood pressure, wherein the pressure transducer is set up to determine and output said at least two blood pressure values from the detected pressure fluctuations, **characterised in that**, in order to reduce the influence of breathing on the determined blood pressure values, the pressure transducer is set up to determine an influence of breathing on the fluctuation from the detected continuous pressure fluctuations and/or to determine breathing states and to derive the blood pressure values from the values detected by means of the pressure transducer, which were detected for a predetermined and/or identical breathing state.

6. A method according to one of claims 1, 2 or 4, wherein pulse wave transit time, pulse wave velocity, the pulse wave contour and/or electrical activity of the heart for different parts of the pulse pressure wave, such as the diastole, the systole or the reflection wave, are determined, used and/or calibrated to the blood pressure independently of one another and/or wherein pulse wave transit time, pulse wave velocity, pulse wave contour and/or electrical activity of the heart are determined by means of an air pressure cuff, plethysmography unit, ECG and/or loading sensor.

7. A method according to one of claims 1, 2, 4 or 6, wherein the pressurised blood pressure measurements and non-pressurised measurements of the pulse wave transit time, pulse wave velocity, pulse wave contour and/or electrical activity of the heart are carried out at the same time, simultaneously and/or at a similar breathing state and/or whereby the pressurised blood pressure measurements and non-pressurised measurements of the pulse wave transit time, pulse wave velocity and/or pulse wave contour take place at different points on the body of the living being, where the points are chosen so that a bloodstream extending from or to the heart reaches the points one after the other.

8. A method according to one of claims 2 or 4, 6 or 7, insofar as these have a reference back to claim 2, wherein, after a calibration, the loading is reduced and the subsequent non-loaded measurements of the pulse wave transit time, pulse wave velocity, pulse wave contour and/or electrical activity of the heart are carried out, in particular for at least 30 minutes, in particular at least 1 hour, in particular at least 6 hours, in particular at least 12 hours, in particular at least 24 hours, in particular at least every five minutes, in particular at least every two minutes, in particular at least every 60 seconds, in particular at least every 20 seconds.

9. A method according to one of claims 1, 2, 4, or 6 to 8, wherein a change in the position of a measuring point relative to the hydrostatic indifference point (HIP) and/or relative to the heart is detected by a position sensor and/or an acceleration sensor and is used in particular to correct the measurements.

10. The method according to any one of claims 1, 2, 4 or 6 to 9, wherein the pulse wave propagation time is determined from the pulse wave contour.

11. A method according to one of claims 1, 2, 4 or 6 to 10 or system according to one of claims 3 or 5, wherein an air pressure cuff is used for the pressurised blood pressure measurement, which has a sensor for determining the arm diameter, in particular a bending sensor, a capacitive and/or inductive sensor and/or a sensor based on "capacitive touch" technology, and/or wherein the air pressure cuff is designed such that it is closed in stages and/or elements are inserted into the air pressure cuff at regular intervals, which can be clearly identified by the sensor, and/or that an air bag of the air pressure cuff is divided into several chambers and, in particular, an active area, in particular pressure area, of the air bag of the air pressure cuff can be adapted to the arm diameter by adding or removing chambers by means of electrically switchable valves, wherein non-connected chambers are not filled with air during the measurement and/or the air pressure cuff is designed such that an active area, in particular a contact pressure area, of the air bag of the air pressure cuff can be adjusted by two chambers, which are held together in particular by belts, by using a first of the two chambers to measure blood pressure and a second of the two chambers to deform the first chamber, in particular by changing the constriction of the first chamber by the straps by changing the pressure.

12. A method according to one of claims 1, 2, 4 or 6 to 1 1, wherein devices are controlled and/or control and/or action instructions are issued on the basis of the measurements of cardiac actions, in particular of blood pressure and/or in particular of the pulse, in particular unstimulated measurements, in particular further measurements, whereby these devices can be, for example, automated medication systems, such as medication pumps, ventilators, emergency call systems, means of transport that can then make an automated emergency call and/or also autonomously driving means of transport, in particular vehicles, which can react autonomously, in particular when critical cardiac conditions are detected, in particular by issuing a warning to the user and/or in particular by triggering an emergency call, in particular by driving the vehicle to the side of the road and/or in particular by initiating a journey to a hospital, in particular to the nearest hospital, whereby the maximum permissible speed is advantageously exceeded, which can be made low-risk in particular by signalling the vehicle to other road users, especially by means of light, sound and/or radio signals.

13. A method according to one of claims 1, 2, 4 or 6 to 12, wherein the detection of the respiratory state, respiration and/or respiratory rate is carried out by means of detection of periodic changes in diastole and systole, their spacing and/or expression and/or by means of detection of periodic changes in the pulse pressure and/or by means of detection of periodic changes in the RR interval and/or by means of detection of periodic changes in the oxygen content in the blood, in particular by means of detection of the change in the reflection property of light, for example by means of a pulse oximeter or camera.

## Revendications

1. Procédé d'obtention de valeurs continues de la pression artérielle, dans laquelle les temps de transit de l'onde de pouls, les vitesses de l'onde de pouls, les contours de l'onde de pouls et/ou l'activité électrique du coeur associés aux valeurs respectives de la pression artérielle de l'onde de pression du pouls sont déterminés pour au moins deux, en particulier au moins quatre, valeurs différentes de la pression artérielle de l'organisme vivant, dans lequel les différentes valeurs de pression artérielle sont prises à différents états respiratoires au moyen d'un dispositif de brassard de pression artérielle et lesdites au moins deux valeurs différentes de pression artérielle prises à différents états respiratoires ont des valeurs différentes en raison de la variation naturelle due aux différences d'arythmie sinusale respiratoire entre les deux différents états respiratoires, et dans lequel les résultats de la mesure du temps de transit de l'onde de pouls, de la vitesse de l'onde de pouls, du contour de l'onde de pouls et/ou de l'activité électrique du coeur sont étalonnés au moyen des mesures de ces deux valeurs de pression artérielle au moins, la variation naturelle étant utilisée pour la calibration.

2. Procédé selon la revendication 1 pour la mesure non invasive et continue de la pression artérielle, **caractérisée par le fait que** des mesures ultérieures non stressées de la pression artérielle, du temps de transit de l'onde de pouls, de la vitesse de l'onde de pouls et/ou du contour de l'onde de pouls sont effectuées et que les valeurs mesurées des mesures ultérieures non stressées sont converties en au moins une valeur de pression artérielle au moyen des données collectées pendant la mesure de la pression artérielle stressée selon la revendication 1.

3. Système de mesure non invasive et continue de la pression artérielle, comprenant des moyens pour effectuer une mesure continue non invasive du temps de transit de l'onde de pouls, de la vitesse de l'onde de pouls, du contour de l'onde de pouls, de l'activité électrique du coeur et/ou de la pression artérielle, dans lequel le système est conçu pour recevoir les valeurs mesurées d'au moins deux mesures de pression artérielle sous pression pour différents états respiratoires, dans lequel lesdites au moins deux valeurs de pression artérielle sous pression prises pour différents états respiratoires ont des valeurs différentes en raison de la variation naturelle due aux différences d'arythmie sinusale respiratoire entre les deux différents états respiratoires, pour traiter conjointement les valeurs mesurées reçues des mesures sous pression et des mesures non sous pression afin d'étalonner les mesures non sous pression sur la base de la mesure de la pression artérielle sous pression, dans lequel le système est configuré pour utiliser la variation naturelle pour la calibration, pour étalonner les mesures non pressurisées sur la base de la mesure de la pression sanguine pressurisée et pour effectuer de nombreuses autres mesures non pressurisées de la pression sanguine, du temps de transit de l'onde de pouls, de la vitesse de l'onde de pouls et/ou du contour de l'onde de pouls et pour convertir les nombreuses autres mesures non pressurisées de la pression sanguine, du temps de transit de l'onde de pouls, de la vitesse de l'onde de pouls et/ou du contour de l'onde de pouls en au moins une valeur de pression sanguine dans chaque cas sur la base de la calibration et pour l'éditer.

4. Procédé selon les revendications 1 ou 2, dans lequel les fluctuations de pression causées par la pression sanguine sont enregistrées en continu pour la mesure de la pression sanguine sous pression au moyen d'au moins un transducteur de pression, dans lequel au moins deux valeurs de pression artérielle sont déterminées à partir des fluctuations de pression détectées, **caractérisées par le fait que**, pour réduire l'influence de la respiration sur les valeurs de pression artérielle déterminées, une influence de la respiration sur la fluctuation est déterminée à partir des fluctuations de pression continues et/ou des états respiratoires sont déterminés et les valeurs de pression artérielle sont dérivées des valeurs détectées au moyen du transducteur de pression, qui ont été détectées pour un état respiratoire prédéterminé et/ou identique.

5. Système selon la revendication 3, comprenant au moins un transducteur de pression pour détecter en continu une fluctuation de pression causée par la pression sanguine, le transducteur de pression étant configuré pour déterminer et émettre lesdites au moins deux valeurs de pression sanguine à partir des fluctuations de pression détectées, **caractérisé par le fait que**, pour réduire l'influence de la respiration sur les valeurs de pression artérielle déterminées, le capteur de pression est configuré pour déterminer une influence de la respiration sur la fluctuation des fluctuations de pression continues détectées et/ou pour déterminer les états respiratoires et pour dériver les valeurs de pression artérielle à partir des valeurs détectées au moyen du capteur de pression, qui ont été détectées pour un état respiratoire prédéterminé et/ou identique.

6. Procédé selon l'une des revendications 1, 2 ou 4, dans lequel le temps de transit de l'onde de pouls, la vitesse de l'onde de pouls, le contour de l'onde de pouls et/ou l'activité électrique du coeur pour différentes parties de l'onde de pression du pouls, telles que la diastole, la systole ou l'onde de réflexion, sont déterminés, utilisés et/ou étalonnés par rapport à la pression sanguine indépendamment les uns des autres et/ou dans laquelle le temps de transit de l'onde de pouls, la vitesse de l'onde de pouls, le contour de l'onde de pouls et/ou l'activité électrique du coeur sont déterminés au moyen d'un brassard de pression d'air, d'une unité de pléthysmographie, d'un ECG et/ou d'un capteur de charge.

7. Procédé selon l'une des revendications 1, 2, 4 ou 6, dans lequel les mesures de la pression artérielle sous pression et les mesures sans pression du temps de transit de l'onde de pouls, de la vitesse de l'onde de pouls, du contour de l'onde de pouls et/ou de l'activité électrique du coeur sont effectuées au même moment, simultanément et/ou dans un état respiratoire similaire et/ou les mesures de la pression artérielle sous pression et les mesures sans pression du temps de transit de l'onde de pouls, de la vitesse de l'onde de pouls et/ou du contour de l'onde de pouls sont effectuées en différents points du corps de l'être vivant, dans lequel les points sont choisis de manière à ce qu'une circulation sanguine partant du coeur ou allant vers le coeur atteigne les points l'un après l'autre.

8. Procédé selon l'une des revendications 2 ou 4, 6 ou 7, dans la mesure où celles-ci renvoient à la revendication 2, dans lequel, après une calibration, la charge est réduite et les mesures ultérieures non chargées du temps de transit de l'onde de pouls, de la vitesse de l'onde de pouls, du contour de l'onde de pouls et/ou de l'activité électrique du coeur sont effectuées, en particulier pendant au moins 30 minutes, en particulier au moins 1 heure, en particulier au moins 6 heures, en particulier au moins 12 heures, en particulier au moins 24 heures, en particulier au moins toutes les cinq minutes, en particulier au moins toutes les deux minutes, en particulier au moins toutes les 60 secondes, en particulier au moins toutes les 20 secondes.

9. Procédé selon l'une des revendications 1, 2, 4, ou 6 à 8, dans lequel un changement de position d'un point de mesure par rapport au point d'indifférence hydrostatique (PIH) et/ou par rapport au coeur est détecté par un capteur de position et/ou un capteur d'accélération et est utilisé notamment pour corriger les mesures.

10. Procédé selon l'une des revendications 1, 2, 4 ou 6 à 9, dans lequel le temps de propagation de l'onde d'impulsion est déterminé à partir du contour de l'onde d'impulsion.

**11.** Procédé selon l'une des revendications 1, 2, 4 ou 6 à 10 ou système selon l'une des revendications 3 ou 5, dans lequel un brassard à pression atmosphérique est utilisé pour la mesure de la pression sanguine sous pression, qui comporte un capteur pour déterminer le diamètre du bras, en particulier un capteur de flexion, un capteur capacitif et/ou inductif et/ou un capteur basé sur la technologie "capacitive touch", et/ou dans lequel le brassard à air comprimé est conçu de manière à être fermé par étapes et/ou des éléments sont insérés dans le brassard à air comprimé à intervalles réguliers, qui peuvent être clairement identifiés par le capteur, et/ou qu'un coussin d'air du brassard à pression est divisé en plusieurs chambres et, en particulier, qu'une zone active, notamment une zone de pression, du coussin d'air du brassard à pression peut être adaptée au diamètre du bras en ajoutant ou en supprimant des chambres au moyen de soupapes commutables électriquement, dans lequel les chambres non reliées ne sont pas remplies d'air pendant la mesure et/ou le brassard est conçu de manière à ce qu'une zone active, en particulier une zone de pression de contact, du sac gonflable du brassard puisse être ajustée par deux chambres, qui sont maintenues ensemble, en particulier par des courroies, en utilisant une première des deux chambres pour mesurer la pression sanguine et une seconde des deux chambres pour déformer la première chambre, notamment en modifiant le rétrécissement de la première chambre par les sangles en modifiant la pression.

**12.** Procédé selon l'une des revendications 1, 2, 4 ou 6 à 11, dans lequel les dispositifs sont commandés et/ou des instructions de commande et/ou d'action sont émises sur la base des mesures des actions cardiaques, en particulier de la pression artérielle et/ou en particulier du pouls, en particulier des mesures non stimulées, en particulier d'autres mesures, ces dispositifs peuvent être, par exemple, des systèmes de médication automatisés, tels que des pompes à médicaments, des ventilateurs, des systèmes d'appel d'urgence, des moyens de transport pouvant émettre un appel d'urgence automatisé et/ou des moyens de transport à conduite autonome, en particulier des véhicules, qui peuvent réagir de manière autonome, notamment lorsque des conditions cardiaques critiques sont détectées, notamment en avertissant l'utilisateur et/ou notamment en déclenchant un appel d'urgence, notamment en conduisant le véhicule sur le bas-côté de la route et/ou notamment en déclenchant un voyage vers un hôpital, notamment vers l'hôpital le plus proche, qui permet de dépasser avantageusement la vitesse maximale autorisée, ce qui peut être rendu moins risqué notamment en signalant le véhicule aux autres usagers de la route, en particulier au moyen de signaux lumineux, sonores et/ou radiophoniques.

**13.** Procédé selon l'une des revendications 1, 2, 4 ou 6 à 12, dans lequel la détection de l'état respiratoire, de la respiration et/ou de la fréquence respiratoire est effectuée au moyen de la détection des changements périodiques de la diastole et de la systole, de leur espacement et/ou de leur expression et/ou au moyen de la détection des changements périodiques de la pression du pouls et/ou au moyen de la détection des changements périodiques de l'intervalle RR et/ou au moyen de la détection des changements périodiques de la teneur en oxygène du sang, en particulier au moyen de la détection du changement de la propriété de réflexion de la lumière, par exemple au moyen d'un oxymètre de pouls ou d'une caméra.

**Fig. 1**

1.1  1.2  1.3  1.4  1.5

1.6  1.7

**Fig. 2**

**Fig. 3**

Fig. 4

Fig. 5

Fig. 6

**Fig. 7**

**Fig. 8**

**Fig. 9**

Fig. 10

10.5   10.4

10.6

09   15

06   18

03   21

10.1
10.2
10.3

Fig. 11

11.1   11.7   11.8   11.2

12:43

BP   Report   11.9

11.3   124/85   11.4

♥ 73

12:25   135/90 (80)   WHO
12:30   132/88 (76)
12:35   120/82 (72)
12:40   122/82 (69)   11.10

07:00  13:00  19:00  01:00  07:00

11.11   11.5   11.6

Fig. 12

12.3

12.1

12.5

Druck

12.7

12.4

12.2   12.6

Zeit

**Fig. 13**

**Fig. 14**

**Fig. 15**

Fig. 16

Fig. 17

Fig. 18

Fig. 19

Fig. 20

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2012136261 A
- DE 102018001390
- EP 2018056275 W

- DE 102018007180
- DE 102018002268

**In der Beschreibung aufgeführte Nicht-Patentliteratur**

- **SIN P.Y.W ; GALLETLY D.C. ; TZENG Y.C.** Influence of breathing frequency on the pattern of respiratory sinus arrhythmia and blood pressure: old questions revisited. *Am J Physiol Heart Circ Physiol,* 2010, vol. 298, H1588-H1599

- **LEWINGTON S. ; CLARKE R. ; QIZILBASH N. ; PETO R. ; COLLINS R.** Age-specific relevance of usual blood pressure to vascular mortality: a metaanalysis of individual data for one million adults in 61 prospective studie. *Lancet,* 2002, vol. 360, 1903-1913